(19) Europäisches Patentamt
European Patent Office
Office européen des brevets

(11) EP 1 117 687 B1

(12) EUROPEAN PATENT SPECIFICATION

(45) Date of publication and mention of the grant of the patent:
**25.06.2008 Bulletin 2008/26**

(21) Application number: **99951690.9**

(22) Date of filing: **30.09.1999**

(51) Int Cl.:
*C07K 14/47* (2006.01) *A61K 48/00* (2006.01)
*G01N 33/50* (2006.01) *C07K 16/30* (2006.01)
*A61K 38/17* (2006.01) *A61P 35/00* (2006.01)

(86) International application number:
**PCT/US1999/022819**

(87) International publication number:
**WO 2000/018795 (06.04.2000 Gazette 2000/14)**

(54) **COMPOSITIONS AND METHODS FOR WT1 SPECIFIC IMMUNOTHERAPY**

ZUSAMMENSETZUNGEN UND VERFAHREN FÜR WT1-SPEZIFISCHE IMMUNOTHERAPIE

COMPOSITIONS ET METHODES RELATIVES A UNE IMMUNOTHERAPIE SPECIFIQUE DU WT1

(84) Designated Contracting States:
**AT BE CH CY DE DK ES FI FR GB GR IE IT LI LU MC NL PT SE**

(30) Priority: **30.09.1998 US 164223**
**25.03.1999 US 276484**

(43) Date of publication of application:
**25.07.2001 Bulletin 2001/30**

(73) Proprietors:
- **CORIXA CORPORATION**
**Hamilton, MT 59840-3131 (US)**
- **Gaiger, Alexander**
**Seattle, WA 98112 (US)**

(72) Inventors:
- **GAIGER, Alexander**
**Seattle, WA 98112 (US)**
- **CHEEVER, Martin**
**Mercer Island, WA 98104 (US)**

(74) Representative: **Walker, Ross Thomson et al**
**Forrester & Boehmert**
**Pettenkoferstrasse 20-22**
**80336 München (DE)**

(56) References cited:
**WO-A-91/07509** **WO-A-95/06725**
**WO-A-95/29995** **WO-A-99/58135**

- **ROTHBARD, J. B. & TAYLOR, W.R.: "A sequence pattern common to T cell epitopes" EMBO JOURNAL, vol. 7, no. 1, 1988, XP000877127 EYNSHAM, OXFORD GB cited in the application**
- **DEAVIN A J ET AL: "STATISTICAL COMPARISON OF ESTABLISHED T-CELL EPITOPE PREDICTORS AGAINST A LARGE DATABASE OF HUMAN AND MURINE ANTIGENS" MOLECULAR IMMUNOLOGY,US,ELMSFORD, NY, vol. 33, no. 2, 1996, pages 145-155, XP000884372 ISSN: 0161-5890**
- **RAUSCHER FJ 3RD, MORRIS JF, FREDERICKS WJ, LOPEZ-GUISA J, BALAKRISHNAN C, JOST M, HERLYN M, RODECK U: "Characterization of monoclonal antibodies directed to the amino-terminus of the WT1, Wilms' tumor suppressor protein." HYBRIDOMA, vol. 17, no. 2, April 1998 (1998-04), pages 191-198, XP000879429**
- **TOES RE, KAST WM, BLOM RJ, BAKKER SC, OFFRINGA R, MELIEF CJ: "Efficient tumor eradication by adoptively transferred cytotoxic T-cell clones in allogeneic hosts." INT J CANCER. 1996 MAY 29;66(5):686-91., XP000884758**
- **SADOVNIKOVA E, JOPLING LA, SOO KS, STAUSS HJ: "Generation of human tumor-reactive cytotoxic T cells against peptides presented by non-self HLA class I molecules." EUR J IMMUNOL. 1998 JAN;28(1):193-200., XP000864767**
- **GAIGER A ET AL: "WT1: A NEW LEUKEMIA AND CANCER ANTIGEN" PROCEEDINGS OF THE ANNUAL MEETING OF THE AMERICAN ASSOCIATION FOR CANCER RESEARCH,US, PHILADELPHIA, PA: AACR, vol. 40, 1999, page 424 XP000876782**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 117 687 B1

- GAIGER A ET AL: "IMMUNITY TO WT1 IN ANIMAL MODELS AND LEUKEMIA PATIENTS" BLOOD, US,W.B. SAUNDERS, PHILADELPHIA, VA, vol. 94, no. 10, 15 November 1999 (1999-11-15), page 78 XP000876777 ISSN: 0006-4971
- BELLANTUONO, I.; GAO, L. ; ELSAESSER, A.; MARLEY, S.; GORDON,: "Selective elimination of leukemic progenitors by allorestricted CTL specific for Wilms tumor antigen-1 (WT-1)" BLOOD, (NOV. 15, 1999) VOL. 94, NO. 10 SUPPL. 1 PART 1, PP. 532A-533A., XP000877199 & Meeting Info.: Forty-first Annual Meeting of the American Society of Hematology New Orleans, Louisiana, USA December 3-7, 1999

## Description

### TECHNICAL FIELD

The present invention relates generally to the immunotherapy of malignant diseases such as leukemia and cancers. The invention is more specifically related to compositions for generating or enhancing an immune response to WT1, and to the use of such compositions for preventing and/or treating malignant diseases.

### BACKGROUND OF THE INVENTION

Cancer and leukemia are significant health problems in the United States and throughout the world. Although advances have been made in detection and treatment of such diseases, no vaccine or other universally successful method for prevention or treatment of cancer and leukemia is currently available. Management of the diseases currently relies on a combination of early diagnosis and aggressive treatment, which may include one or more of a variety of treatments such as surgery, radiotherapy, chemotherapy and hormone therapy. The course of treatment for a particular cancer is often selected based on a variety of prognostic parameters, including an analysis of specific tumor markers. However, the use of established markers often leads to a result that is difficult to interpret, and the high mortality continues to be observed in many cancer patients.

Immunotherapies have the potential to substantially improve cancer and leukemia treatment and survival. Recent data demonstrate that leukemia can be cured by immunotherapy in the context of bone marrow transplantation (e.g., donor lymphocyte infusions). Such therapies may involve the generation or enhancement of an immune response to a tumor-associated antigen (TAA). However, to date, relatively few TAAs are known and the generation of an immune response against such antigens has, with rare exceptions, not been shown to be therapeutically beneficial.

Accordingly, there is a need in the art for improved methods for leukemia and cancer prevention and therapy. The present invention fulfills these needs and further provides other related advantages.

### SUMMARY OF THE INVENTION

Briefly stated, this invention provides compositions and their use methods for the diagnosis and therapy of diseases such as leukemia and cancer. In particular, the present invention provide etc. polypeptide of claim 1 comprising an immunogenic portion of a native WT1. Within certain embodiments, the polypeptide comprises no more than 16 consecutive amino acid residues of a native WT1 polypeptide. The immunogenic portion preferably binds to an MHC class I and/or class II molecule.

Within further aspects, there is also disclosed polypeptides comprising a variant of an immunogenic portion of a WT1 protein, wherein the variant differs from the immunogenic portion due to substitutions at between 1 and 3 amino acid positions within the immunogenic portion such that the ability of the variant to react with antigen-specific antisera and/or T-cell lines or clones is enhanced relative to a native WT1 protein.

The present invention further provides WT1 polynucleotides that encode a WT1 polypeptide as described above.

Within other aspects, the present invention provides pharmaceutical compositions and vaccines. Pharmaceutical compositions may comprise a polypeptide as described above and/or one or more of (i) a WT1 polynucleotide; (ii) an antibody or antigen-binding fragment thereof that specifically binds to a WT1 polypeptide; (iii) a T cell that specifically reacts with a WT1 polypeptide or (iv) an antigen-presenting cell that expresses a WT1 polypeptide, in combination with a pharmaceutically acceptable carrier or excipient. Vaccines comprise a polypeptide as described above and/or one or more of (i) a WT1 polynucleotide, (ii) an antigen-presenting cell that expresses a WT1 polypeptide or (iii) an anti-idiotypic antibody, and a non-specific immune response enhancer. Within certain embodiments, less than 23 consecutive amino acid residues, preferably less than 17 amino acid residues, of a native WT1 polypeptide are present within a WT1 polypeptide employed within such pharmaceutical compositions and vaccines. The immune response enhancer may be an adjuvant. Preferably, an immune response enhancer enhances a T cell response.

Methods for enhancing or inducing an immune response in a patient are also disclosed, comprising administering to a patient a pharmaceutical composition or vaccine as described above. In certain embodiments, the patient is a human.

Methods for inhibiting the development of a malignant disease in a patient are also disclosed, comprising administering to a patient a pharmaceutical composition or vaccine as described above. Malignant diseases include, but are not limited to leukemias *(e.g.,* acute myeloid, acute lymphocytic and chronic myeloid) and cancers (*e.g.*, breast, lung, thyroid or gastrointestinal cancer or a melanoma). The patient may, but need not, be afflicted with the malignant disease, and the administration of the pharmaceutical composition or vaccine may inhibit the onset of such a disease, or may inhibit progression and/or metastasis of an existing disease.

The present invention further provides, within other aspects, methods for removing cells expressing WT1 from bone marrow and/or peripheral blood or fractions thereof, comprising contacting bone marrow, peripheral blood or a fraction of bone marrow or peripheral blood with T cells that specifically react with a WT1 polypeptide, wherein the step of contacting is performed under conditions and for a time sufficient to permit the removal of WT1 positive cells to less than 10%, preferably less than 5% and more preferably less than 1 %, of the number of myeloid or lymphatic cells in the bone marrow, peripheral blood or fraction. Bone marrow, peripheral blood and fractions may be obtained from a patient afflicted with a disease associated with WT1 expression, or may be obtained from a human or non-human mammal not afflicted with such a disease.

Within related aspects, there is also disclosed methods for inhibiting the development of a malignant disease in a patient, comprising administering to a patient bone marrow, peripheral blood or a fraction of bone marrow or peripheral blood prepared as described above. Such bone marrow, peripheral blood or fractions may be autologous, or may be derived from a related or unrelated human or non-human animal (e.g., syngeneic or allogeneic).

In other aspects, the present invention provides methods for stimulating (or priming) and/or expanding T cells, comprising contacting T cells with a WT1 polypeptide under conditions and for a time sufficient to permit the stimulation and/or expansion of T cells. Such T cells may be autologous, allogeneic, syngeneic or unrelated WT1-specific T cells, and may be stimulated *in vitro* or *in vivo*. Expanded T cells may, within certain embodiments, be present within bone marrow, peripheral blood or a fraction of bone marrow or peripheral blood, and may (but need not) be clonal. Within certain embodiments, T cells may be present in a mammal during stimulation and/or expansion. WT1-specific T cells may be used, for example, within donor lymphocyte infusions.

Within related aspects, methods are disclosed for inhibiting the development of a malignant disease in a patient, comprising administering to a patient T cells prepared as described above. Such T cells may, within certain embodiments, be autologous, syngeneic or allogeneic.

The present disclosure further provides, within other aspects, methods for monitoring the effectiveness of an immunization or therapy for a malignant disease associated with WT1 expression in a patient. Such methods are based on monitoring antibody, CD4+ T cell and/or CD8+ T cell responses in the patient. Within certain such aspects, a method may comprise the steps of: (a) incubating a first biological sample with one or more of: (i) a WT1 polypeptide; (ii) a polynucleotide encoding a WT1 polypeptide; or (iii) an antigen presenting cell that expresses a WT1 polypeptide, wherein the first biological sample is obtained from a patient prior to a therapy or immunization, and wherein the incubation is performed under conditions and for a time sufficient to allow immunocomplexes to form; (b) detecting immunocomplexes formed between the WT1 polypeptide and antibodies in the biological sample that specifically bind to the WT1 polypeptide; (c) repeating steps (a) and (b) using a second biological sample obtained from the same patient following therapy or immunization; and (d) comparing the number of immunocomplexes detected in the first and second biological samples, and therefrom monitoring the effectiveness of the therapy or immunization in the patient.

Within certain embodiments of the above methods, the step of detecting comprises (a) incubating the immunocomplexes with a detection reagent that is capable of binding to the immunocomplexes, wherein the detection reagent comprises a reporter group, (b) removing unbound detection reagent, and (c) detecting the presence or absence of the reporter group. The detection reagent may comprise, for example, a second antibody, or antigen-binding fragment thereof, capable of binding to the antibodies that specifically bind to the WT1 polypeptide or a molecule such as Protein A. Within other embodiments, a reporter group is bound to the WT1 polypeptide, and the step of detecting comprises removing unbound WT1 polypeptide and subsequently detecting the presence or absence of the reporter group.

Within further aspects, methods for monitoring the effectiveness of an immunization or therapy for a malignant disease associated with WT1 expression in a patient may comprise the steps of: (a) incubating a first biological sample with one or more of: (i) a WT1 polypeptide; (ii) a polynucleotide encoding a WT1 polypeptide; or (iii) an antigen presenting cell that expresses a WT1 polypeptide, wherein the biological sample comprises CD4+ and/or CD8+ T cells and is obtained from a patient prior to a therapy or immunization, and wherein the incubation is performed under conditions and for a time sufficient to allow specific activation, proliferation and/or lysis of T cells; (b) detecting an amount of activation, proliferation and/or lysis of the T cells; (c) repeating steps (a) and (b) using a second biological sample comprising CD4+ and/or CD8+ T cells, wherein the second biological sample is obtained from the same patient following therapy or immunization; and (d) comparing the amount of activation, proliferation and/or lysis of T cells in the first and second biological samples, and therefrom monitoring the effectiveness of the therapy or immunization in the patient.

The present disclosure further provides methods for inhibiting the development of a malignant disease associated with WT1 expression in a patient, comprising the steps of: (a) incubating $CD4^+$ and/or CD8+ T cells isolated from a patient with one or more of: (i) a WT1 polypeptide; (ii) a polynucleotide encoding a WT1 polypeptide; or (iii) an antigen presenting cell that expresses a WT1 polypeptide, such that the T cells proliferate; and (b) administering to the patient an effective amount of the proliferated T cells, and therefrom inhibiting the development of a malignant disease in the patient. Within certain embodiments, the step of incubating the T cells may be repeated one or more times.

Within other aspects, the present disclosure provides methods for inhibiting the development of a malignant disease associated with WT1 expression in a patient, comprising the steps of: (a) incubating $CD4^+$ and/or CD8+ T cells isolated from a patient with one or more of: (i) a WT1 polypeptide; (ii) a polynucleotide encoding a WT1 polypeptide; or (iii) an antigen presenting cell that expresses a WT1 polypeptide, such that the T cells proliferate; (b) cloning one or more cells that proliferated; and (c) administering to the patient an effective amount of the cloned T cells.

Within other aspects, methods are provided for determining the presence or absence of a malignant disease associated with WT1 expression in a patient, comprising the steps of: (a) incubating $CD4^+$ and/or CD8+ T cells isolated from a patient with one or more of: (i) a WT1 polypeptide; (ii) a polynucleotide encoding a WT1 polypeptide; or (iii) an antigen presenting cell that expresses a WT1 polypeptide; and (b) detecting the presence or absence of specific activation of the T cells, therefrom determining the presence or absence of a malignant disease associated with WT1 expression. Within certain embodiments, the step of detecting comprises detecting the presence or absence of proliferation of the T cells.

Within further aspects, the present disclosure provides methods for determining the presence or absence of a malignant disease associated with WT1 expression in a patient, comprising the steps of: (a) incubating a biological sample obtained from a patient with one or more of: (i) a WT1 polypeptide; (ii) a polynucleotide encoding a WT1 polypeptide; or (iii) an antigen presenting cell that expresses a WT1 polypeptide, wherein the incubation is performed under conditions and for a time sufficient to allow immunocomplexes to form; and (b) detecting immunocomplexes formed between the WT1 polypeptide and antibodies in the biological sample that specifically bind to the WT1 polypeptide; and therefrom determining the presence or absence of a malignant disease associated with WT1 expression.

These and other aspects of the present invention will become apparent upon reference to the following detailed description and attached drawings.

BRIEF DESCRIPTION OF THE DRAWINGS

Figure 1 depicts a comparison of the mouse (MO) and human (HU) WT1 protein sequences (SEQ ID NOS: 320 and 319 respectively).

Figure 2 is a Western blot illustrating the detection of WT1 specific antibodies in patients with hematological malignancy (AML). Lane 1 shows molecular weight markers; lane 2 shows a positive control (WT1 positive human leukemia cell line immunoprecipitated with a WT1 specific antibody); lane 3 shows a negative control (WT1 positive cell line immunoprecipitated with mouse sera); and lane 4 shows a WT1 positive cell line immunoprecipitated with sera of a patient with AML. For lanes 2-4, the immunoprecipitate was separated by gel electrophoresis and probed with a WT1 specific antibody.

Figure 3 is a Western blot illustrating the detection of a WT1 specific antibody response in B6 mice immunized with TRAMP-C, a WT1 positive tumor cell line. Lanes 1, 3 and 5 show molecular weight markers, and lanes 2, 4 and 6 show a WT1 specific positive control (N180, Santa Cruz Biotechnology, polypeptide spanning 180 amino acids of the N-terminal region of the WT1 protein, migrating on the Western blot at 52 kD). The primary antibody used was WT180 in lane 2, sera of non-immunized B6 mice in lane 4 and sera of the immunized B6 mice in lane 6.

Figure 4 is a Western blot illustrating the detection of WT1 specific antibodies in mice immunized with representative WT1 peptides. Lanes 1, 3 and 5 show molecular weight markers and lanes 2, 4 and 6 show a WT1 specific positive control (N180, Santa Cruz Biotechnology, polypeptide spanning 180 amino acids of the N-terminal region of the WT1 protein, migrating on the Western blot at 52 kD). The primary antibody used was WT180 in lane 2, sera of non-immunized B6 mice in lane 4 and sera of the immunized B6 mice in lane 6.

Figures 5A to 5C are graphs illustrating the stimulation of proliferative T cell responses in mice immunized with representative WT1 peptides. Thymidine incorporation assays were performed using one T cell line and two different clones, as indicated, and results were expressed as cpm. Controls indicated on the x axis were no antigen (No Ag) and B6/media; antigens used were p6-22 human (p1), p117-139 (p2) or p244-262 human (p3).

Figure 6A and 6B are histograms illustrating the stimulation of proliferative T cell responses in mice immunized with representative WT1 peptides. Three weeks after the third immunization, spleen cells of mice that had been inoculated with Vaccine A or Vaccine B were cultured with medium alone (medium) or spleen cells and medium (B6/no antigen), B6 spleen cells pulsed with the peptides p6-22 (p6), p117-139 (p117), p244-262 (p244) (Vaccine A; Figure 6A) or p287-301 (p287), p299-313 (p299), p421-435 (p421) (Vaccine B; Figure 6B) and spleen cells pulsed with an irrelevant control peptide (irrelevant peptide) at 25ug/ml and were assayed after 96hr for proliferation by ($^3$H) thymidine incorporation. Bars represent the stimulation index (SI), which is calculated as the mean of the experimental wells divided by the mean of the control (B6 spleen cells with no antigen).

Figures 7A-7D are histograms illustrating the generation of proliferative T-cell lines and clones specific for p117-139 and p6-22. Following *in vivo* immunization, the initial three *in vitro* stimulations (IVS) were carried out using all three peptides of Vaccine A or B, respectively. Subsequent IVS were carried out as single peptide stimulations using only the two relevant peptides p117-139 and p6-22. Clones were derived from both the p6-22 and p117-139 specific T cell lines, as indicated. T cells were cultured with medium alone (medium) or spleen cells and medium (B6/no antigen), B6 spleen cells pulsed with the peptides p6-22 (p6), p1 17-139 (p117) or an irrelevant control peptide (irrelevant peptide) at 25ug/ml and were assayed after 96hr for proliferation by ($^3$H) thymidine incorporation. Bars represent the stimulation index (SI), which is calculated as the mean of the experimental wells divided by the mean of the control (B6 spleen cells with no antigen).

Figures 8A and 8B present the results of TSITES Analysis of human WT1 (SEQ ID NO:319) for peptides that have the potential to elicit Th responses. Regions indicated by "A" are AMPHI midpoints of blocks, "R" indicates residues matching the Rothbard/'Taylor motif, "D" indicates residues matching the IAd motif, and 'd' indicates residues matching the IEd motif.

Figures 9A and 9B are graphs illustrating the elicitation of WT1 peptide-specific CTL in mice immunized with WT1 peptides. Figure 9A illustrates the lysis of target cells by allogeneic cell lines and Figure 9B shows the lysis of peptide coated cell lines. In each case, the % lysis (as determined by standard chromium release assays) is shown at three indicated effector:target ratios. Results are provided for lymphoma cells (LSTRA and E10), as well as E10 + p235-243 (E10+P235). E10 cells are also referred to herein as EL-4 cells.

Figures 10A-10D are graphs illustrating the elicitation of WT1 specific CTL, which kill WT1 positive tumor cell lines but do not kill WT1 negative cell lines, following vaccination of B6 mice with WT1 peptide P117. Figure 10A illustrates that T-cells of non-immunized B6 mice do not kill WT1 positive tumor cell lines. Figure 10B illustrates the lysis of the target cells by allogeneic cell lines. Figures 10C and 10D demonstrate the lysis of WT1 positive tumor cell lines, as compared to WT1 negative cell lines in two different experiments. In addition, Figures 10C and 10D show the lysis of peptide-coated cell lines (WT1 negative cell line E10 coated with the relevant WT1 peptide P117) In each case, the % lysis (as determined by standard chromium release assays) is shown at three indicated effector:target ratios. Results are provided for lymphoma cells (E10), prostate cancer cells (TRAMP-C), a transformed fibroblast cell line (BLK-SV40), as well as E10+p117.

Figures 11A and 11B are histograms illustrating the ability of representative peptide P117-139 specific CTL to lyse WT1 positive tumor cells. Three weeks after the third immunization, spleen cells of mice that had been inoculated with the peptides p235-243 or p117-139 were stimulated *in vitro* with the relevant peptide and tested for ability to lyse targets incubated with WT1 peptides as well as WT1 positive and negative tumor cells. The bars represent the mean % specific lysis in chromium release assays performed in triplicate with an E:T ratio of 25:1. Figure 11A shows the cytotoxic activity of the p235-243 specific T cell line against the WT1 negative cell line EL-4 (EL-4, WT1 negative); EL-4 pulsed with the relevant (used for immunization as well as for restimulation) peptide p235-243 (EL-4+p235); EL-4 pulsed with the irrelevant peptides p117-139 (EL-4+p117), p126-134 (EL-4+p126) or p130-138 (EL-4+p130) and the WT1 positive tumor cells BLK-SV40 (BLK-SV40, WT1 positive) and TRAMP-C (TRAMP-C, WT1 positive), as indicated. Figure 11B shows cytotoxic activity of the p117-139 specific T cell line against EL-4; EL-4 pulsed with the relevant peptide P117-139 (EL-4+p117) and EL-4 pulsed with the irrelevant peptides p123-131 (EL-4+p123), or p128-136 (EL-4+p128); BLK-SV40 and TRAMP-C, as indicated.

Figures 12A and 12B are histograms illustrating the specificity of lysis of WT1 positive tumor cells, as demonstrated by cold target inhibition. The bars represent the mean % specific lysis in chromium release assays performed in triplicate with an E:T ratio of 25:1. Figure 12A shows the cytotoxic activity of the p117-139 specific T cell line against the WT1 negative cell line EL-4 (EL-4, WT1 negative); the WT1 positive tumor cell line TRAMP-C (TRAMP-C, WT1 positive); TRAMP-C cells incubated with a ten-fold excess (compared to the hot target) of EL-4 cells pulsed with the relevant peptide p117-139 (TRAMP-C + p117 cold target) without $^{51}$Cr labeling and TRAMP-C cells incubated with EL-4 pulsed with an irrelevant peptide without $^{51}$Cr labeling (TRAMP-C + irrelevant cold target), as indicated. Figure 12B shows the cytotoxic activity of the p117-139 specific T cell line against the WT1 negative cell line EL-4 (EL-4, WT1 negative); the WT1 positive tumor cell line BLK-SV40 (BLK-SV40, WT1 positive); BLK-SV40 cells incubated with the relevant cold target (BLK-SV40 + p117 cold target) and BLK-SV40 cells incubated with the irrelevant cold target (BLK-SV40 + irrelevant cold target), as indicated.

Figures 13A-13C are histograms depicting an evaluation of the 9mer CTL epitope within p117-139. The p117-139 tumor specific CTL line was tested against peptides within aa 117-139 containing or lacking an appropriate H-2$^b$ class I binding motif and following restimulation with p126-134 or p130-138. The bars represent the mean % specific lysis in chromium release assays performed in triplicate with an E:T ratio of 25:1. Figure 13A shows the cytotoxic activity of the p117-139 specific T cell line against the WT1 negative cell line EL-4 (EL-4, WT1 negative) and EL-4 cells pulsed with the peptides p 117-13 9 (EL-4 + p117), p119-127 (EL-4 + p119), p120-128 (EL-4 + p120), p123-131 (EL-4 + p123), p126-134 (EL-4 + p126), p128-136 (EL-4 + p128), and p130-138 (EL-4 + p130). Figure 13B shows the cytotoxic activity of the CTL line after restimulation with p126-134 against the WT1 negative cell line EL-4, EL-4 cells pulsed with p117-139 (EL-4 + p117), p126-134 (EL-4 + p126) and the WT1 positive tumor cell line TRAMP-C. Figure 13C shows the cytotoxic activity of the CTL line after restimulation with p130-138 against EL-4, EL-4 cells pulsed with p117-139 (EL-4 + p117), p130-138 (EL-4 + p130) and the WT1 positive tumor cell line TRAMP-C.

DETAILED DESCRIPTION OF THE INVENTION

As noted above, the present invention is generally directed to compositions and methods for the immunotherapy and diagnosis of malignant diseases. The compositions described herein may include WT1 polypeptides of the present invention, WT1 polynucleotides, antigen-presenting cells (APC, e.g., dendritic cells) that express a WT1 polypeptide, agents such as antibodies that bind to a WT1 polypeptide and/or immune system cells (*e.g.*, T cells) specific for WT1. WT1 Polypeptides of the present invention generally comprise at least a portion of a Wilms Tumor gene product (WT1). Nucleic acid sequences of the subject invention generally comprise a DNA or RNA sequence that encodes all or a portion of such a polypeptide, or that is complementary to such a sequence. Antibodies are generally immune system proteins, or antigen-binding fragments thereof, that are capable of binding to a portion of a WT1 polypeptide. T cells that may be employed within such compositions are generally T cells (*e.g.*, $CD4^+$ and/or $CD8^+$) that are specific for a WT1 polypeptide. Certain methods described herein further employ antigen-presenting cells that express a WT1 polypeptide as provided herein.

The present invention is based on the discovery that an immune response raised against a Wilms Tumor (WT) gene product (*e.g.*, WT1) can provide prophylactic and/or therapeutic benefit for patients afflicted with malignant diseases characterized by increased WT1 gene expression. Such diseases include, but are not limited to, leukemias (*e.g.*, acute myeloid leukemia (AML), chronic myeloid leukemia (CML), acute lymphocytic leukemia (ALL) and childhood ALL), as well as many cancers such as lung, breast, thyroid and gastrointestinal cancers and melanomas. The WT1 gene was originally identified and isolated on the basis of a cytogenetic deletion at chromosome 11p13 in patients with Wilms' tumor *(see* Call et al., U.S. Patent No. 5,350,840). The gene consists of 10 exons and encodes a zinc finger transcription factor, and sequences of mouse and human WT1 proteins are provided in Figure 1 and SEQ ID NOs: 319 and 320.

WT1 POLYPEPTIDES

Within the context of the present invention, a WT1 polypeptide is a polypeptide that comprises at least an immunogenic portion of a native WT1 *(i.e.,* a WT1 protein expressed by an organism that is not genetically modified), or a variant thereof, as described herein. A WT1 polypeptide may be of any length, provided that it comprises at least an immunogenic portion of a native protein or a variant thereof. In other words, a WT1 polypeptide may be an oligopeptide (*i.e.,* consisting of a relatively small number of amino acid residues, such as 8-10 residues, joined by peptide bonds), a full length WT1 protein (e.g., present within a human or non-human animal, such as a mouse) or a polypeptide of intermediate size. Within certain embodiments, the use of WT1 polypeptides that contain a small number of consecutive amino acid residues of a native WT1 polypeptide is preferred. Such polypeptides are preferred for certain uses in which the generation of a T cell response is desired. For example, such a WT1 polypeptide may contain less than 23, preferably no more than 18, and more preferably no more than 15 consecutive amino acid residues, of a native WT1 polypeptide. Polypeptides comprising nine consecutive amino acid residues of a native WT1 polypeptide are generally suitable for such purposes. Additional sequences derived from the native protein and/or heterologous sequences may be present within any WT1 polypeptide, and such sequences may (but need not) possess further immunogenic or antigenic properties. Polypeptides as provided herein may further be associated (covalently or noncovalently) with other polypeptide or non-polypeptide compounds.

An "immunogenic portion," as used herein is a portion of a polypeptide that is recognized *(i.e.,* specifically bound) by a B-cell and/or T-cell surface antigen receptor. Certain preferred immunogenic portions bind to an MHC class I or class II molecule. As used herein, an immunogenic portion is said to "bind to" an MHC class I or class II molecule if such binding is detectable using any assay known in the art. For example, the ability of a polypeptide to bind to MHC class I may be evaluated indirectly by monitoring the ability to promote incorporation of $^{125}I$ labeled β2-microglobulin (β2m) into MHC class I/β2m/peptide heterotrimeric complexes *(see* Parker et al., J. Immunol. 152:163, 1994). Alternatively, functional peptide competition assays that are known in the art may be employed. Certain immunogenic portions have one or more of the sequences recited within one or more of Tables II - XIV. Representative immunogenic portions include, but are not limited to, RDLNALLPAVPSLGGGG (human WT1 residues 6-22; SEQ ID NO:1), PSQASSGQARMFPNAPYLPSCLE (human and mouse WT1 residues 117-139; SEQ ID NOs: 2 and 3 respectively), GATLKGVAAGSSSSVKWTE (human WT1 residues 244-262; SEQ ID NO:4), GATLKGVAA (human WT1 residues 244-252; SEQ ID NO: 88), CMTWNQMNL (human and mouse WT1 residues 235-243; SEQ ID NOs: 49 and 258 respectively), SCLESQPTI (mouse WT1 residues 136-144; SEQ ID NO:296), SCLESQPAI (human WT1 residues 136-144; SEQ ID NO:198), NLYQMTSQL (human and mouse WT1 residues 225-233; SEQ ID NOs: 147 and 284 respectively); ALLPAVSSL (mouse WT1 residues 10-18; SEQ ID NO:255); or RMFPNAPYL (human and mouse WT1 residues 126-134; SEQ ID NOs: 185 and 293 respectively). Further immunogenic portions are provided herein, and others may generally be identified using well known techniques, such as those summarized in Paul, *Fundamental Immunology,* 3rd ed., 243-247 (Raven Press, 1993) and references cited therein. Representative techniques for identifying immunogenic portions include screening polypeptides for the ability to react with antigen-specific antisera and/or T-cell lines or clones. An immunogenic portion of a native WT1 polypeptide is a portion that reacts with such antisera and/or T-cells at a level that is not substantially less than the reactivity of the full length WT1 (e.g., in an ELISA and/or T-cell reactivity assay). In other words, an immunogenic portion may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988.

Alternatively, immunogenic portions may be identified using computer analysis, such as the Tsites program *(see* Rothbard and Taylor, EMBO J. 7:93-100, 1988; Deavin et al., Mol. Immunol. 33:145-155, 1996), which searches for peptide motifs that have the potential to elicit Th responses. CTL peptides with motifs appropriate for binding to murine and human class I or class II MHC may be identified according to BIMAS (Parker et al., J Immunol. 152:163, 1994) and other HLA peptide binding prediction analyses. To confirm immunogenicity, a peptide may be tested using an HLA A2 transgenic mouse model and/or an *in vitro* stimulation assay using dendritic cells, fibroblasts or peripheral blood cells.

As noted above, a composition may comprise a variant of a native WT1 protein. A polypeptide "variant," as used herein, is a polypeptide that differs from a native polypeptide in one or more substitutions, deletions, additions and/or insertions, such that the immunogenicity of the polypeptide is retained (i.e., the ability of the variant to react with antigen-specific antisera and/or T-cell lines or clones is not substantially diminished relative to the native polypeptide). In other words, the ability of a variant to react with antigen-specific antisera and/or T-cell lines or clones may be enhanced or unchanged, relative to the native polypeptide, or may be diminished by less than 50%, and preferably less than 20%, relative to the native polypeptide. Such variants may generally be identified by modifying one of the above polypeptide sequences and evaluating the reactivity of the modified polypeptide with antisera and/or T-cells as described herein. It has been found, within the context of the present invention, that a relatively small number of substitutions (e.g., 1 to 3) within an immunogenic portion of a WT1 polypeptide may serve to enhance the ability of the polypeptide to elicit an immune response. Suitable substitutions may generally be identified by using computer programs, as described above, and the effect confirmed based on the reactivity of the modified polypeptide with antisera and/or T-cells as described herein. Accordingly, within certain preferred embodiments, a WT1 polypeptide comprises a variant in which 1 to 3 amino acid resides within an immunogenic portion are substituted such that the ability to react with antigen-specific antisera and/or T-cell lines or clones is statistically greater than that for the unmodified polypeptide. Such substitutions are preferably located within an MHC binding site of the polypeptide, which may be identified as described above. Preferred substitutions allow increased binding to MHC class I or class II molecules.

Certain variants contain conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. Variants may also (or alternatively) be modified by, for example, the deletion or addition of amino acids that have minimal influence on the immunogenicity, secondary structure and hydropathic nature of the polypeptide.

As noted above, WT1 polypeptides may be conjugated to a signal (or leader) sequence at the N-terminal end of the protein which co-translationally or post-translationally directs transfer of the protein. A polypeptide may also, or alternatively, be conjugated to a linker or other sequence for ease of synthesis, purification or identification of the polypeptide (*e.g.*, poly-His), or to enhance binding of the polypeptide to a solid support. For example, a polypeptide may be conjugated to an immunoglobulin Fc region.

WT1 polypeptides may be prepared using any of a variety of well known techniques. Recombinant polypeptides encoded by a WT1 polynucleotide as described herein may be readily prepared from the polynucleotide. In general, any of a variety of expression vectors known to those of ordinary skill in the art may be employed to express recombinant WT1 polypeptides. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line such as COS or CHO. Supernatants from suitable host/vector systems which secrete recombinant protein or polypeptide into culture media may be first concentrated using a commercially available filter. The concentrate may then be applied to a suitable purification matrix such as an affinity matrix or an ion exchange resin. Finally, one or more reverse phase HPLC steps can be employed to further purify a recombinant polypeptide. Such techniques may be used to prepare native polypeptides or variants thereof. For example, polynucleotides that encode a variant of a native polypeptide may generally be prepared using standard mutagenesis techniques, such as oligonucleotide-directed site-specific mutagenesis, and sections of the DNA sequence may be removed to permit preparation of truncated polypeptides.

Certain portions and other variants may also be generated by synthetic means, using techniques well known to those of ordinary skill in the art. For example, polypeptides having fewer than about 500 amino acids, preferably fewer than about 100 amino acids, and more preferably fewer than about 50 amino acids, may be synthesized. Polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Applied BioSystems, Inc. (Foster City, CA), and may be operated according to the manufacturer's instructions.

In general, polypeptides and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

Within further aspects, the present invention provides mimetics of WT1 polypeptides. Such mimetics may comprise amino acids linked to one or more amino acid mimetics (i.e., one or more amino acids within the WT1 protein may be replaced by an amino acid mimetic) or may be entirely nonpeptide mimetics. An amino acid mimetic is a compound that is conformationally similar to an amino acid such that it can be substituted for an amino acid within a WT1 polypeptide without substantially diminishing the ability to react with antigen-specific antisera and/or T cell lines or clones. A nonpeptide mimetic is a compound that does not contain amino acids, and that has an overall conformation that is similar to a WT1 polypeptide such that the ability of the mimetic to react with WT1-specific antisera and/or T cell lines or clones is not substantially diminished relative to the ability of a WT1 polypeptide. Such mimetics may be designed based on standard techniques (e.g., nuclear magnetic resonance and computational techniques) that evaluate the three dimensional structure of a peptide sequence. Mimetics may be designed where one or more of the side chain functionalities of the WT1 polypeptide are replaced by groups that do not necessarily have the same size or volume, but have similar chemical and/or physical properties which produce similar biological responses. It should be understood that, within embodiments described herein, a mimetic may be substituted for a WT1 polypeptide.

## WT1 POLYNUCLEOTIDES

Any polynucleotide that encodes a WT1 polypeptide as described herein is a WT1 polynucleotide encompassed by the present invention. Such polynucleotides may be single-stranded (coding or antisense) or double-stranded, and may be DNA (genomic, cDNA or synthetic) or RNA molecules. Additional coding or non-coding sequences may, but need not, be present within a polynucleotide of the present invention, and a polynucleotide may, but need not, be linked to other molecules and/or support materials.

WT1 polynucleotides may encode a native WT1 protein, or may encode a variant of WT1 as described herein. Polynucleotide variants may contain one or more substitutions, additions, deletions and/or insertions such that the immunogenicity of the encoded polypeptide is not diminished, relative to a native WT1 protein. The effect on the immunogenicity of the encoded polypeptide may generally be assessed as described herein. Preferred variants contain nucleotide substitutions, deletions, insertions and/or additions at no more than 20%, preferably at no more than 10%, of the nucleotide positions that encode an immunogenic portion of a native WT1 sequence. Certain variants are substantially homologous to a native gene, or a portion thereof. Such polynucleotide variants are capable of hybridizing under moderately stringent conditions to a naturally occurring DNA sequence encoding a WT1 polypeptide (or a complementary sequence). Suitable moderately stringent conditions include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1 % SDS). Such hybridizing DNA sequences are also within the scope of this invention.

It will be appreciated by those of ordinary skill in the art that, as a result of the degeneracy of the genetic code, there are many nucleotide sequences that encode a WT1 polypeptide. Some of these polynucleotides bear minimal homology to the nucleotide sequence of any native gene. Nonetheless, polynucleotides that vary due to differences in codon usage are specifically contemplated by the present invention.

Once an immunogenic portion of WT1 is identified, as described above, a WT1 polynucleotide may be prepared using any of a variety of techniques. For example, a WT1 polynucleotide may be amplified from cDNA prepared from cells that express WT1. Such polynucleotides may be amplified via polymerase chain reaction (PCR). For this approach, sequence-specific primers may be designed based on the sequence of the immunogenic portion and may be purchased or synthesized. For example, suitable primers for PCR amplification of a human WT1 gene include: first step - P118: 1434-1414: 5' GAG AGT CAG ACT TGA AAG CAGT 3' (SEQ ID NO:5) and P135: 5' CTG AGC CTC AGC AAA TGG GC 3' (SEQ ID NO:6); second step - P136: 5' GAG CAT GCA TGG GCT CCG ACG TGC GGG 3' (SEQ ID NO:7) and P137: 5' GGG GTA CCC ACT GAA CGG TCC CCG A 3' (SEQ ID NO:8). Primers for PCR amplification of a mouse WT1 gene include: first step - P138: 5' TCC GAG CCG CAC CTC ATG 3' (SEQ ID NO:9) and P139: 5' GCC TGG GAT GCT GGA CTG 3' (SEQ ID NO:10), second step - P140: 5' GAG CAT GCG ATG GGT TCC GAC GTG CGG 3' (SEQ ID NO:11) and P141: 5' GGG GTA CCT CAA AGC GCC ACG TGG AGT TT 3' (SEQ ID NO:12).

An amplified portion may then be used to isolate a full length gene from a human genomic DNA library or from a suitable cDNA library, using well known techniques. Alternatively, a full length gene can be constructed from multiple PCR fragments. WT1 polynucleotides may also be prepared by synthesizing oligonucleotide components, and ligating components together to generate the complete polynucleotide.

WT1 polynucleotides may also be synthesized by any method known in the art, including chemical synthesis (e.g., solid phase phosphoramidite chemical synthesis). Modifications in a polynucleotide sequence may also be introduced using standard mutagenesis techniques, such as oligonucleotide-directed site-specific mutagenesis (see Adelman et al., DNA 2:183, 1983). Alternatively, RNA molecules may be generated by *in vitro* or *in vivo* transcription of DNA sequences encoding a WT1 polypeptide, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo (e.g.,* by transfecting antigen-presenting cells such as dendritic cells with a cDNA construct encoding a WT1 polypeptide, and administering the transfected cells to the patient).

Polynucleotides that encode a WT1 polypeptide may generally be used for production of the polypeptide, *in vitro* or *in vivo.* WT1 polynucleotides that are complementary to a coding sequence *(i.e.,* antisense polynucleotides) may also be used as a probe or to inhibit WT1 expression. cDNA constructs that can be transcribed into antisense RNA may also be introduced into cells of tissues to facilitate the production of antisense RNA.

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (*e.g.*, avian pox virus). Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art. cDNA constructs within such a vector may be used, for example, to transfect human or animal cell lines for use in establishing WT1 positive tumor models which may be used to perform tumor protection and adoptive immunotherapy experiments to demonstrate tumor or leukemia-growth inhibition or lysis of such cells.

Other therapeutic formulations for polynucleotides include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome *(i.e.,* an artificial membrane vesicle). The preparation and use of such systems is well known in the art.

## ANTIBODIES AND FRAGMENTS THEREOF

The present invention further provides binding agents, such as antibodies and antigen-binding fragments thereof, that specifically bind to a WT1 polypeptide. As used herein, an agent is said to "specifically bind" to a WT1 polypeptide if it reacts at a detectable level (within, for example, an ELISA) with a WT1 polypeptide, and does not react detectably with unrelated proteins under similar conditions. As used herein, "binding" refers to a noncovalent association between two separate molecules such that a "complex" is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about $10^3$ L/mol. The binding constant maybe determined using methods well known in the art.

Any agent that satisfies the above requirements may be a binding agent. In a preferred embodiment, a binding agent is an antibody or an antigen-binding fragment thereof. Certain antibodies are commercially available from, for example, Santa Cruz Biotechnology (Santa Cruz, CA). Alternatively, antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (e.g., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e.*, reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies and fragments thereof may be coupled to one or more therapeutic agents. Suitable agents in this regard include radioactive tracers and chemotherapeutic agents, which may be used, for example, to purge autologous bone marrow *in vitro).* Representative therapeutic agents include radionuclides, differentiation inducers, drugs, toxins, and derivatives thereof. Preferred radionuclides include $^{90}$Y, $^{123}$I, $^{125}$I, $^{131}$I, $^{186}$Re, $^{188}$Re, $^{211}$ At, and $^{212}$Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein. For diagnostic purposes, coupling of radioactive agents may be used to facilitate tracing of metastases or to determine the location of WT1-positive tumors.

A therapeutic agent may be coupled (e.g., covalently bonded) to a suitable monoclonal antibody either directly or indirectly (e.g., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, e.g., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g.*, U.S. Patent No. 4,489,710, to Spitler), by irradiation of a photolabile bond (*e.g.*, U.S. Patent No. 4,625,014, to Senter et al.), by hydrolysis of derivatized amino acid side chains (*e.g.*, U.S. Patent No. 4,638,045, to Kohn et al.), by serum complement-mediated hydrolysis (*e.g.*, U.S. Patent No. 4,671,958, to Rodwell et al.), and acid-catalyzed hydrolysis (*e.g.*, U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used. A carrier may bear the agents in a variety of ways, including covalent bonding either directly or via a linker group. Suitable carriers include proteins such as albumins (*e.g.*, U.S. Patent No. 4,507,234, to Kato et al.), peptides and polysaccharides such as aminodextran (*e.g.*, U.S. Patent No. 4,699,784, to Shih et al.). A carrier may also bear an agent by noncovalent bonding or by encapsulation, such as within a liposome vesicle (*e.g.*, U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

Also provided herein are anti-idiotypic antibodies that mimic an immunogenic portion of WT1. Such antibodies may be raised against an antibody, or antigen-binding fragment thereof, that specifically binds to an immunogenic portion of WT1, using well known techniques. Anti-idiotypic antibodies that mimic an immunogenic portion of WT1 are those antibodies that bind to an antibody, or antigen-binding fragment thereof, that specifically binds to an immunogenic portion of WT1, as described herein.

T CELLS

Immunotherapeutic compositions may also, or alternatively, comprise T cells specific for WT1. Such cells may generally be prepared *in vitro* or *ex vivo,* using standard procedures. For example, T cells may be present within (or isolated from) bone marrow, peripheral blood or a fraction of bone marrow or peripheral blood of a mammal, such as a patient, using a commercially available cell separation system, such as the CEPRATE™ system, available from CellPro Inc., Bothell WA (see also U.S. Patent No. 5,240,856; U.S. Patent No. 5,215,926; WO 89/06280; WO 91/16116 and WO 92/07243). Alternatively, T cells may be derived from related or unrelated humans, non-human animals, cell lines or cultures.

T cells may be stimulated with WT1 polypeptide, polynucleotide encoding a WT1 polypeptide and/or an antigen presenting cell (APC) that expresses a WT1 polypeptide. Such stimulation is performed under conditions and for a time sufficient to permit the generation of T cells that are specific for the WT1 polypeptide. Preferably, a WT1 polypeptide or polynucleotide is present within a delivery vehicle, such as a microsphere, to facilitate the generation of antigen-specific T cells. Briefly, T cells, which may be isolated from a patient or a related or unrelated donor by routine techniques (such as by Ficoll/Hypaque density gradient centrifugation of peripheral blood lymphocytes), are incubated with WT1 polypeptide. For example, T cells may be incubated *in vitro* for 2-9 days (typically 4 days) at 37°C with WT1 polypeptide *(e.g.,* 5 to 25 μg/ml) or cells synthesizing a comparable amount of WT1 polypeptide. It may be desirable to incubate a separate aliquot of a T cell sample in the absence of WT1 polypeptide to serve as a control.

T cells are considered to be specific for a WT1 polypeptide if the T cells kill target cells coated with a WT1 polypeptide or expressing a gene encoding such a polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (*e.g.*, by pulse-labeling cultures of T cells with tritiated thymidine and measuring the amount of tritiated thymidine incorporated into DNA). Other ways to detect T cell proliferation include measuring increases in interleukin-2 (IL-2) production, $Ca^{2+}$ flux, or dye uptake, such as 3-(4,5-dimethylthiazol-2-yl)-2,5-diphenyl-tetrazolium. Alternatively, synthesis of lymphokines (such as interferon-gamma) can be measured or the relative number of T cells that can respond to a WT1 polypeptide may be quantified. Contact with a WT1 polypeptide (200 ng/ml - 100 μg/ml, preferably 100 ng/ml - 25 μg/ml) for 3 - 7 days should result in at least a two fold increase in proliferation of the T cells and/or contact as described above for 2-3 hours should result in activation of the T cells, as measured using standard cytokine assays in which a two fold increase in the level of cytokine release (e.g., TNF or IFN-γ) is indicative of T cell activation *(see* Coligan et al., Current Protocols in Immunology, vol. 1, Wiley Interscience (Greene 1998). WT1 specific T cells may be expanded using standard techniques. Within preferred embodiments, the T cells are derived from a patient or a related or unrelated donor and are administered to the patient following stimulation and expansion.

T cells that have been activated in response to a WT1 polypeptide, polynucleotide or WT1-expressing APC may be $CD4^+$ and/or $CD8^+$. Specific activation of $CD4^+$ or $CD8^+$ T cells may be detected in a variety of ways. Methods for detecting specific T cell activation include detecting the proliferation of T cells, the production of cytokines (e.g., lymphokines), or the generation of cytolytic activity (i.e., generation of cytotoxic T cells specific for WT1). For $CD4^+$ T cells, a preferred method for detecting specific T cell activation is the detection of the proliferation of T cells. For $CD8^+$ T cells, a preferred method for detecting specific T cell activation is the detection of the generation of cytolytic activity.

For therapeutic purposes, $CD4^+$ or $CD8^+$ T cells that proliferate in response to the WT1 polypeptide, polynucleotide or APC can be expanded in number either *in vitro* or *in vivo.* Proliferation of such T cells *in vitro* may be accomplished in a variety of ways. For example, the T cells can be re-exposed to WT1 polypeptide, with or without the addition of T cell growth factors, such as interleukin-2, and/or stimulator cells that synthesize a WT1 polypeptide. The addition of stimulator cells is preferred where generating $CD8^+$ T cell responses. T cells can be grown to large numbers in *vitro* with retention of specificity in response to intermittent restimulation with WT1 polypeptide. Briefly, for the primary *in vitro* stimulation (IVS), large numbers of lymphocytes (e.g., greater than 4 x $10^7$) may be placed in flasks with media containing human serum. WT1 polypeptide (e.g., peptide at 10 μg/ml) may be added directly, along with tetanus toxoid *(e.g.,* 5 μg/ml). The flasks may then be incubated (e.g., 37°C for 7 days). For a second IVS, T cells are then harvested and placed in new flasks with 2-3 x $10^7$ irradiated peripheral blood mononuclear cells. WT1 polypeptide (e.g., 10 μg/ml) is added directly. The flasks are incubated at 37°C for 7 days. On day 2 and day 4 after the second IVS, 2-5 units of interleukin-2 (IL-2) may be added. For a third IVS, the T cells may be placed in wells and stimulated with the individual's own EBV transformed B cells coated with the peptide. IL-2 may be added on days 2 and 4 of each cycle. As soon as the cells are shown to be specific cytotoxic T cells, they may be expanded using a 10 day stimulation cycle with higher IL-2 (20 units) on days 2, 4 and 6.

Alternatively, one or more T cells that proliferate in the presence of WT1 polypeptide can be expanded in number by cloning. Methods for cloning cells are well known in the art, and include limiting dilution. Responder T cells may be purified from the peripheral blood of sensitized patients by density gradient centrifugation and sheep red cell rosetting and established in culture by stimulating with the nominal antigen in the presence of irradiated autologous filler cells. In order to generate $CD4^+$ T cell lines, WT1 polypeptide is used as the antigenic stimulus and autologous peripheral blood lymphocytes (PBL) or lymphoblastoid cell lines (LCL) immortalized by infection with Epstein Barr virus are used as antigen presenting cells. In order to generate $CD8^+$ T cell lines, autologous antigen-presenting cells transfected with an expression vector which produces WT1 polypeptide may be used as stimulator cells. Established T cell lines may be cloned 2-4 days following antigen stimulation by plating stimulated T cells at a frequency of 0.5 cells per well in 96-well flat-bottom plates with 1 x $10^6$ irradiated PBL or LCL cells and recombinant interleukin-2 (rIL2) (50 U/ml). Wells with established clonal growth may be identified at approximately 2-3 weeks after initial plating and restimulated with appropriate antigen in the presence of autologous antigen-presenting cells, then subsequently expanded by the addition of low doses of rIL2 (10 U/ml) 2-3 days following antigen stimulation. T cell clones may be maintained in 24-well plates by periodic restimulation with antigen and rIL2 approximately every two weeks.

Within certain embodiments, allogeneic T-cells may be primed (i.e., sensitized to WT1) *in vivo* and/or *in vitro.* Such priming may be achieved by contacting T cells with a WT1 polypeptide, a polynucleotide encoding such a polypeptide or a cell producing such a polypeptide under conditions and for a time sufficient to permit the priming of T cells. In general, T cells are considered to be primed if, for example, contact with a WT1 polypeptide results in proliferation and/or activation of the T cells, as measured by standard proliferation, chromium release and/or cytokine release assays as described herein. A stimulation index of more than two fold increase in proliferation or lysis, and more than three fold increase in the level of cytokine, compared to negative controls, indicates T-cell specificity. Cells primed *in vitro* may be employed, for example, within a bone marrow transplantation or as donor lymphocyte infusion.

PHARMACEUTICAL COMPOSITIONS AND VACCINES

Within certain aspects, polypeptides, polynucleotides, antibodies and/or T cells may be incorporated into pharmaceutical compositions or vaccines. Alternatively, a pharmaceutical composition may comprise an antigen-presenting cell (e.g., a dendritic cell) transfected with a WT1 polynucleotide such that the antigen presenting cell expresses a WT1 polypeptide. Pharmaceutical compositions comprise one or more such compounds or cells and a physiologically acceptable carrier or excipient. Certain vaccines may comprise one or more such compounds or cells and a non-specific immune response enhancer, such as an adjuvant or a liposome (into which the compound is incorporated). Pharmaceutical compositions and vaccines may additionally contain a delivery system, such as biodegradable microspheres which are disclosed, for example, in U.S. Patent Nos. 4,897,268 and 5,075,109. Pharmaceutical compositions and vaccines within the scope of the present invention may also contain other compounds, which may be biologically active or inactive.

Within certain embodiments, pharmaceutical compositions and vaccines are designed to elicit T cell responses specific for a WT1 polypeptide in a patient, such as a human. In general, T cell responses may be favored through the use of relatively short polypeptides (*e.g.*, comprising less than 23 consecutive amino acid residues of a native WT1 polypeptide, preferably 4-16 consecutive residues, more preferably 8-16 consecutive residues and still more preferably 8-10 consecutive residues. Alternatively, or in addition, a vaccine may comprise a non-specific immune response enhancer that preferentially enhances a T cell response. In other words, the immune response enhancer may enhance the level of a T cell response to a WT1 polypeptide by an amount that is proportionally greater than the amount by which an antibody response is enhanced. For example, when compared to a standard oil based adjuvant, such as CFA, an immune response enhancer that preferentially enhances a T cell response may enhance a proliferative T cell response by at least two fold, a lytic response by at least 10%, and/or T cell activation by at least two fold compared to WT1-megative control cell lines, while not detectably enhancing an antibody response. The amount by which a T cell or antibody response to a WT1 polypeptide is enhanced may generally be determined using any representative technique known in the art, such as the techniques provided herein.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacterial and viral expression systems and mammalian expression systems. Appropriate nucleic acid expression systems contain the necessary DNA, cDNA or RNA sequences for expression in the patient (such as a suitable promoter and terminating signal). Bacterial delivery systems involve the administration of a bacterium (such as *Bacillus-Calmette-Guerrin)* that expresses an immunogenic portion of the polypeptide on its cell surface. In a preferred embodiment, the DNA may be introduced using a viral expression system (*e.g.*, vaccinia or other pox virus, retrovirus, or adenovirus), which may involve the use of a non-pathogenic (defective), replication competent virus. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

As noted above, a pharmaceutical composition or vaccine may comprise an antigen-presenting cell that expresses a WT1 polypeptide. For therapeutic purposes, as described herein, the antigen presenting cell is preferably an autologous dendritic cell. Such cells may be prepared and transfected using standard techniques, such as those described by Reeves et al., Cancer Res. 56:5672-5677, 1996; Tuting et al., J. Immunol. 160:1139-1147, 1998; and Nair et al., Nature Biotechnol. 16:364-369, 1998). Expression of a WT1 polypeptide on the surface of an antigen-presenting cell may be confirmed by *in vitro* stimulation and standard proliferation as well as chromium release assays, as described herein.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g., polylactate polyglycolate) may also be employed as carriers for the pharmaceutical compositions of this invention. For certain topical applications, formulation as a cream or lotion, using well known components, is preferred.

Such compositions may also comprise buffers (e.g., neutral buffered saline or phosphate buffered saline), carbohydrates (e.g., glucose, mannose, sucrose or dextrans), mannitol, proteins, polypeptides or amino acids such as glycine, antioxidants, chelating agents such as EDTA or glutathione, adjuvants (e.g., aluminum hydroxide) and/or preservatives. Alternatively, compositions of the present invention may be formulated as a lyophilizate. Compounds may also be encapsulated within liposomes using well known technology.

Any of a variety of non-specific immune response enhancers, such as adjuvants, may be employed in the vaccines of this invention. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable non-specific immune response enhancers include alum-based adjuvants (e.g., Alhydrogel, Rehydragel, aluminum phosphate, Algammulin, aluminum hydroxide); oil based adjuvants (Freund's adjuvant (FA), Specol, RIBI, TiterMax, Montanide ISA50 or Seppic MONTANIDE ISA 720; cytokines (e.g., GM-CSF or Flat3-ligand); microspheres; nonionic block copolymer-based adjuvants; dimethyl dioctadecyl ammoniumbromide (DDA) based adjuvants AS-1, AS-2 (Smith Kline Beecham); Ribi Adjuvant system based adjuvants; QS21 (Aquila); saponin based adjuvants (crude saponin, the saponin Quil A ); muramyl dipeptide (MDP) based adjuvants such as SAF (Syntex adjuvant in its microfluidized form (SAF-m)); dimethyl-dioctadecyl ammonium bromide (DDA); human complement based adjuvants *m. vaccae* and derivatives; immune stimulating complex (iscom) based adjuvants; inactivated toxins; and attenuated infectious agents (such as *M. tuberculosis).*

As noted above, within certain embodiments, immune response enhancers are chosen for their ability to preferentially elicit or enhance a T cell response (e.g., $CD4^+$ and/or $CD8^+$) to a WT1 polypeptide. Such immune response enhancers are well known in the art, and include (but are not limited to) Montanide ISA50, Seppic MONTANIDE ISA 720, cytokines (e.g., GM-CSF, Flat3-ligand), microspheres, dimethyl dioctadecyl ammoniumbromide (DDA) based adjuvants, AS-1 (Smith Kline Beecham), AS-2 (Smith Kline Beecham), Ribi Adjuvant system based adjuvants, QS21 (Aquila), saponin based adjuvants (crude saponin, the saponin Quil A), Syntex adjuvant in its microfluidized form (SAF-m), MV, ddMV (Genesis), immune stimulating complex (iscom) based adjuvants and inactivated toxins.

The compositions and vaccines described herein may be administered as part of a sustained release formulation (i.e., a formulation such as a capsule or sponge that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide, antibody or cell dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

## THERAPY OF MALIGNANT DISEASES

In further aspects of the present invention, the compositions and vaccines described herein may be used to inhibit the development of malignant diseases (e.g., progressive or metastatic diseases or diseases characterized by small tumor burden such as minimal residual disease). In general, such methods may be used to prevent, delay or treat a disease associated with WT1 expression. In other words, therapeutic methods provided herein may be used to treat an existing WT1-associated disease, or may be used to prevent or delay the onset of such a disease in a patient who is free of disease or who is afflicted with a disease that is not yet associated with WT1 expression.

As used herein, a disease is "associated with WT1 expression" if diseased cells (e.g., tumor cells) at some time during the course of the disease generate detectably higher levels of a WT1 polypeptide than normal cells of the same tissue. Association of WT1 expression with a malignant disease does not require that WT1 be present on a tumor. For example, overexpression of WT1 may be involved with initiation of a tumor, but the protein expression may subsequently be lost. Alternatively, a malignant disease that is not characterized by an increase in WT1 expression may, at a later time, progress to a disease that is characterized by increased WT1 expression. Accordingly, any malignant disease in which diseased cells formerly expressed, currently express or are expected to subsequently express increased levels of WT1 is considered to be "associated with WT1 expression."

Immunotherapy may be performed using any of a variety of techniques, in which compounds or cells provided herein function to remove WT1-expressing cells from a patient. Such removal may take place as a result of enhancing or inducing an immune response in a patient specific for WT1 or a cell expressing WT1. Alternatively, WT1-expressing cells may be removed *ex vivo (e.g.,* by treatment of autologous bone marrow, peripheral blood or a fraction of bone marrow or peripheral blood). Fractions of bone marrow or peripheral blood may be obtained using any standard technique in the art.

Within such methods, pharmaceutical compositions and vaccines may be administered to a patient. As used herein, a "patient" refers to any warm-blooded animal, preferably a human. A patient may or may not be afflicted with a malignant disease. Accordingly, the above pharmaceutical compositions and vaccines may be used to prevent the onset of a disease *(i.e.,* prophylactically) or to treat a patient afflicted with a disease (e.g., to prevent or delay progression and/or metastasis of an existing disease). A patient afflicted with a disease may have a minimal residual disease (e.g.,

a low tumor burden in a leukemia patient in complete or partial remission or a cancer patient following reduction of the tumor burden after surgery radiotherapy and/or chemotherapy). Such a patient may be immunized to inhibit a relapse *(i.e.,* prevent or delay the relapse, or decrease the severity of a relapse). Within certain preferred embodiments, the patient is afflicted with a leukemia (e.g., AML, CML, ALL or childhood ALL), a myelodysplastic syndrome (MDS) or a cancer (e.g., gastrointestinal, lung, thyroid or breast cancer or a melanoma), where the cancer or leukemia is WT1 positive *(i.e.,* reacts detectably with an anti-WT1 antibody, as provided herein or expresses WT1 mRNA at a level detectable by RT-PCR, as described herein) or suffers from an autoimmune disease directed against WT1-expressing cells.

The compositions provided herein may be used alone or in combination with conventional therapeutic regimens such as surgery, irradiation, chemotherapy and/or bone marrow transplantation (autologous, syngeneic, allogeneic or unrelated). As discussed in greater detail below, binding agents and T cells as provided herein may be used for purging of autologous stem cells. Such purging may be beneficial prior to, for example, bone marrow transplantation or transfusion of blood or components thereof. Binding agents, T cells, antigen presenting cells (APC) and compositions provided herein may further be used for expanding and stimulating (or priming) autologous, allogeneic, syngeneic or unrelated WT1-specific T-cells *in vitro* and/or *in vivo.* Such WT1-specific T cells may be used, for example, within donor lymphocyte infusions.

Routes and frequency of administration, as well as dosage, will vary from individual to individual, and may be readily established using standard techniques. In general, the pharmaceutical compositions and vaccines may be administered by injection (*e.g.*, intracutaneous, intramuscular, intravenous or subcutaneous), intranasally (*e.g.*, by aspiration) or orally. In some tumors, pharmaceutical compositions or vaccines may be administered locally (by, for example, rectocoloscopy, gastroscopy, videoendoscopy, angiography or other methods known in the art). Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response that is at least 10-50% above the basal (*i.e.*, untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g.*, more frequent complete or partial remissions, or longer disease-free and/or overall survival) in vaccinated patients as compared to non-vaccinated patients. In general, for pharmaceutical compositions and vaccines comprising one or more polypeptides, the amount of each polypeptide present in a dose ranges from about 100 $\mu$g to 5 mg. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (*e.g.*, more frequent complete or partial remissions, or longer disease-free and/or overall survival) in treated patients as compared to non-treated patients. Increases in preexisting immune responses to WT1 generally correlate with an improved clinical outcome. Such immune responses may generally be evaluated using standard proliferation, cytotoxicity or cytokine assays, which may be performed using samples obtained from a patient before and after treatment.

Within further aspects, methods for inhibiting the development of a malignant disease associated with WT1 expression involve the administration of autologous T cells that have been activated in response to a WT1 polypeptide or WT1-expressing APC, as described above. Such T cells may be $CD4^+$ and/or $CD8^+$, and may be proliferated as described above. The T cells may be administered to the individual in an amount effective to inhibit the development of a malignant disease. Typically, about 1 x $10^9$ to 1 x $10^{11}$ T cells/$M^2$ are administered intravenously, intracavitary or in the bed of a resected tumor. It will be evident to those skilled in the art that the number of cells and the frequency of administration will be dependent upon the response of the patient.

Within certain embodiments, T cells may be stimulated prior to an autologous bone marrow transplantation. Such stimulation may take place *in vivo* or *in vitro.* For *in vitro* stimulation, bone marrow and/or peripheral blood (or a fraction of bone marrow or peripheral blood) obtained from a patient may be contacted with a WT1 polypeptide, a polynucleotide encoding a WT1 polypeptide and/or an APC that expresses a WT1 polypeptide under conditions and for a time sufficient to permit the stimulation of T cells as described above. Bone marrow, peripheral blood stem cells and/or WT1-specific T cells may then be administered to a patient using standard techniques.

Within related embodiments, T cells of a related or unrelated donor may be stimulated prior to a syngeneic or allogeneic (related or unrelated) bone marrow transplantation. Such stimulation may take place *in vivo* or *in vitro.* For *in vitro* stimulation, bone marrow and/or peripheral blood (or a fraction of bone marrow or peripheral blood) obtained from a related or unrelated donor may be contacted with a WT1 polypeptide, WT1 polynucleotide and/or APC that expresses a WT1 polypeptide under conditions and for a time sufficient to permit the stimulation of T cells as described above. Bone marrow, peripheral blood stem cells and/or WT1-specific T cells may then be administered to a patient using standard techniques.

Within other embodiments, WT1-specific T cells as described herein may be used to remove cells expressing WT1 from autologous bone marrow, peripheral blood or a fraction of bone marrow or peripheral blood (e.g., $CD34^+$ enriched peripheral blood (PB) prior to administration to a patient). Such methods may be performed by contacting bone marrow or PB with such T cells under conditions and for a time sufficient to permit the reduction of WT1 expressing cells to less than 10%, preferably less than 5% and more preferably less than 1 %, of the total number of myeloid or lymphatic cells in the bone marrow or peripheral blood. The extent to which such cells have been removed may be readily determined by standard methods such as, for example, qualitative and quantitative PCR analysis, morphology, immunohistochemistry and FACS analysis. Bone marrow or PB (or a fraction thereof) may then be administered to a patient using standard techniques.

## DIAGNOSTIC METHODS

The present invention further provides methods for detecting a malignant disease associated with WT1 expression, and for monitoring the effectiveness of an immunization or therapy for such a disease. Such methods are based on the discovery, within the present invention, that an immune response specific for WT1 protein can be detected in patients afflicted with such diseases, and that methods which enhance such immune responses may provide a preventive or therapeutic benefit.

To determine the presence or absence of a malignant disease associated with WT1 expression, a patient may be tested for the level of T cells specific for WT1. Within certain methods, a biological sample comprising $CD4^+$ and/or $CD8^+$ T cells isolated from a patient is incubated with a WT1 polypeptide, a polynucleotide encoding a WT1 polypeptide and/or an APC that expresses a WT1 polypeptide, and the presence or absence of specific activation of the T cells is detected, as described herein. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated in *vitro* for 2-9 days (typically 4 days) at 37°C with WT1 polypeptide *(e.g.,* 5 - 25 μg/ml). It may be desirable to incubate another aliquot of a T cell sample in the absence of WT1 polypeptide to serve as a control. For $CD4^-$ T cells, activation is preferably detected by evaluating proliferation of the T cells. For $CD8^+$ T cells, activation is preferably detected by evaluating cytolytic activity. A level of proliferation that is at least two fold greater and/or a level of cytolytic activity that is at least 20% greater than in disease-free patients indicates the presence of a malignant disease associated with WT1 expression. Further correlation may be made, using methods well known in the art, between the level of proliferation and/or cytolytic activity and the predicted response to therapy. In particular, patients that display a higher antibody, proliferative and/or lytic response may be expected to show a greater response to therapy.

Within other methods, a biological sample obtained from a patient is tested for the level of antibody specific for WT1. The biological sample is incubated with a WT1 polypeptide, a polynucleotide encoding a WT1 polypeptide and/or an APC that expresses a WT1 polypeptide under conditions and for a time sufficient to allow immunocomplexes to form. Immunocomplexes formed between the WT1 polypeptide and antibodies in the biological sample that specifically bind to the WT1 polypeptide are then detected. A biological sample for use within such methods may be any sample obtained from a patient that would be expected to contain antibodies. Suitable biological samples include blood, sera, ascites, bone marrow, pleural effusion, and cerebrospinal fluid.

The biological sample is incubated with the WT1 polypeptide in a reaction mixture under conditions and for a time sufficient to permit immunocomplexes to form between the polypeptide and antibodies specific for WT1. For example, a biological sample and WT1 polypeptide may be incubated at 4°C for 24-48 hours.

Following the incubation, the reaction mixture is tested for the presence of immunocomplexes. Detection of immunocomplexes formed between the WT1 polypeptide and antibodies present in the biological sample may be accomplished by a variety of known techniques, such as radioimmunoassays (RIA) and enzyme linked immunosorbent assays (ELISA). Suitable assays are well known in the art and are amply described in the scientific and patent literature (*e.g.*, Harlow and Lane, Antibodies: A Laboratory Manual, Cold Spring Harbor Laboratory, 1988). Assays that may be used include, but are not limited to, the double monoclonal antibody sandwich immunoassay technique of David et al. (U.S. Patent 4,376,110); monoclonal-polyclonal antibody sandwich assays (Wide et al., in Kirkham and Hunter, eds., Radioimmunoassay Methods, E. and S. Livingstone, Edinburgh, 1970); the "western blot" method of Gordon et al. (U.S. Patent 4,452,901); immunoprecipitation of labeled ligand (Brown et al., J. Biol. Chem. 255:4980-4983, 1980); enzyme-linked immunosorbent assays as described by, for example, Raines and Ross (J. Biol. Chem. 257:5154-5160, 1982); immunocytochemical techniques, including the use of fluorochromes (Brooks et al., Clin. Exp. Immunol. 39: 477, 1980); and neutralization of activity (Bowen-Pope et al., Proc. Natl. Acad. Sci. USA 81:2396-2400, 1984). Other immunoassays include, but are not limited to, those described in U.S. Patent Nos.: 3,817,827; 3,850,752; 3,901,654; 3,935,074; 3,984,533; 3,996,345; 4,034,074; and 4,098,876.

For detection purposes, WT1 polypeptide may either be labeled or unlabeled. Unlabeled WT1 polypeptide may be used in agglutination assays or in combination with labeled detection reagents that bind to the immunocomplexes (e.g., anti-immunoglobulin, protein G, protein A or a lectin and secondary antibodies, or antigen-binding fragments thereof, capable of binding to the antibodies that specifically bind to the WT1 polypeptide). If the WT1 polypeptide is labeled, the reporter group may be any suitable reporter group known in the art, including radioisotopes, fluorescent groups, luminescent groups, enzymes, biotin and dye particles.

Within certain assays, unlabeled WT1 polypeptide is immobilized on a solid support. The solid support may be any material known to those of ordinary skill in the art to which the polypeptide may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The polypeptide may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the WT1 polypeptide, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of polypeptide ranging from about 10 ng to about 10 μg, and preferably about 100 ng to about 1 μg, is sufficient to immobilize an adequate amount of polypeptide.

Following immobilization, the remaining protein binding sites on the support are typically blocked. Any suitable blocking agent known to those of ordinary skill in the art, such as bovine serum albumin, Tween 20™ (Sigma Chemical Co., St. Louis, MO), heat-inactivated normal goat serum (NGS), or BLOTTO (buffered solution of nonfat dry milk which also contains a preservative, salts, and an antifoaming agent). The support is then incubated with a biological sample suspected of containing specific antibody. The sample can be applied neat, or, more often, it can be diluted, usually in a buffered solution which contains a small amount (0.1%-5.0% by weight) of protein, such as BSA, NGS, or BLOTTO. In general, an appropriate contact time (i.e., incubation time) is a period of time that is sufficient to detect the presence of antibody that specifically binds WT1 within a sample containing such an antibody. Preferably, the contact time is sufficient to achieve a level of binding that is at least about 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. A detection reagent that binds to the immunocomplexes and that comprises a reporter group may then be added. The detection reagent is incubated with the immunocomplex for an amount of time sufficient to detect the bound antibody. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups (e.g., horseradish peroxidase, beta-galactosidase, alkaline phosphatase and glucose oxidase) may generally be detected by the addition of substrate (generally for a specific period of time), followed by spectroscopic or other analysis of the reaction products. Regardless of the specific method employed, a level of bound detection reagent that is at least two fold greater than background (i.e., the level observed for a biological sample obtained from a disease-free individual) indicates the presence of a malignant disease associated with WT1 expression.

In general, methods for monitoring the effectiveness of an immunization or therapy involve monitoring changes in the level of antibodies or T cells specific for WT1 in the patient. Methods in which antibody levels are monitored may comprise the steps of: (a) incubating a first biological sample, obtained from a patient prior to a therapy or immunization, with a WT1 polypeptide, wherein the incubation is performed under conditions and for a time sufficient to allow immunocomplexes to form; (b) detecting immunocomplexes formed between the WT1 polypeptide and antibodies in the biological sample that specifically bind to the WT1 polypeptide; (c) repeating steps (a) and (b) using a second biological sample taken from the patient following therapy or immunization; and (d) comparing the number of immunocomplexes detected in the first and second biological samples. Alternatively, a polynucleotide encoding a WT1 polypeptide, or an APC expressing a WT1 polypeptide may be employed in place of the WT1 polypeptide. Within such methods, immunocomplexes between the WT1 polypeptide encoded by the polynucleotide, or expressed by the APC, and antibodies in the biological sample are detected.

Methods in which T cell activation and/or the number of WT1 specific precursors are monitored may comprise the steps of (a) incubating a first biological sample comprising CD4+ and/or CD8+ cells (e.g., bone marrow, peripheral blood or a fraction thereof), obtained from a patient prior to a therapy or immunization, with a WT1 polypeptide, wherein the incubation is performed under conditions and for a time sufficient to allow specific activation, proliferation and/or lysis of T cells; (b) detecting an amount of activation, proliferation and/or lysis of the T cells; (c) repeating steps (a) and (b) using a second biological sample comprising CD4+ and/or CD8+ T cells, and taken from the same patient following therapy or immunization; and (d) comparing the amount of activation, proliferation and/or lysis of T cells in the first and second biological samples. Alternatively, a polynucleotide encoding a WT1 polypeptide, or an APC expressing a WT1 polypeptide may be employed in place of the WT1 polypeptide.

A biological sample for use within such methods may be any sample obtained from a patient that would be expected to contain antibodies, CD4+ T cells and/or CD8+ T cells. Suitable biological samples include blood, sera, ascites, bone marrow, pleural effusion and cerebrospinal fluid. A first biological sample may be obtained prior to initiation of therapy or immunization or part way through a therapy or vaccination regime. The second biological sample should be obtained in a similar manner, but at a time following additional therapy or immunization. The second biological sample may be obtained at the completion of, or part way through, therapy or immunization, provided that at least a portion of therapy or immunization takes place between the isolation of the first and second biological samples.

Incubation and detection steps for both samples may generally be performed as described above. A statistically significant increase in the number of immunocomplexes in the second sample relative to the first sample reflects successful therapy or immunization.

The following Examples are offered by way of illustration and not by way of limitation.

## EXAMPLES

### Example 1

### Identification of an Immune Response to WT1

### in Patients with Hematological Malignancies

This Example illustrates the identification of an existent immune response in patients with a hematological malignancy.

To evaluate the presence of preexisting WT1 specific antibody responses in patients, sera of patients with AML, ALL, CML and severe aplastic anemia were analyzed using Western blot analysis. Sera were tested for the ability to immunoprecipitate WT1 from the human leukemic cell line K562 (American Type Culture Collection, Manassas, VA). In each case, immunoprecipitates were separated by gel electrophoresis, transferred to membrane and probed with the anti WT-1 antibody WT180 (Santa Cruz Biotechnology, Inc., Santa Cruz, CA). This Western blot analysis identified potential WT1 specific antibodies in patients with hematological malignancy. A representative Western blot showing the results for a patient with AML is shown in Figure 2. A 52 kD protein in the immunoprecipitate generated using the patient sera was recognized by the WT1 specific antibody. The 52 kD protein migrated at the same size as the positive control.

### Example 2

### Induction of Antibodies to WT1 in Mice Immunized with Cell Lines Expressing WT1

This Example illustrates the use of cells expressing WT1 to induce a WT1 specific antibody response *in vivo.*

Detection of existent antibodies to WT1 in patients with leukemia strongly implied that it is possible to immunize to WT1 protein to elicit immunity to WT1. To test whether immunity to WT1 can be generated by vaccination, mice were injected with TRAMP-C, a WT1 positive tumor cell line of B6 origin. Briefly, male B6 mice were immunized with 5 x $10^6$ TRAMP-C cells subcutaneously and boosted twice with 5 $\times$ $10^6$ cells at three week intervals. Three weeks after the final immunization, sera were obtained and single cell suspensions of spleens were prepared in RPMI 1640 medium (GIBCO) with 25$\mu$M $\beta$-2-mercaptoethanol, 200 units of penicillin per ml, 10mM L-glutamine, and 10% fetal bovine serum.

Following immunization to TRAMP-C, a WT1 specific antibody response in the immunized animals was detectable. A representative Western blot is shown in Figure 3. These results show that immunization to WT1 protein can elicit an immune response to WT1 protein.

Example 3

Induction of Th and Antibody Responses in Mice Immunized with WT1 Peptides

This Example illustrates the ability of immunization with WT1 peptides to elicit an immune response specific for WT1.

Peptides suitable for eliciting Ab and proliferative T cell responses were identified according to the Tsites program (Rothbard and Taylor, EMBO J 7:93-100, 1988; Deavin et al., Mol. Immunol. 33:145-155, 1996), which searches for peptide motifs that have the potential to elicit Th responses. Peptides shown in Table I were synthesized and sequenced.

Table I

| WTI Peptides | | |
|---|---|---|
| Peptide | Sequence | Comments |
| Mouse: p6-22 | RDLNALLPAVSSLGGGG (SEQ ID NO: 13) | 1 mismatch relative to human WT1 sequence |
| Human: p6-22 | RDLNALLPAVPSLGGGG (SEQ ID NO: 1) | |
| Human/mouse: p117-139 | PSQASSGQARMFPNAPYLPSCLE (SEQ ID NOs: 2 and 3) | |
| Mouse: p244-262 | GATLKGMAAGSSSSVKWTE (SEQ ID NO:14) | 1 mismatch relative to human WT1 sequence |
| Human: p244-262 | GATLKGVAAGSSSSVKWTE (SEQ ID NO:4) | |
| Human/mouse: | RIHTHGVFRGIQDVR | |
| p287-301 | (SEQ ID NOs: 15 and 16) | |
| Mouse: p299-313 | VRRVSGVAPTLVRS (SEQ ID NO:17) | 1 mismatch relative to human WT1 sequence |
| Human/mouse: p421-435 | CQKKFARSDELVRHH (SEQ ID NOs: 19 and 20) | |

For immunization, peptides were grouped as follows:

Group A: p6-22 human: 10.9mg in 1ml (101μl = 100μg)
p117-139 human/mouse: 7.6mg in 1ml (14μl = 100μg)
p244-262 human: 4.6.mg in 1ml (22μl = 100μg)

Group B: p287-301 human/mouse: 7.2mg in 1ml (14μl = 100μg)
mouse p299-313: 6.6.mg in 1ml (15μl = 100μg)
p421-435 human/mouse: 3.3mg in 1ml (30μl= 100μg)

Control: (FBL peptide 100μg) + CFA/IFA

Control: (CD45 peptide 100μg) + CFA/IFA

Group A contained peptides present within the amino terminus portion of WT1 (exon 1) and Group B contained peptides present within the carboxy terminus, which contains a four zinc finger region with sequence homology to other DNA-binding proteins. Within group B, p287-301 and p299-313 were derived from exon 7, zinc finger 1, and p421-435 was derived from exon 10, zinc finger IV.

B6 mice were immunized with a group of WT1 peptides or with a control peptide. Peptides were dissolved in 1ml sterile water for injection, and B6 mice were immunized 3 times at time intervals of three weeks. Adjuvants used were CFA/IFA, GM-CSF, and Montinide. The presence of antibodies specific for WT1 was then determined as described in Examples 1 and 2, and proliferative T cell responses were evaluated using a standard thymidine incorporation assay, in which cells were cultured in the presence of antigen and proliferation was evaluated by measuring incorporated radioactivity (Chen et al., Cancer Res. 54: 1 065-1 070, 1994). In particular, lymphocytes were cultured in 96-well plates at $2x10^5$ cells per well with $4x10^5$ irradiated (3000 rads) syngeneic spleen cells and the designated peptide.

Immunization of mice with the group of peptides designated as Group A elicited an antibody response to WT1 (Figure 4). No antibodies were detected following immunization to Vaccine B, which is consistent with a lack of helper T cell response from immunization with Vaccine B. P117-139 elicited proliferative T cell responses (Figures 5A-5C). The stimulation indices (SI) varied between 8 and 72. Other peptides (P6-22 and P299-313) also were shown to elicit proliferative T cell responses. Immunization with P6-22 resulted in a stimulation index (SI) of 2.3 and immunization with P299-313 resulted in a SI of 3.3. Positive controls included ConA stimulated T cells, as well as T cells stimulated with known antigens, such as CD45 and FBL, and allogeneic T cell lines (DeBruijn et al., *Eur. J. Immunol.* 21:2963-2970, 1991).

Figures 6A and 6B show the proliferative response observed for each of the three peptides within vaccine A (Figure 6A) and vaccine B (Figure 6B). Vaccine A elicited proliferative T cell responses to the immunizing peptides p6-22 and p117-139, with stimulation indices (SI) varying between 3 and 8 (bulk lines). No proliferative response to p244-262 was detected (Figure 6A).

Subsequent *in vitro* stimulations were carried out as single peptide stimulations using only p6-22 and p117-139. Stimulation of the Vaccine A specific T cell line with p117-139 resulted in proliferation to p117-139 with no response to p6-22 (Figure7A). Clones derived from the line were specific for p117-139 (Figure 7B). By contrast, stimulation of the Vaccine A specific T cell line with p6-22 resulted in proliferation to p6-22 with no response to p117-139 (Figure 7C). Clones derived from the line were specific for p6-22 (Figure 7D).

These results show that vaccination with WT1 peptides can elicit antibody responses to WT1 protein and proliferative T cell responses to the immunizing peptides.

Example 4

Induction of CTL Responses in Mice Immunized with WT1 Peptides

This Example illustrates the ability of WT1 peptides to elicit CTL immunity.

Peptides (9-mers) with motifs appropriate for binding to class I MHC were identified using a BIMAS HLA peptide binding prediction analysis (Parker et al., J Immunol. 152:163, 1994). Peptides identified within such analyses are shown in Tables II - XLIV. In each of these tables, the score reflects the theoretical binding affinity (half-time of dissociation) of the peptide to the MHC molecule indicated.

Peptides identified using the Tsites program (Rothbard and Taylor, EMBO J. 7:93-100, 1988; Deavin et al., Mol. Immunol. 33:145-155, 1996), which searches for peptide motifs that have the potential to elicit Th responses are further shown in Figures 8A and 8B, and Table XLV.

Table II

| Results of BIMAS HLA Peptide Binding Prediction Analysis for Binding of Human WT1 Peptides to Human HLA A1 | | | |
|---|---|---|---|
| Rank | Start Position | Subsequence Residue Listing | Score (Estimate of Half Time of Disassociation of a Molecule Containing This Subsequence) |
| 1 | 137 | CLESQPAIR (SEQ-ID NO:47) | 18.000 |
| 2 | 80 | GAEPHEEQC (SEQ ID NO:87) | 9.000 |
| 3 | 40 | FAPPGASAY (SEQ ID NO:74) | 5.000 |
| 4 | 354 | QCDFKDCER (SEQ ID NO: 162) | 5.000 |
| 5 | 2 | GSDVRDLNA (SEQ ID NO: 101) | 3.750 |
| 6 | 152 | VTFDGTPSY (SEQ ID NO:244) | 2.500 |
| 7 | 260 | WTEGQSNHS (SEQ ID NO: 247) | 2.250 |
| 8 | 409 | TSEKPFSCR (SEQ ID NO:232) | 1.350 |

(continued)

| Results of BIMAS HLA Peptide Binding Prediction Analysis for Binding of Human WT1 Peptides to Human HLA A1 | | | |
|---|---|---|---|
| Rank | Start Position | Subsequence Residue Listing | Score (Estimate of Half Time of Disassociation of a Molecule Containing This Subsequence) |
| 9 | 73 | KQEPSWGGA (SEQ ID NO: 125) | 1.350 |
| 10 | 386 | KTCQRKFSR (SEQ ID NO: 128) | 1.250 |
| 11 | 37 | VLDFAPPGA (SEQ ID NO:241) | 1.000 |
| 12 | 325 | CAYPGCNKR (SEQ ID NO:44) | 1.000 |
| 13 | 232 | QLECMTWNQ (SEQ ID NO: 167) | 0.900 |
| 14 | 272 | ESDNHTTPI (SEQ ID NO:71) | 0.750 |
| 15 | 366 | RSDQLKRHQ (SEQ ID NO: 193) | 0.750 |
| 16 | 222 | SSDNLYQMT (SEQ ID NO: 217) | 0.750 |
| 17 | 427 | RSDELVRHH (SEQ ID NO: 191) | 0.750 |
| 18 | 394 | RSDHLKTHT (SEQ ID NO: 192) | 0.750 |
| 19 | 317 | TSEKRPFMC (SEQ ID NO: 233) | 0.675 |
| 20 | 213 | QALLLRTPY (SEQ ID NO:160) | 0.500 |

Table III

| Results of BIMAS HLA Peptide Binding Prediction Analysis for Binding of Human WT1 Peptides to Human HLA A 0201 | | | |
|---|---|---|---|
| Rank | Start Position | Subsequence Residue Listing | Score (Estimate of Half Time of Disassociation of a Molecule Containing This Subsequence) |
| 1 | 126 | RMFPNAPYL (SEQ ID NO: 185) | 313.968 |
| 2 | 187 | SLGEQQYSV (SEQ ID NO: 214) | 285.163 |
| 3 | 10 | ALLPAVPSL (SEQ ID NO:34) | 181.794 |
| 4 | 242 | NLGATLKGV (SEQ ID NO: 146) | 159.970 |
| 5 | 225 | NLYQMTSQL (SEQ ID NO: 147) | 68.360 |
| 6 | 292 | GVFRGIQDV (SEQ ID NO: 103) | 51.790 |
| 7 | 191 | QQYSVPPPV (SEQ ID NO: 171) | 22.566 |
| 8 | 280 | ILCGAQYRI (SEQ ID NO:116) | 17.736 |

(continued)

| Results of BIMAS HLA Peptide Binding Prediction Analysis for Binding of Human WT1 Peptides to Human HLA A 0201 | | | |
|---|---|---|---|
| Rank | Start Position | Subsequence Residue Listing | Score (Estimate of Half Time of Disassociation of a Molecule Containing This Subsequence) |
| 9 | 235 | CMTWNQMNL (SEQ ID NO: 49) | 15.428 |
| 10 | 441 | NMTKLQLAL (SEQ ID NO:149) | 15.428 |
| 11 | 7 | DLNALLPAV (SEQ ID NO:58) | 11.998 |
| 12 | 227 | YQMTSQLEC (SEQ ID NO: 251) | 8.573 |
| 13 | 239 | NQMNLGATL (SEQ ID NO: 151) | 8.014 |
| 14 | 309 | TLVRSASET (SEQ ID NO:226) | 7.452 |
| 15 | 408 | KTSEKPFSC (SEQ ID NO:129) | 5.743 |
| 16 | 340 | LQMHSRKHT (SEQ ID NO: 139) | 4.752 |
| 17 | 228 | QMTSQLECM (SEQ ID NO: 169) | 4.044 |
| 18 | 93 | TVHFSGQFT (SEQ ID NO: 235) | 3.586 |
| 19 | 37 | VLDFAPPGA (SEQ ID NO: 241) | 3.378 |
| 20 | 86 | EQCLSAFTV (SEQ ID NO:69) | 3.068 |

Table IV

| Results of BIMAS HLA Peptide Binding Prediction Analysis for Binding of Human WT1 Peptides to Human HLA A 0205 | | | |
|---|---|---|---|
| Rank | Start Position | Subsequence Residue Listing | Score (Estimate of Half Time of Disassociation of a Molecule Containing This Subsequence) |
| 1 | 10 | ALLPAVPSL (SEQ ID NO:34) | 42.000 |
| 2 | 292 | GVFRGIQDV (SEQ ID NO: 103) | 24.000 |
| 3 | 126 | RMFPNAPYL (SEQ ID NO: 185) | 21.000 |
| 4 | 225 | NLYQMTSQL (SEQ ID NO: 147) | 21.000 |
| 5 | 239 | NQMNLGATL (SEQ ID NO: 151) | 16.800 |
| 6 | 302 | RVPGVAPTL (SEQ ID NO: 195) | 14.000 |
| 7 | 441 | NMTKLQLAL (SEQ ID NO:149) | 7.000 |

(continued)

| Results of BIMAS HLA Peptide Binding Prediction Analysis for Binding of Human WT1 Peptides to Human HLA A 0205 | | | |
|---|---|---|---|
| Rank | Start Position | Subsequence Residue Listing | Score (Estimate of Half Time of Disassociation of a Molecule Containing This Subsequence) |
| 8 | 235 | CMTWNQMNL (SEQ ID NO: 49) | 7.000 |
| 9 | 187 | SLGEQQYSV (SEQ ID NO: 214) | 6.000 |
| 10 | 191 | QQYSVPPPV (SEQ ID NO: 171) | 4.800 |
| 11 | 340 | LQMHSRKHT (SEQ ID NO: 139) | 4.080 |
| 12 | 242 | NLGATLKGV (SEQ ID NO: 146) | 4.000 |
| 13 | 227 | YQMTSQLEC (SEQ ID NO: 251) | 3.600 |
| 14 | 194 | SVPPPVYGC (SEQ ID NO: 218) | 2.000 |
| 15 | 93 | TVHFSGQFT (SEQ ID NO: 235) | 2.000 |
| 16 | 280 | ILCGAQYRI (SEQ ID NO:116) | 1.700 |
| 17 | 98 | GQFTGTAGA (SEQ ID NO:99) | 1.200 |
| 18 | 309 | TLVRSASET (SEQ ID NO:226) | 1.000 |
| 19 | 81 | AEPHEEQCL (SEQ ID NO:30) | 0.980 |
| 20 | 73 | KQEPSWGGA (SEQ ID NO: 125) | 0.960 |

Table V

| Results of BIMAS HLA Peptide Binding Prediction Analysis for Binding of Human WT1 Peptides to Human HLA A24 | | | |
|---|---|---|---|
| Rank | Start Position | Subsequence Residue Listing | Score (Estimate of Half Time of Disassociation of a Molecule Containing This Subsequence) |
| 1 | 302 | RVPGVAPTL (SEQ ID NO:195) | 16.800 |
| 2 | 218 | RTPYSSDNL (SEQ ID NO:194) | 12.000 |
| 3 | 356 | DFKDCERRF (SEQ ID NO:55) | 12.000 |
| 4 | 126 | RMFPNAPYL (SEQ ID NO:185) | 9.600 |
| 5 | 326 | AYPGCNKRY (SEQ ID NO:42) | 7.500 |
| 6 | 270 | GYESDNHT (SEQ ID NO:106)T | 7.500 |
| 7 | 239 | NQMNLGATL (SEQ ID NO:151) | 7.200 |
| 8 | 10 | ALLPAVPSL (SEQ ID NO:34) | 7.200 |
| 9 | 130 | NAPYLPSCL (SEQ ID NO:144) | 7.200 |
| 10 | 329 | GCNKRYFKL (SEQ ID NO:90) | 6.600 |

(continued)

| Results of BIMAS HLA Peptide Binding Prediction Analysis for Binding of Human WT1 Peptides to Human HLA A24 | | | |
|---|---|---|---|
| Rank | Start Position | Subsequence Residue Listing | Score (Estimate of Half Time of Disassociation of a Molecule Containing This Subsequence) |
| 11 | 417 | RWPSCQKKF (SEQ ID NO: 196) | 6.600 |
| 12 | 47 | AYGSLGGPA (SEQ ID NO:41) | 6.000 |
| 13 | 180 | DPMGQQGSL (SEQ ID NO:59) | 6.000 |
| 14 | 4 | DVRDLNALL (SEQ ID NO:62) | 5.760 |
| 15 | 285 | QYRIHTHGV (SEQ ID NO: 175) | 5.000 |
| 16 | 192 | QYSVPPPVY (SEQ ID NO: 176) | 5.000 |
| 17 | 207 | DSCTGSQAL (SEQ ID NO:61) | 4.800 |
| 18 | 441 | NMTKLQLAL (SEQ ID NO: 149) | 4.800 |
| 19 | 225 | NLYQMTSQL (SEQ ID NO: 147) | 4.000 |
| 20 | 235 | CMTWNQMNL (SEQ ID NO:49) | 4.000 |

Table VI

| Results of BIMAS HLA Peptide Binding Prediction Analysis for Binding of Human WT1 Peptides to Human HLA A3 | | | |
|---|---|---|---|
| Rank | Start Position | Subsequence Residue Listing | Score (Estimate of Half Time of Disassociation of a Molecule Containing This Subsequence) |
| 1 | 436 | NMHQRNMTK (SEQ ID NO: 148) | 40.000 |
| 2 | 240 | QMNLGATLK (SEQ ID NO: 168) | 20.000 |
| 3 | 88 | CLSAFTVHF (SEQ ID NO:48) | 6.000 |
| 4 | 126 | RMFPNAPYL (SEQ ID NO: 185) | 4.500 |
| 5 | 169 | AQFPNHSFK (SEQ ID NO:36) | 4.50.0 |
| 6 | 10 | ALLPAVPSL (SEQ ID NO:34) | 4.050 |
| 7 | 137 | CLESQPAIR (SEQ ID NO:47) | 4.000 |
| 8 | 225 | NLYQMTSQL (SEQ ID NO: 147) | 3.000 |
| 9 | 32 | AQWAPVLDF (SEQ ID NO:37) | 2.700 |
| 10 | 280 | ILCGAQYRI (SEQ ID NO:116) | 2.700 |
| 11 | 386 | KTCQRKFSR (SEQ ID NO: 128) | 1.800 |
| 12 | 235 | CMTWNQMNL (SEQ ID NO: 49) | 1.200 |
| 13 | 441 | NMTKLQLAL (SEQ ID NO:149) | 1.200 |
| 14 | 152 | VTFDGTPSY (SEQ ID NO:244) | 1.000 |

(continued)

| Results of BIMAS HLA Peptide Binding Prediction Analysis for Binding of Human WT1 Peptides to Human HLA A3 | | | |
|---|---|---|---|
| Rank | Start Position | Subsequence Residue Listing | Score (Estimate of Half Time of Disassociation of a Molecule Containing This Subsequence) |
| 15 | 187 | SLGEQQYSV (SEQ ID NO: 214) | 0.900 |
| 16 | 383 | FQCKTCQRK (SEQ ID NO: 80) | 0.600 |
| 17 | 292 | GVFRGIQDV (SEQ ID NO: 103) | 0.450 |
| 18 | 194 | SVPPPVYGC (SEQ ID NO: 218) | 0.405 |
| 19 | 287 | RIHTHGVFR (SEQ ID NO:182) | 0.400 |
| 20 | 263 | GQSNHSTGY (SEQ ID NO: 100) | 0.360 |

Table VII

| Results of BIMAS HLA Peptide Binding Prediction Analysis for Binding of Human WT1 Peptides to Human HLA A68.1 | | | |
|---|---|---|---|
| Rank | Start Position | Subsequence Residue Listing | Score (Estimate of Half Time of Disassociation of a Molecule Containing This Subsequence) |
| 1 | 100 | FTGTAGACR (SEQ ID NO: 84) | 100.000 |
| 2 | 386 | KTCQRKFSR (SEQ ID NO: 128) | 50.000 |
| 3 | 368 | DQLKRHQRR (SEQ ID NO:60) | 30.000 |
| 4 | 312 | RSASETSEK (SEQ ID NO:190) | 18.000 |
| 5 | 337 | LSHLQMHSR (SEQ ID NO: 141) | 15.000 |
| 6 | 364 | FSRSDQLKR (SEQ ID NO:83) | 15.000 |
| 7 | 409 | TSEKPFSCR (SEQ ID NO:232) | 15.000 |
| 8 | 299 | DVRRVPGVA (SEQ ID NO:63) | 12.000 |
| 9 | 4 | DVRDLNALL (SEQ ID NO:62) | 12.000 |
| 10 | 118 | SQASSGQAR (SEQ ID NO: 216) | 10.000 |
| 11 | 343 | HSRKHTGEK (SEQ ID NO: 111) | 9.000 |
| 12 | 169 | AQFPNHSFK (SEQ ID NO:36) | 9.000 |
| 13 | 292 | GVFRGIQDV (SEQ ID NO:103) | 8.000 |
| 14 | 325 | CAYPGCNKR (SEQ ID NO:44) | 7.500 |
| 15 | 425 | FARSDELVR (SEQ ID NO:75) | 7.500 |
| 16 | 354 | QCDFKDCER (SEQ ID NO: 162) | 7.500 |
| 17 | 324 | MCAYPGCNK (SEQ ID NO: 142) | 6.000 |

(continued)

| Results of BIMAS HLA Peptide Binding Prediction Analysis for Binding of Human WT1 Peptides to Human HLA A68.1 | | | |
|---|---|---|---|
| Rank | Start Position | Subsequence Residue Listing | Score (Estimate of Half Time of Disassociation of a Molecule Containing This Subsequence) |
| 18 | 251 | AAGSSSSVK (SEQ ID NO:28) | 6.000 |
| 19 | 379 | GVKPFQCKT (SEQ ID NO: 104) | 6.000 |
| 20 | 137 | CLESQPAIR (SEQ ID NO:47) | 5.000 |

Table VIII

| Results of BIMAS HLA Peptide Binding Prediction Analysis for Binding of Human WT1 Peptides to Human HLA A 1101 | | | |
|---|---|---|---|
| Rank | Start Position | Subsequence Residue Listing | Score (Estimate of Half Time of Disassociation of a Molecule Containing This Subsequence) |
| 1 | 386 | KTCQRKFSR (SEQ ID NO: 128) | 1.800 |
| 2 | 169 | AQFPNHSFK (SEQ ID NO:36) | 1.200 |
| 3 | 436 | NMHQRNMTK (SEQ ID NO: 148) | 0.800 |
| 4 | 391 | KFSRSDHLK (SEQ ID NO: 120) | 0.600 |
| 5 | 373 | HQRRHTGVK (SEQ ID NO: 109) | 0.600 |
| 6 | 383 | FQCKTCQRK (SEQ ID NO: 80) | 0.600 |
| 7 | 363 | RFSRSDQLK (SEQ ID NO: 178) | 0.600 |
| 8 | 240 | QMNLGATLK (SEQ ID NO: 168) | 0.400 |
| 9 | 287 | RIHTHGVFR (SEQ ID NO: 182) | 0.240 |
| 10 | 100 | FTGTAGACR (SEQ ID NO: 84) | 0.200 |
| 11 | 324 | MCAYPGCNK (SEQ ID NO: 142) | 0.200 |
| 12 | 251 | AAGSSSSVK (SEQ ID NO:28) | 0.200 |
| 13 | 415 | SCRWPSCQK (SEQ ID NO: 201) | 0.200 |
| 14 | 118 | SQASSGQAR (SEQ ID NO: 216) | 0.120 |
| 15 | 292 | GVFRGIQDV (SEQ ID NO: 103) | 0.120 |
| 16 | 137 | CLESQPAIR (SEQ ID NO:47) | 0.080 |

(continued)

| Results of BIMAS HLA Peptide Binding Prediction Analysis for Binding of Human WT1 Peptides to Human HLA A 1101 | | | |
|---|---|---|---|
| Rank | Start Position | Subsequence Residue Listing | Score (Estimate of Half Time of Disassociation of a Molecule Containing This Subsequence) |
| 17 | 425 | FARSDELVR (SEQ ID NO:75) | 0.080 |
| 18 | 325 | CAYPGCNKR (SEQ ID NO: 44) | 0.080 |
| 19 | 312 | RSASETSEK (SEQ ID NO: 190) | 0.060 |
| 20 | 65 | PPPPHSFI (SEQ ID NO:156) K | 0.060 |

Table IX

| Results of BIMAS HLA Peptide Binding Prediction Analysis for Binding of Human WT1 Peptides to Human HLA A 3101 | | | |
|---|---|---|---|
| Rank | Start Position | Subsequence Residue Listing | Score (Estimate of Half Time of Disassociation of a Molecule Containing This Subsequence) |
| 1 | 386 | KTCQRKFSR (SEQ ID NO: 128) | 9.000 |
| 2 | 287 | RIHTHGVFR (SEQ ID NO: 182) | 6.000 |
| 3 | 137 | CLESQPAIR (SEQ ID NO:47) | 2.000 |
| 4 | 118 | SQASSGQAR (SEQ ID NO: 216) | 2.000 |
| 5 | 368 | DQLKRHQRR (SEQ ID NO: 60) | 1.200 |
| 6 | 100 | FTGTAGACR (SEQ ID NO: 84) | 1.000 |
| 7 | 293 | VFRGIQDVR (SEQ ID NO: 238) | 0.600 |
| 8 | 325 | CAYPGCNKR (SEQ ID NO: 44) | 0.600 |
| 9 | 169 | AQFPNHSFK (SEQ ID NO:36) | 0.600 |
| 10 | 279 | PILCGAQYR (SEQ ID NO: 155) | 0.400 |
| 11 | 436 | NMHQRNMTK (SEQ ID NO: 148) | 0.400 |
| 12 | 425 | FARSDELVR (SEQ ID NO:75) | 0.400 |
| 13 | 32 | AQWAPVLDF (SEQ ID NO: 37) | 0.240 |
| 14 | 240 | QMNLGATLK (SEQ ID NO: 168) | 0.200 |
| 15 | 354 | QCDFKDCER (SEQ ID NO: 162) | 0.200 |

(continued)

| Results of BIMAS HLA Peptide Binding Prediction Analysis for Binding of Human WT1 Peptides to Human HLA A 3101 | | | |
|---|---|---|---|
| Rank | Start Position | Subsequence Residue Listing | Score (Estimate of Half Time of Disassociation of a Molecule Containing This Subsequence) |
| 16 | 373 | HQRRHTGVK (SEQ ID NO: 109) | 0.200 |
| 17 | 383 | FQCKTCQRK (SEQ ID NO: 80) | 0.200 |
| 18 | 313 | SASETSEKR (SEQ ID NO: 197) | 0.200 |
| 19 | 358 | KDCERRFSR (SEQ ID NO: 118) | 0.180 |
| 20 | 391 | KFSRSDHLK (SEQ ID NO: 120) | 0.180 |

Table X

| Results of BIMAS HLA Peptide Binding Prediction Analysis for Binding of Human WT1 Peptides to Human HLA A 3302 | | | |
|---|---|---|---|
| Rank | Start Position | Subsequence Residue Listing | Score (Estimate of Half Time of Disassociation of a Molecule Containing This Subsequence) |
| 1 | 337 | LSHLQMHSR (SEQ ID NO: 141) | 15.000 |
| 2 | 409 | TSEKPFSCR (SEQ ID NO: 232) | 15.000 |
| 3 | 364 | FSRSDQLKR (SEQ ID NO:83) | 15.000 |
| 4 | 137 | CLESQPAIR (SEQ ID NO:47) | 9.000 |
| 5 | 368 | DQLKRHQRR (SEQ ID NO: 60) | 9.000 |
| 6 | 287 | RIHTHGVFR (SEQ ID NO: 182) | 4.500 |
| 7 | 210 | TGSQALLLR (SEQ ID NO: 223) | 3.000 |
| 8 | 425 | FARSDELVR (SEQ ID NO:75) | 3.000 |
| 9 | 313 | SASETSEKR (SEQ ID NO: 197) | 3.000 |
| 10 | 293 | VFRGIQDVR (SEQ ID NO: 238) | 3.000 |
| 11 | 354 | QCDFKDCER (SEQ ID NO: 162) | 3.000 |
| 12 | 100 | FTGTAGACR (SEQ ID NO:84) | 3.000 |
| 13 | 118 | SQASSGQAR (SEQ ID NO: 216) | 3.000 |
| 14 | 325 | CAYPGCNKR (SEQ ID NO:44) | 3.000 |

(continued)

| Results of BIMAS HLA Peptide Binding Prediction Analysis for Binding of Human WT1 Peptides to Human HLA A 3302 | | | |
|---|---|---|---|
| Rank | Start Position | Subsequence Residue Listing | Score (Estimate of Half Time of Disassociation of a Molecule Containing This Subsequence) |
| 15 | 207 | DSCTGSQAL (SEQ ID NO:61) | 1.500 |
| 16 | 139 | ESQPAIRNQ (SEQ ID NO:72) | 1.500 |
| 17 | 299 | DVRRVPGVA (SEQ ID NO:63) | 1.500 |
| 18 | 419 | PSCQKKFAR (SEQ ID NO: 159) | 1.500 |
| 19 | 272 | ESDNHTTPI (SEQ ID NO:71) | 1.500 |
| 20 | 4 | DVRDLNALL (SEQ ID NO:62) | 1.500 |

Table XI

| Results of BIMAS HLA Peptide Binding Prediction Analysis for Binding of Human WT1 Peptides to Human HLA B14 | | | |
|---|---|---|---|
| Rank | Start Position | Subsequence Residue Listing | Score (Estimate of Half Time of Disassociation of a Molecule Containing This Subsequence) |
| 1 | 362 | RRFSRSDQL (SEQ ID NO: 187) | 1000.000 |
| 2 | 332 | KRYFKLSHL (SEQ ID NO:127) | 300.000 |
| 3 | 423 | KKFARSDEL (SEQ ID NO:122) | 150.000 |
| 4 | 390 | RKFSRSDHL (SEQ ID NO:183) | 150.000 |
| 5 | 439 | QRNMTKLQL (SEQ ID NO: 173) | 20.000 |
| 6 | 329 | GCNKRYFKL (SEQ ID NO:90) | 10.000 |
| 7 | 10 | ALLPAVPSL (SEQ ID NO:34) | 10.000 |
| 8 | 180 | DPMGQQGSL (SEQ ID NO:59) | 9.000 |
| 9 | 301 | RRVPGVAPT (SEQ ID NO: 189) | 6.000 |
| 10 | 126 | RMFPNAPYL (SEQ ID NO: 185) | 5.000 |
| 11 | 371 | KRHQRRHTG (SEQ ID NO: 126) | 5.000 |
| 12 | 225 | NLYQMTSQL (SEQ ID NO: 147) | 5.000 |
| 13 | 144 | IRNQGYSTV (SEQ ID NO:117) | 4.000 |
| 14 | 429 | DELVRHHNM (SEQ ID NO:53) | 3.000 |
| 15 | 437 | MHQRNMTKL (SEQ ID NO: 143) | 3.000 |
| 16 | 125 | ARMFPNAPY (SEQ ID NO:38) | 3.000 |
| 17 | 239 | NQMNLGATL (SEQ ID NO: 151) | 3.000 |

(continued)

| Results of BIMAS HLA Peptide Binding Prediction Analysis for Binding of Human WT1 Peptides to Human HLA B14 | | | |
|---|---|---|---|
| Rank | Start Position | Subsequence Residue Listing | Score (Estimate of Half Time of Disassociation of a Molecule Containing This Subsequence) |
| 18 | 286 | YRIHTHGVF (SEQ ID NO:252) | 3.000 |
| 19 | 174 | HSFKHEDPM (SEQ ID NO: 110) | 3.000 |
| 20 | 372 | RHQRRHTGV (SEQ ID NO: 181) | 3.000 |

Table XII

| Results of BIMAS HLA Peptide Binding Prediction Analysis for Binding of Human WT1 Peptides to Human HLA B40 | | | |
|---|---|---|---|
| Rank | Start Position | Subsequence Residue Listing | Score (Estimate of Half Time of Disassociation of a Molecule Containing This Subsequence) |
| 1 | 81 | AEPHEEQCL (SEQ ID NO:30) | 40.000 |
| 2 | 429 | DELVRHHNM (SEQ ID NO:53) | 24.000 |
| 3 | 410 | SEKPFSCRW (SEQ ID NO: 207) | 20.000 |
| 4 | 318 | SEKRPFMCA (SEQ ID NO: 208) | 15.000 |
| 5 | 233 | LECMTWNQM (SEQ ID NO: 131) | 12.000 |
| 6 | 3 | SDVRDLNAL (SEQ ID NO:206) | 10.000 |
| 7 | 349 | GEKPYQCDF (SEQ ID NO:91) | 8.000 |
| 8 | 6 | RDLNALLPA (SEQ ID NO:177) | 5.000 |
| 9 | 85 | EEQCLSAFT (SEQ ID NO:65) | 4.000 |
| 10 | 315 | SETSEKRPF (SEQ ID NO:209) | 4.000 |
| 11 | 261 | TEGQSNHST (SEQ ID NO: 221) | 4.000 |
| 12 | 23 | GCALPVSGA (SEQ ID NO:89) | 3.000 |
| 13 | 38 | LDFAPPGAS (SEQ ID NO:130) | 3.000 |
| 14 | 273 | SDNHTTPIL (SEQ ID NO:204) | 2.500 |
| 15 | 206 | TDSCTGSQA (SEQ ID NO: 220) | 2.500 |
| 16 | 24 | CALPVSGAA (SEQ ID NO:43) | 2.000 |
| 17 | 98 | GQFTGTAGA (SEQ ID NO:99) | 2.000 |
| 18 | 30 | GAAQWAPVL (SEQ ID NO: 86) | 2.000 |
| 19 | 84 | HEEQCLSAF (SEQ ID NO:107) | 2.000 |
| 20 | 26 | LPVSGAAQW (SEQ ID NO: 138) | 2.000 |

Table XIII

| Results of BIMAS HLA Peptide Binding Prediction Analysis for Binding of Human WT1 Peptides to Human HLA B60 | | | |
|---|---|---|---|
| Rank | Start Position | Subsequence Residue Listing | Score (Estimate of Half Time of Disassociation of a Molecule Containing This Subsequence) |
| 1 | 81 | AEPHEEQCL (SEQ ID NO:30) | 160.000 |
| 2 | 3 | SDVRDLNAL (SEQ ID NO:206) | 40.000 |
| 3 | 429 | DELVRHHNM (SEQ ID NO:53) | 40.000 |
| 4 | 233 | LECMTWNQM (SEQ ID NO: 131) | 22.000 |
| 5 | 273 | SDNHTTPIL (SEQ ID NO:204) | 20.000 |
| 6 | 209 | CTGSQALLL (SEQ ID NO:52) | 8.000 |
| 7 | 30 | GAAQWAPVL (SEQ ID NO:86) | 8.000 |
| 8 | 318 | SEKRPFMCA (SEQ ID NO: 208) | 8.000 |
| 9 | 180 | DPMGQQGSL (SEQ ID NO:59) | 8.000 |
| 10 | 138 | LESQPAIRN (SEQ ID NO:132) | 5.280 |
| 11 | 239 | NQMNLGATL (SEQ ID NO: 151) | 4.400 |
| 12 | 329 | GCNKRYFKL (SEQ ID NO:90) | 4.400 |
| 13 | 130 | NAPYLPSCL (SEQ ID NO:144) | 4.400 |
| 14 | 85 | EEQCLSAFT (SEQ ID NO:65) | 4.400 |
| 15 | 208 | SCTGSQALL (SEQ ID NO:202) | 4.000 |
| 16 | 207 | DSCTGSQAL (SEQ ID NO:61) | 4.000 |
| 17 | 218 | RTPYSSDNL (SEQ ID NO:194) | 4.000 |
| 18 | 261 | TEGQSNHST (SEQ ID NO: 221) | 4.000 |
| 19 | 18 | LGGGGGCAL (SEQ ID NO: 134) | 4.000 |
| 20 | 221 | YSSDNLYQM (SEQ ID NO: 253) | 2.200 |

Table XIV

| Results of BIMAS HLA Peptide Binding Prediction Analysis for Binding of Human WT1 Peptides to Human HLA B61 | | | |
|---|---|---|---|
| Rank | Start Position | Subsequence Residue Listing | Score (Estimate of Half Time of Disassociation of a Molecule Containing This Subsequence) |
| 1 | 318 | SEKRPFMCA (SEQ ID NO: 208) | 20.000 |
| 2 | 429 | DELVRHHNM (SEQ ID NO:53) | 16.000 |
| 3 | 298 | QDVRRVPGV (SEQ ID NO: 164) | 10.000 |
| 4 | 81 | AEPHEEQCL (SEQ ID NO:30) | 8.000 |

(continued)

| Results of BIMAS HLA Peptide Binding Prediction Analysis for Binding of Human WT1 Peptides to Human HLA B61 | | | |
|---|---|---|---|
| Rank | Start Position | Subsequence Residue Listing | Score (Estimate of Half Time of Disassociation of a Molecule Containing This Subsequence) |
| 5 | 233 | LECMTWNQM (SEQ ID NO: 131) | 8.000 |
| 6 | 6 | RDLNALLPA (SEQ ID NO:177) | 5.500 |
| 7 | 85 | EEQCLSAFT(SEQ ID NO:65) | 4.000 |
| 8 | 261 | TEGQSNHST (SEQ ID NO: 221) | 4.000 |
| 9 | 206 | TDSCTGSQA (SEQ ID NO: 220) | 2.500 |
| 10 | 295 | RGIQDVRRV (SEQ ID NO: 179) | 2.200 |
| 11 | 3 | SDVRDLNAL (SEQ ID NO:206) | 2.000 |
| 12 | 250 | VAAGSSSSV (SEQ ID NO:236) | 2.000 |
| 13 | 29 | SGAAQWAPV (SEQ ID NO:21 1) | 2.000 |
| 14 | 315 | SETSEKRPF (SEQ ID NO:209) | 1.600 |
| 15 | 138 | LESQPAIRN (SEQ ID NO: 132) | 1.200 |
| 16 | 244 | GATLKGVAA (SEQ ID NO: 88) | 1.100 |
| 17 | 20 | GGGGCALPV (SEQ ID NO:92) | 1.100 |
| 18 | 440 | RNMTKLQLA (SEQ ID NO: 186) | 1.100 |
| 19 | 23 | GCALPVSGA (SEQ ID NO:89) | 1.100 |
| 20 | 191 | QQYSVPPPV (SEQ ID NO: 171) | 1.000 |

Table XV

| Results of BIMAS HLA Peptide Binding Prediction Analysis for Binding of Human WT1 Peptides to Human HLA B62 | | | |
|---|---|---|---|
| Rank | Start Position | Subsequence Residue Listing | Score (Estimate of Half Time of Disassociation of a Molecule Containing This Subsequence) |
| 1 | 146 | NQGYSTVTF (SEQ ID NO: 150) | 211.200 |
| 2 | 32 | AQWAPVLDF (SEQ ID NO:37) | 96.000 |
| 3 | 263 | GQSNHSTGY (SEQ ID NO: 100) | 96.000 |
| 4 | 88 | CLSAFTVHF (SEQ ID NO:48) | 96.000 |
| 5 | 17 | SLGGGGGCA (SEQ ID NO: 215) | 9.600 |
| 6 | 239 | NQMNLGATL (SEQ ID NO: 151) | 8.800 |

(continued)

| Results of BIMAS HLA Peptide Binding Prediction Analysis for Binding of Human WT1 Peptides to Human HLA B62 | | | |
|---|---|---|---|
| Rank | Start Position | Subsequence Residue Listing | Score (Estimate of Half Time of Disassociation of a Molecule Containing This Subsequence) |
| 7 | 191 | QQYSVPPPV (SEQ ID NO: 171) | 8.000 |
| 8 | 98 | GQFTGTAGA (SEQ ID NO:99) | 8.000 |
| 9 | 384 | QCKTCQRKF (SEQ ID NO: 163) | 6.000 |
| 10 | 40 | FAPPGASAY (SEQ ID NO:74) | 4.800 |
| 11 | 227 | YQMTSQLEC (SEQ ID NO: 251) | 4.800 |
| 12 | 187 | SLGEQQYSV (SEQ ID NO: 214) | 4.400 |
| 13 | 86 | EQCLSAFTV (SEQ ID NO:69) | 4.400 |
| 14 | 152 | VTFDGTPSY (SEQ ID NO:244) | 4.400 |
| 15 | 101 | TGTAGACRY (SEQ ID NO: 224) | 4.000 |
| 16 | 242 | NLGATLKGV (SEQ ID NO:146) | 4.000 |
| 17 | 92 | FTVHFSGQF (SEQ ID NO:85) | 4.000 |
| 18 | 7 | DLNALLPAV (SEQ ID NO:58) | 4.000 |
| 19 | 123 | GQARMFPNA (SEQ ID NO:98) | 4.000 |
| 20 | 280 | ILCGAQYRI (SEQ ID NO:116) | 3.120 |

Table XVI

| Results of BIMAS HLA Peptide Binding Prediction Analysis for Binding of Human WT1 Peptides to Human HLA B7 | | | |
|---|---|---|---|
| Rank | Start Position | Subsequence Residue Listing | Score (Estimate of Half Time of Disassociation of a Molecule Containing This Subsequence) |
| 1 | 180 | DPMGQQGSL (SEQ ID NO:59) | 240.000 |
| 2 | 4 | DVRDLNALL (SEQ ID NO:62) | 200.000 |
| 3 | 302 | RVPGVAPTL (SEQ ID NO: 195) | 20.000 |
| 4 | 30 | GAAQWAPVL (SEQ ID NO:86) | 12.000 |
| 5 | 239 | NQMNLGATL (SEQ ID NO: 151) | 12.000 |
| 6 | 130 | NAPYLPSCL (SEQ ID NO:144) | 12.000 |
| 7 | 10 | ALLPAVPSL (SEQ ID NO:34) | 12.000 |
| 8 | 299 | DVRRVPGVA (SEQ ID NO:63) | 5.000 |
| 9 | 208 | SCTGSQALL (SEQ ID NO:202) | 4.000 |
| 10 | 303 | VPGVAPTLV (SEQ ID NO:242) | 4.000 |
| 11 | 18 | LGGGGGCAL (SEQ ID NO: 134) | 4.000 |

(continued)

| Results of BIMAS HLA Peptide Binding Prediction Analysis for Binding of Human WT1 Peptides to Human HLA B7 | | | |
|---|---|---|---|
| Rank | Start Position | Subsequence Residue Listing | Score (Estimate of Half Time of Disassociation of a Molecule Containing This Subsequence) |
| 12 | 218 | RTPYSSDNL (SEQ ID NO:194) | 4.000 |
| 13 | 207 | DSCTGSQAL (SEQ ID NO:61) | 4.000 |
| 14 | 209 | CTGSQALLL (SEQ ID NO:52) | 4.000 |
| 15 | 329 | GCNKRYFKL (SEQ ID NO:90) | 4.000 |
| 16 | 235 | CMTWNQMNL (SEQ ID NO: 49) | 4.000 |
| 17 | 441 | NMTKLQLAL (SEQ ID NO:149) | 4.000 |
| 18 | 126 | RMFPNAPYL (SEQ ID NO:185) | 4.000 |
| 19 | 225 | NLYQMTSQL (SEQ ID NO: 147) | 4.000 |
| 20 | 143 | AIRNQGYST (SEQ ID NO:33) | 3.000 |

Table XVII

| Results of BIMAS HLA Peptide Binding Prediction Analysis for Binding of Human WT1 Peptides to Human HLA B8 | | | |
|---|---|---|---|
| Rank | Start Position | Subsequence Residue Listing | Score (Estimate of Half Time of Disassociation of a Molecule Containing This Subsequence) |
| 1 | 329 | GCNKRYFKL (SEQ ID NO:90) | 16.000 |
| 2 | 4 | DVRDLNALL (SEQ ID NO:62) | 12.000 |
| 3 | 316 | ETSEKRPFM (SEQ ID NO:73) | 3.000 |
| 4 | 180 | DPMGQQGSL (SEQ ID NO:59) | 1.600 |
| 5 | 208 | SCTGSQALL (SEQ ID NO:202) | 0.800 |
| 6 | 130 | NAPYLPSCL (SEQ ID NO:144) | 0.800 |
| 7 | 244 | GATLKGVAA (SEQ ID NO:88) | 0.800 |
| 8 | 30 | GAAQWAPVL (SEQ ID NO:86) | 0.800 |
| 9 | 299 | DVRRVPGVA (SEQ ID NO:63) | 0.400 |
| 10 | 420 | SCQKKFARS (SEQ ID NO: 200) | 0.400 |
| 11 | 387 | TCQRKFSRS (SEQ ID NO: 219) | 0.400 |
| 12 | 225 | NLYQMTSQL (SEQ ID NO: 147) | 0.400 |
| 13 | 141 | QPAIRNQGY (SEQ ID NO:170) | 0.400 |
| 14 | 10 | ALLPAVPSL (SEQ ID NO:34) | 0.400 |
| 15 | 207 | DSCTGSQAL (SEQ ID NO:61) | 0.400 |
| 16 | 384 | QCKTCQRKF (SEQ ID NO: 163) | 0.400 |
| 17 | 136 | SCLESQPAI (SEQ ID NO:198) | 0.300 |

(continued)

| Results of BIMAS HLA Peptide Binding Prediction Analysis for Binding of Human WT1 Peptides to Human HLA B8 | | | |
|---|---|---|---|
| Rank | Start Position | Subsequence Residue Listing | Score (Estimate of Half Time of Disassociation of a Molecule Containing This Subsequence) |
| 18 | 347 | HTGEKPYQC (SEQ ID NO: 112) | 0.300 |
| 19 | 401 | HTRTHTGKT (SEQ ID NO: 114) | 0.200 |
| 20 | 332 | KRYFKLSHL (SEQ ID NO:127) | 0.200 |

Table XVIII

| Results of BIMAS HLA Peptide Binding Prediction Analysis for Binding of Human WT1 Peptides to Human HLA B 2702 | | | |
|---|---|---|---|
| Rank | Start Position | Subsequence Residue Listing | Score (Estimate of Half Time of Disassociation of a Molecule Containing This Subsequence) |
| 1 | 332 | KRYFKLSHL (SEQ ID NO: 127) | 900.000 |
| 2 | 362 | RRFSRSDQL (SEQ ID NO: 187) | 900.000 |
| 3 | 286 | YRIHTHGVF (SEQ ID NO: 252) | 200.000 |
| 4 | 125 | ARMFPNAPY (SEQ ID NO: 38) | 200.000 |
| 5 | 375 | RRHTGVKPF (SEQ ID NO: 188) | 180.000 |
| 6 | 32 | AQWAPVLDF (SEQ ID NO: 37) | 100.000 |
| 7 | 301 | RRVPGVAPT (SEQ ID NO: 189) | 60.000 |
| 8 | 439 | QRNMTKLQL (SEQ ID NO: 173) | 60.000 |
| 9 | 126 | RMFPNAPYL (SEQ ID NO: 185) | 22.500 |
| 10 | 426 | ARSDELVRH (SEQ ID NO:39) | 20.000 |
| 11 | 146 | NQGYSTVTF (SEQ ID NO: 150) | 20.000 |
| 12 | 144 | IRNQGYSTV (SEQ ID NO: 117) | 20.000 |
| 13 | 389 | QRKFSRSDH (SEQ ID NO: 172) | 20.000 |
| 14 | 263 | GQSNHSTGY (SEQ ID NO: 100) | 20.000 |
| 15 | 416 | CRWPSCQKK (SEQ ID NO: 50) | 20.000 |

(continued)

| Results of BIMAS HLA Peptide Binding Prediction Analysis for Binding of Human WT1 Peptides to Human HLA B 2702 | | | |
|---|---|---|---|
| Rank | Start Position | Subsequence Residue Listing | Score (Estimate of Half Time of Disassociation of a Molecule Containing This Subsequence) |
| 16 | 191 | QQYSVPPPV (SEQ ID NO: 171) | 10.000 |
| 17 | 217 | LRTPYSSDN (SEQ ID NO: 140) | 10.000 |
| 18 | 107 | CRYGPFGPP (SEQ ID NO: 51) | 10.000 |
| 19 | 98 | GQFTGTAGA (SEQ ID NO: 99) | 10.000 |
| 20 | 239 | NQMNLGATL (SEQ ID NO: 151) | 6.000 |

Table XIX

| Results of BIMAS HLA Peptide Binding Prediction Analysis for Binding of Human WT1 Peptides to Human HLA B 2705 | | | |
|---|---|---|---|
| Rank | Start Position | Subsequence Residue Listing | Score (Estimate of Half Time of Disassociation of a Molecule Containing This Subsequence) |
| 1 | 332 | KRYFKLSHL (SEQ ID NO: 127) | 30000.000 |
| 2 | 362 | RRFSRSDQL (SEQ ID NO: 187) | 30000.000 |
| 3 | 416 | CRWPSCQKK (SEQ ID NO: 50) | 10000.000 |
| 4 | 439 | QRNMTKLQL (SEQ ID NO: 173) | 2000.000 |
| 5 | 286 | YRIHTHGVF (SEQ ID NO: 252) | 1000.000 |
| 6 | 125 | ARMFPNAPY (SEQ ID NO: 38) | 1000.000 |
| 7 | 294 | FRGIQDVRR (SEQ ID NO:81) | 1000.000 |
| 8 | 432 | VRHHNMHQR (SEQ ID NO: 243) | 1000.000 |
| 9 | 169 | AQFPNHSFK (SEQ ID NO:36) | 1000.000 |
| 10 | 375 | RRHTGVKPF (SEQ ID NO: 188) | 900.000 |
| 11 | 126 | RMFPNAPYL (SEQ ID NO: 185) | 750.000 |
| 12 | 144 | IRNQGYSTV (SEQ ID NO: 117) | 600.000 |
| 13 | 301 | RRVPGVAPT (SEQ ID NO: 189) | 600.000 |

(continued)

| Results of BIMAS HLA Peptide Binding Prediction Analysis for Binding of Human WT1 Peptides to Human HLA B 2705 | | | |
|---|---|---|---|
| Rank | Start Position | Subsequence Residue Listing | Score (Estimate of Half Time of Disassociation of a Molecule Containing This Subsequence) |
| 14 | 32 | AQWAPVLDF (SEQ ID NO: 37) | 500.000 |
| 15 | 191 | QQYSVPPPV (SEQ ID NO: 171) | 300.000 |
| 16 | 373 | HQRRHTGVK (SEQ ID NO: 109) | 200.000 |
| 17 | 426 | ARSDELVRH (SEQ ID NO:39) | 200.000 |
| 18 | 383 | FQCKTCQRK (SEQ ID NO: 80) | 200.000 |
| 19 | 239 | NQMNLGATL (SEQ ID NO: 151) | 200.000 |
| 20 | 389 | QRKFSRSDH (SEQ ID NO: 172) | 200.000 |

Table XX

| Results of BIMAS HLA Peptide Binding Prediction Analysis for Binding of Human WT1 Peptides to Human HLA B 3501 | | | |
|---|---|---|---|
| Rank | Start Position | Subsequence Residue Listing | Score (Estimate of Half Time of Disassociation of a Molecule Containing This Subsequence) |
| 1 | 278 | TPILCGAQY (SEQ ID NO: 227) | 40.000 |
| 2 | 141 | QPAIRNQGY (SEQ ID NO: 170) | 40.000 |
| 3 | 219 | TPYSSDNLY (SEQ ID NO: 231) | 40.000 |
| 4 | 327 | YPGCNKRYF (SEQ ID NO: 250) | 20.000 |
| 5 | 163 | TPSHHAAQF (SEQ ID NO: 228) | 20.000 |
| 6 | 180 | DPMGQQGSL (SEQ ID NO: 59) | 20.000 |
| 7 | 221 | YSSDNLYQM (SEQ ID NO: 253) | 20.000 |
| 8 | 26 | LPVSGAAQW (SEQ ID NO: 138) | 10.000 |
| 9 | 174 | HSFKHEDPM (SEQ ID NO: 110) | 10.000 |
| 10 | 82 | EPHEEQCLS (SEQ ID NO:68) | 6.000 |
| 11 | 213 | QALLLRTPY (SEQ ID NO: 160) | 6.000 |

(continued)

| Results of BIMAS HLA Peptide Binding Prediction Analysis for Binding of Human WT1 Peptides to Human HLA B 3501 | | | |
|---|---|---|---|
| Rank | Start Position | Subsequence Residue Listing | Score (Estimate of Half Time of Disassociation of a Molecule Containing This Subsequence) |
| 12 | 119 | QASSGQARM (SEQ ID NO: 161) | 6.000 |
| 13 | 4 | DVRDLNALL (SEQ ID NO:62) | 6.000 |
| 14 | 40 | FAPPGASAY (SEQ ID NO:74) | 6.000 |
| 15 | 120 | ASSGQARMF (SEQ ID NO: 40) | 5.000 |
| 16 | 207 | DSCTGSQAL (SEQ ID NO: 61) | 5.000 |
| 17 | 303 | VPGVAPTLV (SEQ ID NO: 242) | 4.000 |
| 18 | 316 | ETSEKRPFM (SEQ ID NO:73) | 4.000 |
| 19 | 152 | VTFDGTPSY (SEQ ID NO: 244) | 4.000 |
| 20 | 412 | KPFSCRWPS (SEQ ID NO: 123) | 4.000 |

Table XXI

| Results of BIMAS HLA Peptide Binding Prediction Analysis for Binding of Human WT1 Peptides to Human HLA B 3701 | | | |
|---|---|---|---|
| Rank | Start Position | Subsequence Residue Listing | Score (Estimate of Half Time of Disassociation of a Molecule Containing This Subsequence) |
| 1 | 3 | SDVRDLNAL (SEQ ID NO: 206) | 40.000 |
| 2 | 273 | SDNHTTPIL (SEQ ID NO:204) | 40.000 |
| 3 | 81 | AEPHEEQCL (SEQ ID NO:30) | 10.000 |
| 4 | 298 | QDVRRVPGV (SEQ ID NO: 164) | 8.000 |
| 5 | 428 | SDELVRHHN (SEQ ID NO: 203) | 6.000 |
| 6 | 85 | EEQCLSAFT (SEQ .ID NO:65) | 5.000 |
| 7 | 208 | SCTGSQALL (SEQ ID NO: 202) | 5.000 |
| 8 | 4 | DVRDLNALL (SEQ ID NO:62) | 5.000 |
| 9 | 209 | CTGSQALLL (SEQ ID NO:52) | 5.000 |
| 10 | 38 | LDFAPPGAS (SEQ ID NO: 130) | 4.000 |
| 11 | 223 | SDNLYQMTS (SEQ ID NO: 205) | 4.000 |
| 12 | 179 | EDPMGQQGS (SEQ ID NO: 64) | 4.000 |

(continued)

| Results of BIMAS HLA Peptide Binding Prediction Analysis for Binding of Human WT1 Peptides to Human HLA B 3701 | | | |
|---|---|---|---|
| Rank | Start Position | Subsequence Residue Listing | Score (Estimate of Half Time of Disassociation of a Molecule Containing This Subsequence) |
| 13 | 206 | TDSCTGSQA (SEQ ID NO: 220) | 4.000 |
| 14 | 6 | RDLNALLPA (SEQ ID NO:177) | 4.000 |
| 15 | 84 | HEEQCLSAF (SEQ ID NO: 107) | 2.000 |
| 16 | 233 | LECMTWNQM (SEQ ID NO: 131) | 2.000 |
| 17 | 429 | DELVRHHNM (SEQ ID NO:53) | 2.000 |
| 18 | 315 | SETSEKRPF (SEQ ID NO: 209) | 2.000 |
| 19 | 349 | GEKPYQCDF (SEQ ID NO:91) | 2.000 |
| 20 | 302 | RVPGVAPTL (SEQ ID NO: 195) | 1.500 |

Table XXII

| Results of BIMAS HLA Peptide Binding Prediction Analysis for Binding of Human WT1 Peptides to Human HLA B 3801 | | | |
|---|---|---|---|
| Rank | Start Position | Subsequence Residue Listing | Score (Estimate of Half Time of Disassociation of a Molecule Containing This Subsequence) |
| 1 | 437 | MHQRNMTKL (SEQ ID NO: 143) | 36.000 |
| 2 | 434 | HHNMHQRNM (SEQ ID NO: 108) | 6.000 |
| 3 | 372 | RHQRRHTGV (SEQ ID NO: 181) | 6.000 |
| 4 | 180 | DPMGQQGSL (SEQ ID NO:59) | 4.000 |
| 5 | 433 | RHHNMHQRN (SEQ ID NO: 180) | 3.900 |
| 6 | 165 | SHHAAQFPN (SEQ ID NO: 213) | 3.900 |
| 7 | 202 | CHTPTDSCT (SEQ ID NO:45) | 3.000 |
| 8 | 396 | DHLKTHTRT (SEQ ID NO:57) | 3.000 |
| 9 | 161 | GHTPSHHAA (SEQ ID NO:94) | 3.000 |
| 10 | 302 | RVPGVAPTL (SEQ ID NO: 195) | 2.600 |
| 11 | 417 | RWPSCQKKF (SEQ ID NO: 196) | 2.400 |
| 12 | 327 | YPGCNKRYF (SEQ ID NO: 250) | 2.400 |

(continued)

| Results of BIMAS HLA Peptide Binding Prediction Analysis for Binding of Human WT1 Peptides to Human HLA B 3801 | | | |
|---|---|---|---|
| Rank | Start Position | Subsequence Residue Listing | Score (Estimate of Half Time of Disassociation of a Molecule Containing This Subsequence) |
| 13 | 208 | SCTGSQALL (SEQ ID NO:202) | 2.000 |
| 14 | 163 | TPSHHAAQF (SEQ ID NO: 228) | 2.000 |
| 15 | 120 | ASSGQARMF (SEQ ID NO:40) | 2.000 |
| 16 | 18 | LGGGGGCAL (SEQ ID NO: 134) | 2.000 |
| 17 | 177 | KHEDPMGQQ (SEQ ID NO: 121) | 1.800 |
| 18 | 83 | PHEEQCLSA (SEQ ID NO: 154) | 1.800 |
| 19 | 10 | ALLPAVPSL (SEQ ID NO:34) | 1.300 |
| 20 | 225 | NLYQMTSQL (SEQ ID NO: 147) | 1.300 |

Table XXIII

| Results of BIMAS HLA Peptide Binding Prediction Analysis for Binding of Human WT1 Peptides to Human HLA B 3901 | | | |
|---|---|---|---|
| Rank | Start Position | Subsequence Residue Listing | Score (Estimate of Half Time of Disassociation of a Molecule Containing This Subsequence) |
| 1 | 437 | MHQRNMTKL (SEQ ID NO: 143) | 135.000 |
| 2 | 332 | KRYFKLSHL (SEQ ID NO: 127) | 45.000 |
| 3 | 434 | HHNMHQRNM (SEQ ID NO: 108) | 30.000 |
| 4 | 362 | RRFSRSDQL (SEQ ID NO: 187) | 30.000 |
| 5 | 372 | RHQRRHTGV (SEQ ID NO: 181) | 30.000 |
| 6 | 10 | ALLPAVPSL (SEQ ID NO:34) | 9.000 |
| 7 | 439 | QRNMTKLQL (SEQ ID NO: 173) | 7.500 |
| 8 | 390 | RKFSRSDHL (SEQ ID NO: 183) | 6.000 |
| 9 | 396 | DHLKTHTRT (SEQ ID NO:57) | 6.000 |
| 10 | 239 | NQMNLGATL (SEQ ID NO: 151) | 6.000 |
| 11 | 423 | KKFARSDEL (SEQ ID N0:122) | 6.000 |

(continued)

| Results of BIMAS HLA Peptide Binding Prediction Analysis for Binding of Human WT1 Peptides to Human HLA B 3901 | | | |
|---|---|---|---|
| Rank | Start Position | Subsequence Residue Listing | Score (Estimate of Half Time of Disassociation of a Molecule Containing This Subsequence) |
| 12 | 126 | RMFPNAPYL (SEQ ID NO: 185) | 6.000 |
| 13 | 225 | NLYQMTSQL (SEQ ID NO: 147) | 6.000 |
| 14 | 180 | DPMGQQGSL (SEQ ID NO:59) | 6.000 |
| 15 | 144 | IRNQGYSTV (SEQ ID NO:117) | 5.000 |
| 16 | 136 | SCLESQPAI (SEQ ID NO:198) | 4.000 |
| 17 | 292 | GVFRGIQDV (SEQ ID NO: 103) | 3.000 |
| 18 | 302 | RVPGVAPTL (SEQ ID NO:195) | 3.000 |
| 19 | 208 | SCTGSQALL (SEQ ID NO:202) | 3.000 |
| 20 | 207 | DSCTGSQAL (SEQ ID NO:61) | 3.000 |

Table XXIV

| Results of BIMAS HLA Peptide Binding Prediction Analysis for Binding of Human WT1 Peptides to Human HLA B 3902 | | | |
|---|---|---|---|
| Rank | Start Position | Subsequence Residue Listing | Score (Estimate of Half Time of Disassociation of a Molecule Containing This Subsequence) |
| 1 | 239 | NQMNLGATL (SEQ ID NO: 151) | 24.000 |
| 2 | 390 | RKFSRSDHL (SEQ ID NO: 183) | 20.000 |
| 3 | 423 | KKFARSDEL (SEQ ID NO: 122) | 20.000 |
| 4 | 32 | AQWAPVLDF (SEQ ID NO: 37) | 5.000 |
| 5 | 146 | NQGYSTVTF (SEQ ID NO: 150) | 5.000 |
| 6 | 130 | NAPYLPSCL (SEQ ID NO: 144) | 2.400 |
| 7 | 225 | NLYQMTSQL (SEQ ID NO: 147) | 2.400 |
| 8 | 30 | GAAQWAPVL (SEQ ID NO: 86) | 2.400 |
| 9 | 441 | NMTKLQLAL (SEQ ID NO: 149) | 2.400 |
| 10 | 302 | RVPGVAPTL (SEQ ID NO: 195) | 2.400 |

(continued)

| Results of BIMAS HLA Peptide Binding Prediction Analysis for Binding of Human WT1 Peptides to Human HLA B 3902 | | | |
|---|---|---|---|
| Rank | Start Position | Subsequence Residue Listing | Score (Estimate of Half Time of Disassociation of a Molecule Containing This Subsequence) |
| 11 | 126 | RMFPNAPYL (SEQ ID NO: 185) | 2.000 |
| 12 | 218 | RTPYSSDNL (SEQ ID NO: 194) | 2.000 |
| 13 | 209 | CTGSQALLL (SEQ ID NO:52) | 2.000 |
| 14 | 332 | KRYFKLSHL (SEQ ID NO: 127) | 2.000 |
| 15 | 180 | DPMGQQGSL (SEQ ID NO: 59) | 2.000 |
| 16 | 437 | MHQRNMTKL (SEQ ID NO: 143) | 2.000 |
| 17 | 207 | DSCTGSQAL (SEQ ID NO: 61) | 2.000 |
| 18 | 208 | SCTGSQALL (SEQ ID NO: 202) | 2.000 |
| 19 | 329 | GCNKRYFKL (SEQ ID NO:90) | 2.000 |
| 20 | 10 | ALLPAVPSL (SEQ ID NO:34) | 2.000 |

Table XXV

| Results of BIMAS HLA Peptide Binding Prediction Analysis for Binding of Human WT1 Peptides to Human HLA B 4403 | | | |
|---|---|---|---|
| Rank | Start Position | Subsequence Residue Listing | Score (Estimate of Half Time of Disassociation of a Molecule Containing This Subsequence) |
| 1 | 315 | SETSEKRPF (SEQ ID NO: 209) | 80.000 |
| 2 | 349 | GEKPYQCDF (SEQ ID NO:91) | 80.000 |
| 3 | 84 | HEEQCLSAF (SEQ ID NO: 107) | 60.000 |
| 4 | 410 | SEKPFSCRW (SEQ ID NO: 207) | 48.000 |
| 5 | 429 | DELVRHHNM (SEQ ID NO:53) | 24.000 |
| 6 | 278 | TPILCGAQY (SEQ ID NO:227) | 15.000 |
| 7 | 141 | QPAIRNQGY (SEQ ID NO: 170) | 9.000 |
| 8 | 40 | FAPPGASAY (SEQ ID NO: 74) | 9.000 |
| 9 | 213 | QALLLRTPY (SEQ ID NO:160) | 9.000 |
| 10 | 318 | SEKRPFMCA (SEQ ID NO: 208) | 8.000 |
| 11 | 81 | AEPHEEQCL (SEQ ID NO:30) | 8.000 |

(continued)

| Results of BIMAS HLA Peptide Binding Prediction Analysis for Binding of Human WT1 Peptides to Human HLA B 4403 | | | |
|---|---|---|---|
| Rank | Start Position | Subsequence Residue Listing | Score (Estimate of Half Time of Disassociation of a Molecule Containing This Subsequence) |
| 12 | 152 | VTFDGTPSY (SEQ ID NO: 244) | 4.500 |
| 13 | 101 | TGTAGACRY (SEQ ID NO: 224) | 4.500 |
| 14 | 120 | ASSGQARMF (SEQ ID NO:40) | 4.500 |
| 15 | 261 | TEGQSNHST (SEQ ID NO: 221) | 4.000 |
| 16 | 85 | EEQCLSAFT (SEQ ID NO:65) | 4.000 |
| 17 | 233 | LECMTWNQM (SEQ ID NO: 131) | 4.000 |
| 18 | 104 | AGACRYGPF (SEQ ID NO:31) | 4.000 |
| 19 | 3 | SDVRDLNAL (SEQ ID NO: 206) | 3.000 |
| 20 | 185 | QGSLGEQQY (SEQ ID NO: 166) | 3.000 |

Table XXVI

| Results of BIMAS HLA Peptide Binding Prediction Analysis for Binding of Human WT1 Peptides to Human HLA B 5101 | | | |
|---|---|---|---|
| Rank | Start Position | Subsequence Residue Listing | Score (Estimate of Half Time of Disassociation of a Molecule Containing This Subsequence) |
| 1 | 303 | VPGVAPTLV (SEQ ID NO: 242) | 314.600 |
| 2 | 180 | DPMGQQGSL (SEQ ID NO: 59) | 242.000 |
| 3 | 250 | VAAGSSSSV (SEQ ID NO: 236) | 157.300 |
| 4 | 130 | NAPYLPSCL (SEQ ID NO: 144) | 50.000 |
| 5 | 30 | GAAQWAPVL (SEQ ID NO: 86) | 50.000 |
| 6 | 20 | GGGGCALPV (SEQ ID NO: 92) | 44.000 |
| 7 | 64 | PPPPPHSFI (SEQ ID NO: 157) | 40.000 |
| 8 | 29 | SGAAQWAPV (SEQ ID NO: 211) | 40.000 |
| 9 | 18 | LGGGGGCAL (SEQ ID NO: 134) | 31.460 |
| 10 | 295 | RGIQDVRRV (SEQ ID NO: 179) | 22.000 |

(continued)

| Results of BIMAS HLA Peptide Binding Prediction Analysis for Binding of Human WT1 Peptides to Human HLA B 5101 | | | |
|---|---|---|---|
| Rank | Start Position | Subsequence Residue Listing | Score (Estimate of Half Time of Disassociation of a Molecule Containing This Subsequence) |
| 11 | 119 | QASSGQARM (SEQ ID NO: 161) | 18.150 |
| 12 | 418 | WPSCQKKFA (SEQ ID NO: 246) | 12.100 |
| 13 | 82 | EPHEEQCLS (SEQ ID NO:68) | 12.100 |
| 14 | 110 | GPFGPPPPS (SEQ ID NO: 96) | 11.000 |
| 15 | 272 | ESDNHTTPI (SEQ ID NO:71) | 8.000 |
| 16 | 306 | VAPTLVRSA (SEQ ID NO: 237) | 7.150 |
| 17 | 280 | ILCGAQYRI (SEQ ID NO:116) | 6.921 |
| 18 | 219 | TPYSSDNLY (SEQ ID NO: 231) | 6.600 |
| 19 | 128 | FPNAPYLPS (SEQ ID NO:79) | 6.500 |
| 20 | 204 | TPTDSCTGS (SEQ ID NO: 230) | 6.050 |

Table XXVII

| Results of BIMAS HLA Peptide Binding Prediction Analysis for Binding of Human WT1 Peptides to Human HLA B 5102 | | | |
|---|---|---|---|
| Rank | Start Position | Subsequence Residue Listing | Score (Estimate of Half Time of Disassociation of a Molecule Containing This Subsequence) |
| 1 | 295 | RGIQDVRRV (SEQ ID NO: 179) | 290.400 |
| 2 | 303 | VPGVAPTLV (SEQ ID NO: 242) | 200.000 |
| 3 | 180 | DPMGQQGSL (SEQ ID NO: 59) | 133.100 |
| 4 | 250 | VAAGSSSSV (SEQ ID NO: 236) | 110.000 |
| 5 | 30 | GAAQWAPVL (SEQ ID NO: 86) | 55.000 |
| 6 | 130 | NAPYLPSCL (SEQ ID NO: 144) | 50.000 |
| 7 | 20 | GGGGCALPV (SEQ ID NO: 92) | 44.000 |
| 8 | 29 | SGAAQWAPV (SEQ ID NO: 211) | 44.000 |
| 9 | 64 | PPPPPHSFI (SEQ ID NO: 157) | 40.000 |

(continued)

| Results of BIMAS HLA Peptide Binding Prediction Analysis for Binding of Human WT1 Peptides to Human HLA B 5102 | | | |
|---|---|---|---|
| Rank | Start Position | Subsequence Residue Listing | Score (Estimate of Half Time of Disassociation of a Molecule Containing This Subsequence) |
| 10 | 119 | QASSGQARM (SEQ ID NO: 161) | 36.300 |
| 11 | 110 | GPFGPPPPS (SEQ ID NO: 96) | 27.500 |
| 12 | 412 | KPFSCRWPS (SEQ ID NO: 123) | 25.000 |
| 13 | 18 | LGGGGGCAL (SEQ ID NO: 134) | 24.200 |
| 14 | 24 | CALPVSGAA (SEQ ID NO:43) | 16.500 |
| 15 | 219 | TPYSSDNLY (SEQ ID NO: 231) | 15.000 |
| 16 | 292 | GVFRGIQDV (SEQ ID NO: 103) | 14.641 |
| 17 | 136 | SCLESQPAI (SEQ ID NO: 198) | 14.520 |
| 18 | 418 | WPSCQKKFA (SEQ ID NO: 246) | 12.100 |
| 19 | 269 | TGYESDNHT (SEQ ID NO: 225) | 11.000 |
| 20 | 351 | KPYQCDFKD (SEQ ID NO: 124) | 11.000 |

Table XXVIII

| Results of BIMAS HLA Peptide Binding Prediction Analysis for Binding of Human WT1 Peptides to Human HLA B 5201 | | | |
|---|---|---|---|
| Rank | Start Position | Subsequence Residue Listing | Score (Estimate of Half Time of Disassociation of a Molecule Containing This Subsequence) |
| 1 | 191 | QQYSVPPPV (SEQ ID NO: 171) | 100.000 |
| 2 | 32 | AQWAPVLDF (SEQ ID NO: 37) | 30.000 |
| 3 | 243 | LGATLKGVA (SEQ ID NO: 133) | 16.500 |
| 4 | 303 | VPGVAPTLV (SEQ ID NO: 242) | 13.500 |
| 5 | 86 | EQCLSAFTV (SEQ ID NO:69) | 12.000 |
| 6 | 295 | RGIQDVRRV (SEQ ID NO: 179) | 10.000 |
| 7 | 98 | GQFTGTAGA (SEQ ID NO: 99) | 8.250 |

(continued)

| Results of BIMAS HLA Peptide Binding Prediction Analysis for Binding of Human WT1 Peptides to Human HLA B 5201 | | | |
|---|---|---|---|
| Rank | Start Position | Subsequence Residue Listing | Score (Estimate of Half Time of Disassociation of a Molecule Containing This Subsequence) |
| 8 | 292 | GVFRGIQDV (SEQ ID NO: 103) | 8.250 |
| 9 | 29 | SGAAQWAPV (SEQ ID NO: 211) | 6.000 |
| 10 | 146 | NQGYSTVTF (SEQ ID NO: 150) | 5.500 |
| 11 | 20 | GGGGCALPV (SEQ ID NO: 92) | 5.000 |
| 12 | 239 | NQMNLGATL (SEQ ID NO: 151) | 4.000 |
| 13 | 64 | PPPPPHSFI (SEQ ID NO: 157) | 3.600 |
| 14 | 273 | SDNHTTPIL (SEQ ID NO:204) | 3.300 |
| 15 | 286 | YRIHTHGVF (SEQ ID NO: 252) | 3.000 |
| 16 | 269 | TGYESDNHT (SEQ ID NO: 225) | 3.000 |
| 17 | 406 | TGKTSEKPF (SEQ ID NO: 222) | 2.750 |
| 18 | 327 | YPGCNKRYF (SEQ ID NO: 250) | 2.750 |
| 19 | 7 | DLNALLPAV (SEQ ID NO:58) | 2.640 |
| 20 | 104 | AGACRYGPF (SEQ ID NO: 31) | 2.500 |

Table XXIX

| Results of BIMAS HLA Peptide Binding Prediction Analysis for Binding of Human WT1 Peptides to Human HLA B 5801 | | | |
|---|---|---|---|
| Rank | Start Position | Subsequence Residue Listing | Score (Estimate of Half Time of Disassociation of a Molecule Containing This Subsequence) |
| 1 | 230 | TSQLECMTW (SEQ ID NO: 234) | 96.800 |
| 2 | 92 | FTVHFSGQF (SEQ ID NO:85) | 60.000 |
| 3 | 120 | ASSGQARMF (SEQ ID NO: 40) | 40.000 |
| 4 | 168 | AAQFPNHSF (SEQ ID NO:29) | 20.000 |
| 5 | 408 | KTSEKPFSC (SEQ ID NO: 129) | 12.000 |
| 6 | 394 | RSDHLKTHT (SEQ ID NO: 192) | 9.900 |

(continued)

| Results of BIMAS HLA Peptide Binding Prediction Analysis for Binding of Human WT1 Peptides to Human HLA B 5801 | | | |
|---|---|---|---|
| Rank | Start Position | Subsequence Residue Listing | Score (Estimate of Half Time of Disassociation of a Molecule Containing This Subsequence) |
| 7 | 276 | HTTPILCGA (SEQ ID NO:115) | 7.200 |
| 8 | 218 | RTPYSSDNL (SEQ ID NO: 194) | 6.600 |
| 9 | 152 | VTFDGTPSY (SEQ ID NO: 244) | 6.000 |
| 10 | 40 | FAPPGASAY (SEQ ID NO:74) | 6.000 |
| 11 | 213 | QALLLRTPY (SEQ ID NO:160) | 4.500 |
| 12 | 347 | HTGEKPYQC (SEQ ID NO: 112) | 4.400 |
| 13 | 252 | AGSSSSVKW (SEQ ID NO: 32) | 4.400 |
| 14 | 211 | GSQALLLRT (SEQ ID NO: 102) | 4.356 |
| 15 | 174 | HSFKHEDPM (SEQ ID NO: 110) | 4.000 |
| 16 | 317 | TSEKRPFMC (SEQ ID NO: 233) | 4.000 |
| 17 | 26 | LPVSGAAQW (SEQ ID NO: 138) | 4.000 |
| 18 | 289 | HTHGVFRGI (SEQ ID NO: 113) | 3.600 |
| 19 | 222 | SSDNLYQMT (SEQ ID NO: 217) | 3.300 |
| 20 | 96 | FSGQFTGTA (SEQ ID NO:82) | 3.300 |

Table XXX

| Results of BIMAS HLA Peptide Binding Prediction Analysis for Binding of Human WT1 Peptides to Human HLA CW0301 | | | |
|---|---|---|---|
| Rank | Start Position | Subsequence Residue Listing | Score (Estimate of Half Time of Disassociation of a Molecule Containing This Subsequence) |
| 1 | 10 | ALLPAVPSL (SEQ ID NO:34) | 100.000 |
| 2 | 332 | KRYFKLSHL (SEQ ID NO: 127) | 48.000 |
| 3 | 126 | RMFPNAPYL (SEQ ID NO: 185) | 36.000 |
| 4 | 3 | SDVRDLNAL (SEQ ID NO: 206) | 30.000 |

(continued)

| Results of BIMAS HLA Peptide Binding Prediction Analysis for Binding of Human WT1 Peptides to Human HLA CW0301 | | | |
|---|---|---|---|
| Rank | Start Position | Subsequence Residue Listing | Score (Estimate of Half Time of Disassociation of a Molecule Containing This Subsequence) |
| 5 | 239 | NQMNLGATL (SEQ ID NO: 151) | 24.000 |
| 6 | 225 | NLYQMTSQL (SEQ ID NO: 147) | 24.000 |
| 7 | 180 | DPMGQQGSL (SEQ ID NO: 59) | 20.000 |
| 8 | 362 | RRFSRSDQL (SEQ ID NO: 187) | 12.000 |
| 9 | 329 | GCNKRYFKL (SEQ ID NO: 90) | 10.000 |
| 10 | 286 | YRIHTHGVF (SEQ ID NO: 252) | 10.000 |
| 11 | 301 | RRVPGVAPT (SEQ ID NO: 189) | 10.000 |
| 12 | 24 | CALPVSGAA (SEQ ID NO:43) | 10.000 |
| 13 | 136 | SCLESQPAI (SEQ ID NO: 198) | 7.500 |
| 14 | 437 | MHQRNMTKL (SEQ ID NO: 143) | 7.200 |
| 15 | 390 | RKFSRSDHL (SEQ ID NO: 183) | 6.000 |
| 16 | 423 | KKFARSDEL (SEQ ID NO: 122) | 6.000 |
| 17 | 92 | FTVHFSGQF (SEQ ID NO:85) | 5.000 |
| 18 | 429 | DELVRHHNM (SEQ ID NO: 53) | 5.000 |
| 19 | 130 | NAPYLPSCL (SEQ ID NO: 144) | 4.800 |
| 20 | 30 | GAAQWAPVL (SEQ ID NO: 86) | 4.000 |

Table XXXI

| Results of BIMAS HLA Peptide Binding Prediction Analysis for Binding of Human WT1 Peptides to Human HLA CW0401 | | | |
|---|---|---|---|
| Rank | Start Position | Subsequence Residue Listing | Score (Estimate of Half Time of Disassociation of a Molecule Containing This Subsequence) |
| 1 | 356 | DFKDCERRF (SEQ ID NO: 55) | 120.000 |

(continued)

| Results of BIMAS HLA Peptide Binding Prediction Analysis for Binding of Human WT1 Peptides to Human HLA CW0401 | | | |
|---|---|---|---|
| Rank | Start Position | Subsequence Residue Listing | Score (Estimate of Half Time of Disassociation of a Molecule Containing This Subsequence) |
| 2 | 334 | YFKLSHLQM (SEQ ID NO: 248) | 100.000 |
| 3 | 180 | DPMGQQGSL (SEQ ID NO: 59) | 88.000 |
| 4 | 163 | TPSHHAAQF (SEQ ID NO: 228) | 52.800 |
| 5 | 327 | YPGCNKRYF (SEQ ID NO: 250) | 40.000 |
| 6 | 285 | QYRIHTHGV (SEQ ID NO: 175) | 27.500 |
| 7 | 424 | KFARSDELV (SEQ ID NO: 119) | 25.000 |
| 8 | 326 | AYPGCNKRY (SEQ ID NO: 42) | 25.000 |
| 9 | 192 | QYSVPPPVY (SEQ ID NO: 176) | 25.000 |
| 10 | 417 | RWPSCQKKF (SEQ ID NO: 196) | 22.000 |
| 11 | 278 | TPILCGAQY (SEQ ID NO: 227) | 12.000 |
| 12 | 10 | ALLPAVPSL (SEQ ID NO:34) | 11.616 |
| 13 | 141 | QPAIRNQGY (SEQ ID NO: 170) | 11.000 |
| 14 | 303 | VPGVAPTLV (SEQ ID NO: 242) | 11.000 |
| 15 | 219 | TPYSSDNLY (SEQ ID NO: 231) | 10.000 |
| 16 | 39 | DFAPPGASA (SEQ ID NO: 54) | 7.920 |
| 17 | 99 | QFTGTAGAC (SEQ ID NO: 165) | 6.000 |
| 18 | 4 | DVRDLNALL (SEQ ID NO:62) | 5.760 |
| 19 | 70 | SFIKQEPSW (SEQ ID NO: 210) | 5.500 |
| 20 | 63 | PPPPPPHSF (SEQ ID NO: 158) | 5.280 |

Table XXXII

| Results of BIMAS HLA Peptide Binding Prediction Analysis for Binding of Human WT1 Peptides to Human HLA CW0602 | | | |
|---|---|---|---|
| Rank | Start Position | Subsequence Residue Listing | Score (Estimate of Half Time of Disassociation of a Molecule Containing This Subsequence) |
| 1 | 332 | KRYFKLSHL (SEQ ID NO: 127) | 9.680 |
| 2 | 239 | NQMNLGATL (SEQ ID NO: 151) | 6.600 |
| 3 | 130 | NAPYLPSCL (SEQ ID NO: 144) | 6.600 |
| 4 | 7 | DLNALLPAV (SEQ ID NO: 58) | 6.000 |
| 5 | 441 | NMTKLQLAL (SEQ ID NO: 149) | 6.000 |
| 6 | 225 | NLYQMTSQL (SEQ ID NO: 147) | 6.000 |
| 7 | 4 | DVRDLNALL (SEQ ID NO:62) | 6.000 |
| 8 | 3 | SDVRDLNAL (SEQ ID NO: 206) | 4.400 |
| 9 | 10 | ALLPAVPSL (SEQ ID NO:34) | 4.000 |
| 10 | 213 | QALLLRTPY (SEQ ID NO: 160) | 3.300 |
| 11 | 319 | EKRPFMCAY (SEQ ID NO: 67) | 3.000 |
| 12 | 30 | GAAQWAPVL (SEQ ID NO: 86) | 2.200 |
| 13 | 242 | NLGATLKGV (SEQ ID NO: 146) | 2.200 |
| 14 | 292 | GVFRGIQDV (SEQ ID NO: 103) | 2.200 |
| 15 | 207 | DSCTGSQAL (SEQ ID NO: 61) | 2.200 |
| 16 | 362 | RRFSRSDQL (SEQ ID NO: 187) | 2.200 |
| 17 | 439 | QRNMTKLQL (SEQ ID NO: 173) | 2.200 |
| 18 | 295 | RGIQDVRRV (SEQ ID NO: 179) | 2.200 |
| 19 | 423 | KKFARSDEL (SEQ ID NO: 122) | 2.200 |
| 20 | 180 | DPMGQQGSL (SEQ ID NO: 59) | 2.200 |

Table XXXIII

| Results of BIMAS HLA Peptide Binding Prediction Analysis for Binding of Human WT1 Peptides to Human HLA CW0702 | | | |
|---|---|---|---|
| Rank | Start Position | Subsequence Residue Listing | Score (Estimate of Half Time of Disassociation of a Molecule Containing This Subsequence) |
| 1 | 319 | EKRPFMCAY (SEQ ID NO:67) | 26.880 |
| 2 | 326 | AYPGCNKRY (SEQ ID NO:42) | 24.000 |
| 3 | 40 | FAPPGASAY (SEQ ID NO:74) | 14.784 |
| 4 | 192 | QYSVPPPVY (SEQ ID NO: 176) | 12.000 |
| 5 | 278 | TPILCGAQY (SEQ ID NO:227) | 12.000 |
| 6 | 219 | TPYSSDNLY (SEQ ID NO: 231) | 12.000 |
| 7 | 213 | QALLLRTPY (SEQ ID NO: 160) | 8.800 |
| 8 | 125 | ARMFPNAPY (SEQ ID NO:38) | 8.000 |
| 9 | 327 | YPGCNKRYF (SEQ ID NO: 250) | 6.600 |
| 10 | 152 | VTFDGTPSY (SEQ ID NO: 244) | 5.600 |
| 11 | 141 | QPAIRNQGY (SEQ ID NO: 170) | 4.800 |
| 12 | 345 | RKHTGEKPY (SEQ ID NO: 184) | 4.000 |
| 13 | 185 | QGSLGEQQY (SEQ ID NO: 166) | 4.000 |
| 14 | 101 | TGTAGACRY (SEQ ID NO: 224) | 4.000 |
| 15 | 375 | RRHTGVKPF (SEQ ID NO: 188) | 4.000 |
| 16 | 263 | GQSNHSTGY (SEQ ID NO: 100) | 4.000 |
| 17 | 163 | TPSHHAAQF (SEQ ID NO: 228) | 3.000 |
| 18 | 33 | QWAPVLDFA (SEQ ID NO: 174) | 2.688 |
| 19 | 130 | NAPYLPSCL (SEQ ID NO: 144) | 2.640 |
| 20 | 84 | HEEQCLSAF (SEQ ID NO: 107) | 2.400 |

Table XXXIV

| Results of BIMAS HLA Peptide Binding Prediction Analysis for Binding of Human WT1 Peptides to Mouse MHC Class I Db | | | |
|---|---|---|---|
| Rank | Start Position | Subsequence Residue Listing | Score (Estimate of Half Time of Disassociation of a Molecule Containing This Subsequence) |
| 1 | 235 | CMTWNQMNL (SEQ ID NO: 49) | 5255.712 |
| 2 | 126 | RMFPNAPYL (SEQ ID NO: 185) | 1990.800 |
| 3 | 221 | YSSDNLYQM (SEQ ID NO: 253) | 930.000 |
| 4 | 228 | QMTSQLECM (SEQ ID NO: 169) | 33.701 |
| 5 | 239 | NQMNLGATL (SEQ ID NO: 151) | 21.470 |
| 6 | 441 | NMTKLQLAL (SEQ ID NO: 149) | 19.908 |
| 7 | 437 | MHQRNMTKL (SEQ ID NO: 143) | 19.837 |
| 8 | 136 | SCLESQPAI (SEQ ID NO: 198) | 11.177 |
| 9 | 174 | HSFKHEDPM (SEQ ID NO: 110) | 10.800 |
| 10 | 302 | RVPGVAPTL (SEQ ID NO: 195) | 10.088 |
| 11 | 130 | NAPYLPSCL (SEQ ID NO: 144) | 8.400 |
| 12 | 10 | ALLPAVPSL (SEQ ID NO:34) | 5.988 |
| 13 | 208 | SCTGSQALL (SEQ ID NO: 202) | 4.435 |
| 14 | 209 | CTGSQALLL (SEQ ID NO: 52) | 3.548 |
| 15 | 238 | WNQMNLGAT(SEQ ID NO: 245) | 3.300 |
| 16 | 218 | RTPYSSDNL (SEQ ID NO: 194) | 3.185 |
| 17 | 24 | CALPVSGAA (SEQ ID NO: 43) | 2.851 |
| 18 | 18 | LGGGGGCAL (SEQ ID NO: 134) | 2.177 |
| 19 | 142 | PAIRNQGYS (SEQ ID NO: 152) | 2.160 |
| 20 | 30 | GAAQWAPVL (SEQ ID NO: 86) | 1.680 |

Table XXXV

| Results of BIMAS HLA Peptide Binding Prediction Analysis for Binding of Human WT1 Peptides to Mouse MHC Class I Dd | | | |
|---|---|---|---|
| Rank | Start Position | Subsequence Residue Listing | Score (Estimate of Half Time of Disassociation of a Molecule Containing This Subsequence) |
| 1 | 112 | FGPPPPSQA (SEQ ID NO: 76) | 48.000 |
| 2 | 122 | SGQARMFPN (SEQ ID NO: 212) | 36.000 |
| 3 | 104 | AGACRYGPF (SEQ ID NO: 31) | 30.000 |
| 4 | 218 | RTPYSSDNL (SEQ ID NO: 194) | 28.800 |
| 5 | 130 | NAPYLPSCL (SEQ ID NO: 144) | 20.000 |
| 6 | 302 | RVPGVAPTL (SEQ ID NO: 195) | 20.000 |
| 7 | 18 | LGGGGGCAL (SEQ ID NO: 134) | 20.000 |
| 8 | 81 | AEPHEEQCL (SEQ ID NO: 30) | 10.000 |
| 9 | 29 | SGAAQWAPV (SEQ ID NO: 211) | 7.200 |
| 10 | 423 | KKFARSDEL (SEQ ID NO: 122) | 7.200 |
| 11 | 295 | RGIQDVRRV (SEQ ID NO: 179) | 7.200 |
| 12 | 390 | RKFSRSDHL (SEQ ID NO: 183) | 6.000 |
| 13 | 332 | KRYFKLSHL (SEQ ID NO: 127) | 6.000 |
| 14 | 362 | RRFSRSDQL (SEQ ID NO: 187) | 6.000 |
| 15 | 417 | RWPSCQKKF (SEQ ID NO: 196) | 6.000 |
| 16 | 160 | YGHTPSHHA (SEQ ID NO: 249) | 6.000 |
| 17 | 20 | GGGGCALPV (SEQ ID NO: 92) | 6.000 |
| 18 | 329 | GCNKRYFKL (SEQ ID NO: 90) | 5.000 |
| 19 | 372 | RHQRRHTGV (SEQ ID NO: 181) | 4.500 |
| 20 | 52 | GGPAPPPAP (SEQ ID NO: 93) | 4.000 |

Table XXXVI

| Results of BIMAS HLA Peptide Binding Prediction Analysis for Binding of Human WT1 Peptides to Mouse MHC Class I Kb | | | |
|---|---|---|---|
| Rank | Start Position | Subsequence Residue Listing | Score (Estimate of Half Time of Disassociation of a Molecule Containing This Subsequence) |
| 1 | 329 | GCNKRYFKL (SEQ ID NO: 90) | 24.000 |
| 2 | 225 | NLYQMTSQL (SEQ ID NO: 147) | 10.000 |
| 3 | 420 | SCQKKFARS (SEQ ID NO: 200) | 3.960 |
| 4 | 218 | RTPYSSDNL (SEQ ID NO: 194) | 3.630 |
| 5 | 437 | MHQRNMTKL (SEQ ID NO: 143) | 3.600 |
| 6 | 387 | TCQRKFSRS (SEQ ID NO: 219) | 3.600 |
| 7 | 302 | RVPGVAPTL (SEQ ID NO: 195) | 3.300 |
| 8 | 130 | NAPYLPSCL (SEQ ID NO: 144) | 3.000 |
| 9 | 289 | HTHGVFRGI (SEQ ID NO: 113) | 3.000 |
| 10 | 43 | PGASAYGSL (SEQ ID NO: 153) | 2.400 |
| 11 | 155 | DGTPSYGHT (SEQ ID NO: 56) | 2.400 |
| 12 | 273 | SDNHTTPIL (SEQ ID NO: 204) | 2.200 |
| 13 | 126 | RMFPNAPYL (SEQ ID NO: 185) | 2.200 |
| 14 | 128 | FPNAPYLPS (SEQ ID NO: 79) | 2.000 |
| 15 | 3 | SDVRDLNAL (SEQ ID NO: 206) | 1.584 |
| 16 | 207 | DSCTGSQAL (SEQ ID NO: 61) | 1.584 |
| 17 | 332 | KRYFKLSHL (SEQ ID NO: 127) | 1.500 |
| 18 | 18 | LGGGGGCAL (SEQ ID NO: 134) | 1.320 |
| 19 | 233 | LECMTWNQM (SEQ ID NO: 131) | 1.320 |
| 20 | 441 | NMTKLQLAL (SEQ ID NO: 149) | 1.200 |

Table XXXVII

| Results of BIMAS HLA Peptide Binding Prediction Analysis for Binding of Human WT1 Peptides to Mouse MHC Class I Kd | | | |
|---|---|---|---|
| Rank | Start Position | Subsequence Residue Listing | Score (Estimate of Half Time of Disassociation of a Molecule Containing This Subsequence) |
| 1 | 285 | QYRIHTHGV (SEQ ID NO: 175) | 600.000 |
| 2 | 424 | KFARSDELV (SEQ ID NO: 119) | 288.000 |
| 3 | 334 | YFKLSHLQM (SEQ ID NO: 248) | 120.000 |
| 4 | 136 | SCLESQPTI (SEQ ID NO: 199) | 115.200 |
| 5 | 239 | NQMNLGATL (SEQ ID NO: 151) | 115.200 |
| 6 | 10 | ALLPAVSSL (SEQ ID NO:35) | 115.200 |
| 7 | 47 | AYGSLGGPA (SEQ ID NO: 41) | 86.400 |
| 8 | 180 | DPMGQQGSL (SEQ ID NO: 59) | 80.000 |
| 9 | 270 | GYESDNHTA (SEQ ID NO: 105) | 72.000 |
| 10 | 326 | AYPGCNKRY (SEQ ID NO: 42) | 60.000 |
| 11 | 192 | QYSVPPPVY (SEQ ID NO: 176) | 60.000 |
| 12 | 272 | ESDNHTAPI (SEQ ID NO:70) | 57.600 |
| 13 | 289 | HTHGVFRGI (SEQ ID NO: 113) | 57.600 |
| 14 | 126 | DVRDLNALL (SEQ ID NO: 62) | 57.600 |
| 15 | 4 | CTGSQALLL (SEQ ID NO: 52) | 57.600 |
| 16 | 208 | SCTGSQALL (SEQ ID NO: 202) | 48.000 |
| 17 | 441 | NMTKLQLAL (SEQ ID NO: 149) | 48.000 |
| 18 | 207 | DSCTGSQAL (SEQ ID NO: 61) | 48.000 |
| 19 | 130 | NAPYLPSCL (SEQ ID NO: 144) | 48.000 |
| 20 | 235 | CMTWNQMNL (SEQ ID NO: 49) | 48.000 |

Table XXXVIII

| Results of BIMAS HLA Peptide Binding Prediction Analysis for Binding of Human WT1 Peptides to Mouse MHC Class I Kk | | | |
|---|---|---|---|
| Rank | Start Position | Subsequence Residue Listing | Score (Estimate of Half Time of Disassociation of a Molecule Containing This Subsequence) |
| 1 | 81 | AEPHEEQCL (SEQ ID NO: 30) | 40.000 |
| 2 | 85 | EEQCLSAFT (SEQ ID NO: 65) | 40.000 |
| 3 | 429 | DELVRHHNM (SEQ ID NO: 53) | 20.000 |
| 4 | 315 | SETSEKRPF (SEQ ID NO: 209) | 20.000 |
| 5 | 261 | TEGQSNHST (SEQ ID NO: 221) | 20.000 |
| 6 | 410 | SEKPFSCRW (SEQ ID NO: 207) | 10.000 |
| 7 | 272 | ESDNHTTPI (SEQ ID NO:71) | 10.000 |
| 8 | 318 | SEKRPFMCA (SEQ ID NO: 208) | 10.000 |
| 9 | 138 | LESQPAIRN (SEQ ID NO: 132) | 10.000 |
| 10 | 233 | LECMTWNQM (SEQ ID NO: 131) | 10.000 |
| 11 | 298 | QDVRRVPGV (SEQ ID NO: 164) | 10.000 |
| 12 | 84 | HEEQCLSAF (SEQ ID NO: 107) | 10.000 |
| 13 | 349 | GEKPYQCDF (SEQ ID NO: 91) | 10.000 |
| 14 | 289 | HTHGVFRGI (SEQ ID NO: 113) | 10.000 |
| 15 | 179 | EDPMGQQGS (SEQ ID NO: 64) | 8.000 |
| 16 | 136 | SCLESQPAI (SEQ ID NO: 198) | 5.000 |
| 17 | 280 | ILCGAQYRI (SEQ ID NO: 116) | 5.000 |
| 18 | 273 | SDNHTTPIL (SEQ ID NO: 204) | 4.000 |
| 19 | 428 | SDELVRHHN (SEQ ID NO: 203) | 4.000 |
| 20 | 3 | SDVRDLNAL (SEQ ID NO: 206) | 4.000 |

Table XXXIX

| Results of BIMAS HLA Peptide Binding Prediction Analysis for Binding of Human WT1 Peptides to Mouse MHC Class I Ld | | | |
|---|---|---|---|
| Rank | Start Position | Subsequence Residue Listing | Score (Estimate of Half Time of Disassociation of a Molecule Containing This Subsequence) |
| 1 | 163 | TPSHHAAQF (SEQ ID NO: 228) | 360.000 |
| 2 | 327 | YPGCNKRYF (SEQ ID NO: 250) | 300.000 |
| 3 | 180 | DPMGQQGSL (SEQ ID NO: 59) | 150.000 |
| 4 | 26 | LPVSGAAQW (SEQ ID NO: 138) | 93.600 |
| 5 | 278 | TPILCGAQY (SEQ ID NO: 227) | 72.000 |
| 6 | 141 | QPAIRNQGY (SEQ ID NO: 170) | 60.000 |
| 7 | 219 | TPYSSDNLY (SEQ ID NO: 231) | 60.000 |
| 8 | 303 | VPGVAPTLV (SEQ ID NO: 242) | 60.000 |
| 9 | 120 | ASSGQARMF (SEQ ID NO: 40) | 50.000 |
| 10 | 63 | PPPPPPHSF (SEQ ID NO: 158) | 45.000 |
| 11 | 113 | GPPPPSQAS (SEQ ID NO: 97) | 45.000 |
| 12 | 157 | TPSYGHTPS (SEQ ID NO: 229) | 39.000 |
| 13 | 207 | DSCTGSQAL (SEQ ID NO: 61) | 32.500 |
| 14 | 110 | GPFGPPPPS (SEQ ID NO: 96) | 30.000 |
| 15 | 82 | EPHEEQCLS (SEQ ID NO: 68) | 30.000 |
| 16 | 412 | KPFSCRWPS (SEQ ID NO: 123) | 30.000 |
| 17 | 418 | WPSCQKKFA (SEQ ID NO: 246) | 30.000 |
| 18 | 221 | YSSDNLYQM (SEQ ID NO: 253) | 30.000 |
| 19 | 204 | TPTDSCTGS (SEQ ID NO: 230) | 30.000 |
| 20 | 128 | FPNAPYLPS (SEQ ID NO: 79) | 30.000 |

Table XL

| Results of BIMAS HLA Peptide Binding Prediction Analysis for Binding of Human WT1 Peptides to Cattle HLA A20 | | | |
|---|---|---|---|
| Rank | Start Position | Subsequence Residue Listing | Score (Estimate of Half Time of Disassociation of a Molecule Containing This Subsequence) |
| 1 | 350 | EKPYQCDFK (SEQ ID NO:66) | 1000.00 |
| 2 | 319 | EKRPFMCAY (SEQ ID NO:67) | 500.000 |
| 3 | 423 | KKFARSDEL (SEQ ID NO:122) | 500.000 |
| 4 | 345 | RKHTGEKPY (SEQ ID NO: 184) | 500.000 |
| 5 | 390 | RKFSRSDHL (SEQ ID NO:183) | 500.000 |
| 6 | 137 | CLESQPAIR (SEQ ID NO:47) | 120.000 |
| 7 | 380 | VKPFQCKTC (SEQ ID NO:239) | 100.000 |
| 8 | 407 | GKTSEKPFS (SEQ ID NO:95) | 100.000 |
| 9 | 335 | FKLSHLQMH (SEQ ID NO:78) | 100.000 |
| 10 | 247 | LKGVAAGSS (SEQ ID NO: 135) | 100.000 |
| 11 | 370 | LKRHQRRHT (SEQ ID NO: 136) | 100.000 |
| 12 | 258 | VKWTEGQSN (SEQ ID NO: 240) | 100.000 |
| 13 | 398 | LKTHTRTHT (SEQ ID NO:137) | 100.000 |
| 14 | 331 | NKRYFKLSH (SEQ ID NO: 145) | 100.000 |
| 15 | 357 | FKDCERRFS (SEQ ID NO:77) | 100.000 |
| 16 | 385 | CKTCQRKFS (SEQ ID NO:46) | 100.000 |
| 17 | 294 | FRGIQDVRR (SEQ ID NO:81) | 80.000 |
| 18 | 368 | DQLKRHQRR (SEQ ID NO:60) | 80.000 |
| 19 | 432 | VRHHNMHQR (SEQ ID NO: 243) | 80.000 |
| 20 | 118 | SQASSGQAR (SEQ ID NO: 216) | 80.000 |

Table XLI

| Results of BIMAS HLA Peptide Binding Prediction Analysis for Binding of Mouse WT1 Peptides to Mouse MHC Class I A 0201 | | | |
|---|---|---|---|
| Rank | Start Position | Subsequence Residue Listing | Score (Estimate of Half Time of Disassociation of a Molecule Containing This Subsequence) |
| 1 | 126 | RMFPNAPYL (SEQ ID NO: 293) | 313.968 |
| 2 | 187 | SLGEQQYSV (SEQ ID NO: 299) | 285.163 |

(continued)

| Results of BIMAS HLA Peptide Binding Prediction Analysis for Binding of Mouse WT1 Peptides to Mouse MHC Class I A 0201 | | | |
|---|---|---|---|
| Rank | Start Position | Subsequence Residue Listing | Score (Estimate of Half Time of Disassociation of a Molecule Containing This Subsequence) |
| 3 | 10 | ALLPAVSSL (SEQ ID NO: 255) | 181.794 |
| 4 | 225 | NLYQMTSQL (SEQ ID NO: 284) | 68.360 |
| 5 | 292 | GVFRGIQDV (SEQ ID NO: 270) | 51.790 |
| 6 | 93 | TLHFSGQFT (SEQ ID NO: 302) | 40.986 |
| 7 | 191 | QQYSVPPPV (SEQ ID NO: 290) | 22.566 |
| 8 | 280 | ILCGAQYRI (SEQ ID NO: 274) | 17.736 |
| 9 | 441 | NMTKLHVAL (SEQ ID NO: 285) | 15.428 |
| 10 | 235 | CMTWNQMNL (SEQ ID NO: 258) | 15.428 |
| 11 | 7 | DLNALLPAV (SEQ ID NO: 261) | 11.998 |
| 12 | 242 | NLGATLKGM (SEQ ID NO: 283) | 11.426 |
| 13 | 227 | YQMTSQLEC (SEQ ID NO: 307) | 8.573 |
| 14 | 239 | NQMNLGATL (SEQ ID NO: 286) | 8.014 |
| 15 | 309 | TLVRSASET (SEQ ID NO: 303) | 7.452 |
| 16 | 408 | KTSEKPFSC (SEQ ID NO: 277) | 5.743 |
| 17 | 340 | LQMHSRKHT (SEQ ID NO: 280) | 4.752 |
| 18 | 228 | QMTSQLECM (SEQ ID NO: 289) | 4.044 |
| 19 | 37 | VLDFAPPGA (SEQ ID NO: 304) | 3.378 |
| 20 | 302 | RVSGVAPTL (SEQ ID NO: 295) | 1.869 |

Table XLII

| Results of BIMAS HLA Peptide Binding Prediction Analysis for Binding of Mouse WT1 Peptides to Mouse MHC Class I Db | | | |
|---|---|---|---|
| Rank | Start Position | Subsequence Residue Listing | Score (Estimate of Half Time of Disassociation of a Molecule Containing This Subsequence) |
| 1 | 221 | YSSDNLYQM (SEQ ID NO: 308) | 312.000 |
| 2 | 126 | RMFPNAPYL (SEQ ID NO: 293) | 260.000 |
| 3 | 235 | CMTWNQMNL (SEQ ID NO: 258) | 260.000 |
| 4 | 437 | MHQRNMTKL (SEQ ID NO: 281) | 200.000 |
| 5 | 238 | WNQMNLGAT (SEQ ID NO: 305) | 12.000 |
| 6 | 130 | NAPYLPSCL (SEQ ID NO: 282) | 8.580 |
| 7 | 3 | SDVRDLNAL (SEQ ID NO: 298) | 7.920 |
| 8 | 136 | SCLESQPTI (SEQ ID NO: 296) | 7.920 |
| 9 | 81 | AEPHEEQCL (SEQ ID NO: 254) | 6.600 |
| 10 | 10 | ALLPAVSSL (SEQ ID NO: 255) | 6.600 |
| 11 | 218 | RTPYSSDNL (SEQ ID NO: 294) | 6.000 |
| 12 | 441 | NMTKLHVAL (SEQ ID NO: 285) | 3.432 |
| 13 | 228 | QMTSQLECM (SEQ ID NO: 289) | 3.120 |
| 14 | 174 | HSFKHEDPM (SEQ ID NO: 272) | 3.120 |
| 15 | 242 | NLGATLKGM (SEQ ID NO: 283) | 2.640 |
| 16 | 261 | TEGQSNHGI (SEQ ID NO: 301) | 2.640 |
| 17 | 225 | NLYQMTSQL (SEQ ID NO: 284) | 2.640 |
| 18 | 207 | DSCTGSQAL (SEQ ID NO: 263) | 2.600 |
| 19 | 119 | QASSGQARM (SEQ ID NO: 288) | 2.600 |
| 20 | 18 | LGGGGGCGL (SEQ ID NO: 279) | 2.600 |

Table XLIII

| Results of BIMAS HLA Peptide Binding Prediction Analysis for Binding of Mouse WT1 Peptides to Mouse MHC Class I Kb | | | |
|---|---|---|---|
| Rank | Start Position | Subsequence Residue Listing | Score (Estimate of Half Time of Disassociation of a Molecule Containing This Subsequence) |
| 1 | 329 | GCNKRYFKL (SEQ ID NO: 268) | 24.000 |
| 2 | 225 | NLYQMTSQL (SEQ ID NO: 284) | 10.000 |
| 3 | 420 | SCQKKFARS (SEQ ID NO: 297) | 3.960 |
| 4 | 218 | RTPYSSDNL (SEQ ID NO: 294) | 3.630 |
| 5 | 437 | MHQRNMTKL (SEQ ID NO: 281) | 3.600 |
| 6 | 387 | TCQRKFSRS (SEQ ID NO: 300) | 3.600 |
| 7 | 289 | HTHGVFRGI (SEQ ID NO: 273) | 3.000 |
| 8 | 130 | NAPYLPSCL (SEQ ID NO: 282) | 3.000 |
| 9 | 43 | PGASAYGSL (SEQ ID NO: 287) | 2.400 |
| 10 | 155 | DGAPSYGHT (SEQ ID NO: 260) | 2.400 |
| 11 | 126 | RMFPNAPYL (SEQ ID NO: 293) | 2.200 |
| 12 | 128 | FPNAPYLPS (SEQ ID NO: 267) | 2.000 |
| 13 | 207 | DSCTGSQAL (SEQ ID NO: 263) | 1.584 |
| 14 | 3 | SDVRDLNAL (SEQ ID NO: 298) | 1.584 |
| 15 | 332 | KRYFKLSHL (SEQ ID NO: 276) | 1.500 |
| 16 | 233 | LECMTWNQM (SEQ ID NO: 278) | 1.320 |
| 17 | 18 | LGGGGGCGL (SEQ ID NO: 279) | 1.320 |
| 18 | 242 | NLGATLKGM (SEQ ID NO: 283) | 1.200 |
| 19 | 123 | GQARMFPN (SEQ ID NO: 269)A | 1.200 |
| 20 | 441 | NMTKLHVAL (SEQ ID NO: 285) | 1.200 |

Table XLIV

| Results of BIMAS HLA Peptide Binding Prediction Analysis for Binding of Mouse WT1 Peptides to Mouse MHC Class I Kd | | | |
|---|---|---|---|
| Rank | Start Position | Subsequence Residue Listing | Score (Estimate of Half Time of Disassociation of a Molecule Containing This Subsequence) |
| 1 | 285 | QYRIHTHGV (SEQ ID NO: 291) | 600.000 |
| 2 | 424 | KFARSDELV (SEQ ID NO: 275) | 288.000 |
| 3 | 334 | YFKLSHLQM (SEQ ID NO: 306) | 120.000 |
| 4 | 136 | SCLESQPTI (SEQ ID NO: 296) | 115.200 |
| 5 | 239 | NQMNLGATL (SEQ ID NO: 286) | 115.200 |
| 6 | 10 | ALLPAVSSL (SEQ ID NO: 255) | 115.200 |
| 7 | 47 | AYGSLGGPA (SEQ ID NO: 256) | 86.400 |
| 8 | 180 | DPMGQQGSL (SEQ ID NO: 262) | 80.000 |
| 9 | 270 | GYESDNHTA (SEQ ID NO: 271) | 72.000 |
| 10 | 192 | QYSVPPPVY (SEQ ID NO: 292) | 60.000 |
| 11 | 326 | AYPGCNKRY (SEQ ID NO: 257) | 60.000 |
| 12 | 289 | HTHGVFRGI (SEQ ID NO: 273) | 57.600 |
| 13 | 4 | DVRDLNALL (SEQ ID NO: 264) | 57.600 |
| 14 | 126 | RMFPNAPYL (SEQ ID NO: 293) | 57.600 |
| 15 | 209 | CTGSQALLL (SEQ ID NO: 259) | 48.000 |
| 16 | 86 | EQCLSAFTL (SEQ ID NO: 265) | 48.000 |
| 17 | 302 | RVSGVAPTL (SEQ ID NO: 295) | 48.000 |
| 18 | 218 | RTPYSSDNL (SEQ ID NO: 294) | 48.000 |
| 19 | 272 | ESDNHTAPI (SEQ ID NO: 266) | 48.000 |
| 20 | 225 | NLYQMTSQL (SEQ ID NO: 284) | 48.000 |

Table XLV

| Results of TSites Peptide Binding Prediction Analysis for Human WT1 Peptides Capable of Eliciting a Helper T cell Response | |
|---|---|
| Peptide | Sequence |
| p6-23 | RDLNALLPAVPSLGGGG (SEQ ID NO:1) |
| p30-35 | GAAQWA (SEQ ID NO:309) |
| p45-56 | ASAYGSLGGPAP (SEQ ID NO:310) |
| p91-105 | AFTVHFSGQFTGTAG (SEQ ID NO:311) |
| p117-139 | PSQASSGQARMFPNAPYLPSCLE (SEQ ID NO:2) |
| p167-171 | HAAQF (SEQ ID NO:312) |
| p202-233 | CHTPTDSCTGSQALLLRTPYSSDNLYQMTSQL (SEQ ID NO:313) |
| p244-262 | GATLKGVAAGSSSSVKWTE (SEQ ID NO:4) |
| p287-318 | RIHTHGVFRGIQDVRRVPGVAPTLVRSASETS (SEQ ID NO:314) |
| p333-336 | RYFK (SEQ ID NO:315) |
| p361-374 | ERRFSRSDQLKRHQ (SEQ ID NO:316) |
| p389-410 | QRKFSRSDHLKTHTRTHTGKTS (SEQ ID NO:317) |
| p421-441 | CQKKFARSDELVRHHNMHQRN (SEQ ID NO:318) |

Certain CTL peptides (shown in Table XLVI) were selected for further study. For each peptide in Table XLVI, scores obtained using BIMAS HLA peptide binding prediction analysis are provided.

Table XLVI

| WT1 Peptide Sequences and HLA Peptide Binding Predictions | | |
|---|---|---|
| Peptide | Sequence | Comments |
| p329-337 | GCNKRYFKL (SEQ ID NOs: 90 and 268) | Score 24,000 |
| p225-233 | NLYQMTSQL (SEQ ID NOs: 147 and 284) | binds also to class II and HLA A2, Kd, score 10,000 |
| p235-243 | CMTWNQMNL (SEQ ID NOs: 49 and 258) | binds also to HLA A2, score 5,255,712 |
| p126-134 | RMFPNAPYL (SEQ ID NOs: 185 and 293) | binds also to Kd, class II and HLA A2, score 1,990,800 |
| p221-229 | YSSDNLYQM (SEQ ID NOs: 253 and 308) | binds also to Ld, score 312,000 |
| p228-236 | QMTSQLECM (SEQ ID NOs: 169 and 289) | score 3,120 |
| p239-247 | NQMNLGATL (SEQ ID NOs: 151 and 286) | binds also to HLA A 0201, Kd, score 8,015 |
| mouse p136-144 | SCLESQPTI (SEQ ID NO:296) | binds also to Kd, 1mismatch to human |
| human p136-144 | SCLESQPAI (SEQ ID NO:198) | score 7,920 |
| mouse p10-18 | ALLPAVSSL (SEQ ID NO:255) | binds also to Kd, HLA A2, 1 mismatch to human |
| human p10-18 | ALLPAVPSL (SEQ ID NO:34) | score 6,600 |

Peptide binding to C57B1/6 murine MHC was confirmed using the leukemia cell line RMA-S, as described by Ljunggren et al., Nature 346:476-480, 1990. In brief, RMA-S cells were cultured for 7 hours at 26°C in complete medium supplemented with 1% FCS. A total of $10^6$ RMA-S cells were added into each well of a 24-well plate and incubated either alone or with the designated peptide (25ug/ml) for 16 hours at 26°C and additional 3 hours at 37°C in complete medium. Cells were then washed three times and stained with fluorescein isothiocyanate-conjugated anti $D^b$ or anti-$K^b$ antibody (PharMingen, San Diego, CA). Labeled cells were washed twice, resuspended and fixed in 500μl of PBS with

1% paraformaldehyde and analyzed for fluorescence intensity in a flow cytometer (Becton-Dickinson FACSCalibur®). The percentage of increase of $D^b$ or $K^b$ molecules on the surface of the RMA-S cells was measured by increased mean fluorescent intensity of cells incubated with peptide compared with that of cells incubated in medium alone.

Mice were immunized with the peptides capable of binding to murine class I MHC. Following immunization, spleen cells were stimulated *in vitro* and tested for the ability to lyse targets incubated with WT1 peptides. CTL were evaluated with a standard chromium release assay (Chen et al., Cancer Res. 54:1065-1070, 1994). $10^6$ target cells were incubated at 37°C with 150μCi of sodium $^{51}Cr$ for 90 minutes, in the presence or absence of specific peptides. Cells were washed three times and resuspended in RPMI with 5% fetal bovine serum. For the assay, $10^4$ $^{51}Cr$-labeled target cells were incubated with different concentrations of effector cells in a final volume of 200μl in U-bottomed 96-well plates. Supernatants were removed after 4 to 7 hours at 37°C, and the percentage specific lysis was determined by the formula:

% specific lysis = 100 x (experimental release - spontaneous release)/(maximum release-spontaneous release).

The results, presented in Table XLVII, show that some WT1 peptides can bind to class I MHC molecules, which is essential for generating CTL. Moreover, several of the peptides were able to elicit peptide specific CTL (Figures 9A and 9B), as determined using chromium release assays. Following immunization to CTL peptides p10-18 human, p136-144 human, p136-144 mouse and p235-243, peptide specific CTL lines were generated and clones were established. These results indicate that peptide specific CTL can kill malignant cells expressing WT1.

Table XLVII

| Binding of WT1 CTL Peptides to mouse B6 class I antigens | |
|---|---|
| Peptide | Binding Affinity to Mouse MHC Class I |
| Positive control | 91 % |
| negative control | 0.5.-1.3% |
| p235-243 | 33.6% |
| p136-144 mouse | 27.9% |
| p136-144 human | 52% |
| p10-18: human | 2.2% |
| p225-233 | 5.8% |
| p329-337 | 1.2% |
| p126-134 | 0.9% |
| p221-229 | 0.8% |
| p228-236 | 1.2% |
| p239-247 | 1% |

Example 5

Use of a WT1 Polypeptide to Elicit WT1 Specific CTL in Mice

This Example illustrates the ability of a representative WT1 polypeptide to elicit CTL immunity capable of killing WT1 positive tumor cell lines.

P117-139, a peptide with motifs appropriate for binding to class I and class II MHC, was identified as described above using TSITES and BIMAS HLA peptide binding prediction analyses. Mice were immunized as described in Example 3. Following immunization, spleen cells were stimulated *in vitro* and tested for the ability to lyse targets incubated with WT1 peptides, as well as WT1 positive and negative tumor cells. CTL were evaluated with a standard chromium release assay. The results, presented in Figures 10A-10D, show that P117 can elicit WT1 specific CTL capable of killing WT1 positive tumor cells, whereas no killing of WT1 negative cells was observed. These results demonstrate that peptide specific CTL in fact kill malignant cells expressing WT1 and that vaccine and T cell therapy are effective against malignancies that express WT1.

Similar immunizations were performed using the 9-mer class I MHC binding peptides p136-144, p225-233, p235-243 as well as the 23-mer peptide p117-139. Following immunization, spleen cells were stimulated *in vitro* with each of the 4 peptides and tested for ability to lyse targets incubated with WT1 peptides. CTL were generated specific for p136-144, p235-243 and p117-139, but not for p225-233. CTL data for p235-243 and p117-139 are presented in Figures 11A and 11B. Data for peptides p136-144 and p225-233 are not depicted.

CTL lysis demands that the target WT1 peptides are endogenously processed and presented in association with tumor cell class I MHC molecules. The above WT1 peptide specific CTL were tested for ability to lyse WT1 positive versus negative tumor cell lines. CTL specific for p235-243 lysed targets incubated with the p235-243 peptides, but failed to lyse cell lines that expressed WT1 proteins (Figure 11A). By marked contrast, CTL specific for p117-139 lysed targets incubated with p117-139 peptides and also lysed malignant cells expressing WT1 (Figure 11B). As a negative control, CTL specific for p117-139 did not lyse WT1 negative EL-4 (also referred to herein as E10).

Specificity of WT1 specific lysis was confirmed by cold target inhibition (Figures 12A-12B). Effector cells were plated for various effector: target ratios in 96-well U-bottom plates. A ten-fold excess (compared to hot target) of the indicated peptide-coated target without $^{51}$Cr labeling was added. Finally, $10^4$ $^{51}$Cr-labeled target cells per well were added and the plates incubated at 37°C for 4 hours. The total volume per well was 200μl.

Lysis of TRAMP-C by p117-139 specific CTL was blocked from 58% to 36% by EL-4 incubated with the relevant peptide p117-139, but not with EL-4 incubated with an irrelevant peptide (Figure 12A). Similarly, lysis of BLK-SV40 was blocked from 18% to 0% by EL-4 incubated with the relevant peptide p117-139 (Figure 12B). Results validate that WT1 peptide specific CTL specifically kill malignant cells by recognition of processed WT1.

Several segments with putative CTL motifs are contained within p117-139. To determine the precise sequence of the CTL epitope all potential 9-mer peptides within p117-139 were synthesized (Table XLVIII). Two of these peptides (p126-134 and p130-138) were shown to bind to H-$2^b$ class I molecules (Table XLVIII). CTL generated by immunization with p117-139 lysed targets incubated with p126-134 and p130-138, but not the other 9-mer peptides within p117-139 (Figure 13A).

The p117-139 specific CTL line was restimulated with either p126-134 or p130-138. Following restimulation with p126-134 or p130-138, both T cell lines demonstrated peptide specific lysis, but only p130-138 specific CTL showed lysis of a WT1 positive tumor cell line (Figures 13B and 13C). Thus, p130-138 appears to be the naturally processed epitope.

Table XLVIII

| Binding of WT1 CTL 9mer Peptides within p117-139 to mouse B6 class I antigens | |
|---|---|
| Peptide | Binding Affinity to Mouse MHC Class I |
| P117-125 PSQASSGQA (SEQ ID NO:221) | 2% |
| P118-126 SQASSGQAR (SEQ ID NO:216) | 2% |
| P119-127 QASSGQARM (SEQ ID NOs: 161 and 288) | 2% |
| P120-128 ASSGQARMF (SEQ ID NO:40 | 1% |
| P121-129 SSGQARMFP (SEQ ID NO:222) | 1% |
| P122-130 SGQARMFPN (SEQ ID NO:212) | 1% |
| P123-131 GQARMFPNA (SEQ ID NOs: 98 and 269) | 1% |
| P124-132 QARMFPNAP (SEQ ID NO:223) | 1% |
| P125-133 ARMFPNAPY (SEQ ID NO:38) | 1% |
| P126-134 RMFPNAPYL (SEQ ID NOs: 185 and 293) | 79% |
| P127-135 MFPNAPYLP (SEQ ID NO:224) | 2% |
| P128-136 FPNAPYLPS (SEQ ID NOs: 79 and 267) | 1% |
| P129-137 PNAPYLPSC (SEQ ID NO:225) | 1% |
| P130-138 NAPYLPSCL (SEQ ID NOs: 144 and 282) | 79% |
| P131-139 APYLPSCLE (SEQ ID NO:226) | 1% |

Example 6

Identification of WT1 Specific mRNA in Mouse Tumor Cell Lines

This Example illustrates the use of RT-PCR to detect WT1 specific mRNA in cells and cell lines.

Mononuclear cells were isolated by density gradient centrifugation, and were immediately frozen and stored at -80°C until analyzed by RT-PCR for the presence of WT1 specific mRNA. RT-PCR was generally performed as described by Fraizer et al., Blood 86:4704-4706, 1995. Total RNA was extracted from $10^7$ cells according to standard procedures. RNA pellets were resuspended in 25 $\mu$L diethylpyrocarbonate treated water and used directly for reverse transcription. The zinc-finger region (exons 7 to 10) was amplified by PCR as a 330 bp mouse cDNA. Amplification was performed in a thermocycler during one or, when necessary, two sequential rounds of PCR. AmpliTaq DNA Polymerase (Perkin Elmer Cetus, Norwalk, CT), 2.5 mM $MgCl_2$ and 20 pmol of each primer in a total reaction volume of 50$\mu$l were used. Twenty $\mu$L aliquots of the PCR products were electrophoresed on 2% agarose gels stained with ethidium bromide. The gels were photographed with Polaroid film (Polaroid 667, Polaroid Ltd., Hertfordshire, England). Precautions against cross contamination were taken following the recommendations of Kwok and Higuchi, Nature 339:237-238, 1989. Negative controls included the cDNA- and PCR-reagent mixes with water instead of cDNA in each experiment. To avoid false negatives, the presence of intact RNA and adequate cDNA generation was evaluated for each sample by a control PCR using β-actin primers. Samples that did not amplify with these primers were excluded from analysis.

Primers for amplification of WT1 in mouse cell lines were: P115: 1458-1478: 5' CCC AGG CTG CAA TAA GAG ATA 3' (forward primer; SEQ ID NO:2 1); and P116: 1767-1787: 5' ATG TTG TGA TGG CGG ACC AAT 3' (reverse primer; SEQ ID NO:22) *(see* Inoue et al, Blood 88:2267-2278, 1996; Fraizer et al., Blood 86:4704-4706, 1995).

Beta Actin primers used in the control reactions were: 5' GTG GGG CGC CCC AGG CAC CA 3' (sense primer; SEQ ID NO:23); and 5' GTC CTT AAT GTC ACG CAC GAT TTC 3' (antisense primer; SEQ ID NO:24)

Primers for use in amplifying human WT1 include: P117: 954-974: 5' GGC ATC TGA GAC CAG TGA GAA 3' (SEQ ID NO:25); and P118: 1434-1414: 5' GAG AGT CAG ACT TGA AAG CAGT 3' (SEQ ID NO:5). For nested RT-PCR, primers may be: P119: 1023-1043: 5' GCT GTC CCA CTT ACA GAT GCA 3' (SEQ ID NO:26); and P120: 1345-1365: 5' TCA AAG CGC CAG CTG GAG TTT 3' (SEQ ID NO:27).

Table XLVIII shows the results of WT1 PCR analysis of mouse tumor cell lines. Within Table IV, (+++) indicates a strong WT1 PCR amplification product in the first step RT PCR, (++) indicates a WT1 amplification product that is detectable by first step WT1 RT PCR, (+) indicates a product that is detectable only in the second step of WT1 RT PCR, and (-) indicates WT1 PCR negative.

Table XLIX

| Detection of WT1 mRNA in Mouse Tumor Cell Lines | |
|---|---|
| Cell Line | WT1 mRNA |
| K562 (human leukemia; ATCC): Positive control; (Lozzio and Lozzio, Blood 45:321-334, 1975) | +++ |
| TRAMPC (SV40 transformed prostate, B6); Foster et al., Cancer Res. 57:3325-3330, 1997 | +++ |
| BLK-SV40 HD2 (SV40-transf. fibroblast, B6; ATCC); Nature 276:510-511,1978 | ++ |
| CTLL (T-cell, B6; ATCC); Gillis, Nature 268:154-156, 1977) | + |
| FM (FBL-3 subline, leukemia, B6); Glynn and Fefer, Cancer Res. 28:434-439, 1968 | + |
| BALB 3T3 (ATCC); Aaroston and Todaro, J. Cell. Physiol. 72:141-148, 1968 | + |
| S49.1 (Lymphoma, T-cell like, B/C; ATCC); Horibata and Harris, Exp. Cell. Res. 60:61, 1970 | + |
| BNL CL.2 (embryonic liver, B/C; ATCC); Nature 276:510-511, 1978 | + |
| MethA (sarcoma, B/C); Old et al., Ann. NY Acad Sci. 101:80-106, 1962 | |
| P3.6.2.8.1 (myeloma, B/C; ATCC); Proc. Natl. Acad. Sci. USA 66:344, 1970 | - |
| P2N (leukemia, DBA/2; ATCC); Melling et al., J. Immunol. 117:1267-1274,1976 | - |
| BCL1 (lymphoma, B/C; ATCC); Slavin and Strober, Nature 272:624-626, 1977 | - |
| LSTRA (lymphoma, B/C); Glynn et al., Cancer Res. 28:434-439, 1968 | - |
| E10/EL-4 (lymphoma, B6); Glynn et al., Cancer Res. 28:434-439, 1968 | - |

From the foregoing it will be appreciated that, although specific embodiments of the invention have been described herein for purposes of illustration, various modifications may be made without deviating from the scope of the invention. Accordingly, the invention is not limited except as by the appended claims.

## Claims

1. A polypeptide wherein the polypeptide is an immunogenic portion of a native WT1 polypeptide and consists of a sequence selected from SEQ ID NO:2 or SEQ ID NO:144.

2. A polynucleotide encoding the polypeptide according to claim 1.

3. A pharmaceutical composition comprising a pharmaceutically acceptable carrier or excipient in combination with a polypeptide according to claim 1.

**4.** A pharmaceutical composition comprising a pharmaceutically acceptable carrier or excipient in combination with a polynucleotide according to claim 2

**5.** A pharmaceutical composition, comprising:

(a) an antibody or antigen-binding fragment thereof that specifically binds to the polypeptide of claim 1; and
(b) a pharmaceutically acceptable carrier or excipient.

**6.** A pharmaceutical composition, comprising:

(a) a T cell that specifically reacts with the polypeptide of claim 1; and
(b) a pharmaceutically acceptable carrier or excipient.

**7.** A pharmaceutical composition, comprising:

(a) an antigen presenting cell that expresses the polypeptide of claim 1; and
(b) a pharmaceutically acceptable carrier or excipient.

**8.** A vaccine comprising:

(a) the polypeptide according to claim 1; and
(b) a non-specific immune response enhancer that preferentially enhances a T cell response in a patient.

**9.** A vaccine comprising:

(a) a polynucleotide according to claim 2; and
(b) a non-specific immune response enhancer that preferentially enhances a T cell response in a patient.

**10.** A vaccine according to claim 8 or 9, wherein the immune response enhancer is an adjuvant.

**11.** A vaccine according to claim 8 or 9, wherein the immune response enhancer comprises a Ribi Adjuvant system based adjuvant.

**12.** A vaccine according to claim 8 or 9, wherein the immune response enhancer comprises GM-CSF.

**13.** A vaccine according to claim 8 or 9, wherein the immune response enhancer is selected from the group consisting of Montanide ISA50, Seppic MONTANIDE ISA 720, Flt3-ligand, a microsphere, a dimethyl dioctadecyl ammoniumbromide (DDA) based adjuvant, AS-1, AS-2, QS21, a saponin based adjuvant, Syntex adjuvant in its microfluidized form, MV, ddMV, an immune stimulating complex (iscom) based adjuvant and an inactivated toxin.

**14.** A vaccine, comprising:

(a) an antigen presenting cell that expresses the polypeptide of claim 1; and
(b) a non-specific immune response enhancer.

**15.** A vaccine comprising:

(a) an anti-idiotypic antibody or antigen-binding fragment thereof that is specifically bound by an antibody that specifically binds to the polypeptide according to claim 1; and
(b) non-specific immune response enhancer.

**16.** A vaccine according to any one of claims 14 or 15, wherein the immune response enhancer is an adjuvant.

**17.** A vaccine according to any one of claims 14 or 15, wherein the immune response enhancer preferentially enhances a T cell response in a patient.

**18.** The use of the pharmaceutical composition according to any one of claims 3-7 for the manufacture of a medicament for the treatment of a malignant disease associated with WT1 expression in a human patient.

**19.** The use of the vaccine according to any one of claims 8-17 for the manufacture of a medicament for the treatment of a malignant disease associated with WT1 expression in a human patient.

**20.** The use of the pharmaceutical composition according to any one of claims 3-7 for the manufacture of a medicament for inhibiting the development of a malignant disease associated with WT1 expression in a human patient.

**21.** The use of the vaccine according to any one of claims 8-17 for the manufacture of a medicament for inhibiting the development of a malignant disease associated with WT1 expression in a human patient.

**22.** The use according to any one of claims 18 through to 21, wherein the malignant disease is a leukemia.

**23.** The use according to claim 22, wherein the leukemia is acute myeloid leukemia, acute lymphocytic leukemia or chronic myeloid leukemia.

**24.** The use according to any one of claims 18 through to 21, wherein the malignant disease is a cancer.

**25.** The use according to claim 24, wherein the cancer is breast, lung, thyroid or gastrointestinal cancer or a melanoma.

**26.** An in vitro method for removing cells expressing WT1 from bone marrow, peripheral blood, or a fraction of bone marrow or peripheral blood, comprising contacting bone marrow, peripheral blood or a fraction of bone marrow or peripheral blood with T cells that specifically react with a WT1 polypeptide according to claim 1, wherein the step of contacting is performed under conditions and for a time sufficient to permit the removal of WT1 positive cells to less than 10% of the number of myeloid or lymphatic cells in the bone marrow, peripheral blood or a fraction of bone marrow or peripheral blood.

**27.** The use of bone marrow, peripheral blood or a fraction of bone marrow or peripheral blood prepared according to the method of claim 26 for the manufacture of a medicament for inhibiting the development of a malignant disease associated with WT1 expression in a patient.

**28.** The use according to claim 27, wherein the bone marrow, peripheral blood or fraction is autologous.

**29.** The use according to claim 27, wherein the bone marrow, peripheral blood or fraction is syngeneic or allogeneic.

**30.** An in vitro method for stimulating and/or expanding T cells, comprising contacting T cells with a WT1 polypeptide according to claim 1, a polynucleotide according to claim 2 and/or an antigen presenting cell that expresses a WT1 polypeptide according to claim 1, under conditions and for a time sufficient to permit the stimulation and/or expansion of T cells.

**31.** The method according to claim 30, wherein the T cells are present within bone marrow, peripheral blood or a fraction of bone marrow or peripheral blood.

**32.** The method according to claim 30, wherein the bone marrow, peripheral blood or fraction is obtained from a patient afflicted with a malignant disease associated with WT1 expression.

**33.** The method according to claim 30, wherein the bone marrow, peripheral blood or fraction is obtained from a mammal that is not afflicted with a malignant disease associated with WT1 expression.

**34.** The method according to claim 30, wherein the T cells are cloned prior to expansion.

**35.** The use of T cells obtainable by the method of any one of claims 30 through to 34 for the manufacture of a medicament for inhibiting the development of a malignant disease associated with WT1 expression in a patient.

**36.** The use of any one or more of the following:

(a) a polypeptide according to claim 1;
(b) a polynucleotide according to claim 2;
(c) an antigen-presenting cell that expresses a polypeptide according to claim 1;
(d) an antibody or antigen-binding fragment thereof that specifically binds to a WT1 polypeptide of claim 1; and

(e) a T cell that specifically reacts with a WT1 polypeptide of claim 1; for the manufacture of a medicament for monitoring the effectiveness of an immunization or therapy for a malignant disease associated with WT1 expression in a patient

**37.** The use of any one or more of the following:

(a) a polypeptide according to claim 1;
(b) a polynucleotide according to claim 2;
(c) an antigen-presenting cell that expresses a polypeptide according to claim 1;
(d) an antibody or antigen-binding fragment thereof that specifically binds to a WT1 polypeptide of claim 1; and
(e) a T cell that specifically reacts with a WT1 polypeptide of claim 1 for the manufacture of a medicament for determining the presence or absence of a malignant disease associated with WT1 expression in a patient.

**38.** The use according to claim 37, wherein the malignant disease is a cancer or a leukemia.

**39.** A polypeptide according to claim 1 or a polynucleotide according to claim 2, for use as an active therapeutic substance.

**40.** A polypeptide according to claim 1 or a polynucleotide according to claim 2 for use in enhancing or inducing an immune response in a patient.

**41.** A polypeptide according to claim 1 or a polynucleotide according to claim 2 for use in inhibiting the development of a malignant disease associated with WT1 expression in a human patient.

**Patentansprüche**

**1.** Ein Polypeptid, wobei das Polypeptid ein immunogener Teil eines natürlichen WT1 Polypeptids ist und aus einer Sequenz, die aus SEQ ID NO:2 oder SEQ ID NO:144 ausgewählt wird, besteht.

**2.** Ein Polynukleotid, das für das Polypeptid gemäß Anspruch 1 kodiert.

**3.** Eine pharmazeutische Zusammensetzung, die einen pharmazeutisch geeigneten Träger oder Arzneimittelträger zusammen mit einem Polypeptid gemäß Anspruch 1 umfasst.

**4.** Eine pharmazeutische Zusammensetzung, die einen pharmazeutisch geeigneten Träger oder Arzneimittelträger zusammen mit einem Polynukleotid gemäß Anspruch 2 umfasst.

**5.** Eine pharmazeutische Zusammensetzung, die umfasst:

(a) einen Antikörper oder ein Antigen-bindendes Fragment davon, der/das spezifisch an das Polypeptid nach Anspruch 1 bindet; und
(b) einen pharmazeutisch geeigneten Träger oder Arzneimittelträger.

**6.** Eine pharmazeutische Zusammensetzung, die umfasst:

(a) eine T-Zelle, die spezifisch mit dem Polypeptid nach Anspruch 1 reagiert; und
(b) einen pharmazeutisch geeigneten Träger oder Arzneimittelträger.

**7.** Eine pharmazeutische Zusammensetzung, die umfasst:

(a) eine Antigen-präsentierende Zelle, die das Polypeptid nach Anspruch 1 exprimiert; und
(b) einen pharmazeutisch geeigneten Träger oder Hilfsstoff.

**8.** Ein Impfstoff, der umfasst:

(a) das Polypeptid gemäß Anspruch 1; und
(b) einen nicht-spezifischen Immunantwortverstärker, der bevorzugt eine T-Zellenantwort bei einem Patienten verstärkt.

**9.** Ein Impfstoff, der umfasst:

(a) ein Polynukleotid gemäß Anspruch 2; und
(b) einen nicht-spezifischen Immunantwortverstärker, der bevorzugt eine T-Zellenantwort bei einem Patienten verstärkt.

**10.** Ein Impfstoff gemäß Anspruch 8 oder 9, wobei der Immunantwortverstärker ein Hilfsmittel ist.

**11.** Ein Impfstoff gemäß Anspruch 8 oder 9, wobei der Immunantwortverstärker ein auf einem Ribi Hilfsmittelsystem basierendes Hilfsmittel umfasst.

**12.** Ein Impfstoff gemäß Anspruch 8 oder 9, wobei der Immunantwortverstärker GM-CSF umfasst.

**13.** Ein Impfstoff gemäß Anspruch 8 oder 9, wobei der Immunantwortverstärker aus der Gruppe, die aus Montanide ISA50, Seppic MONTANIDE ISA 720, Flt3-Ligand, einem Mikrokügelchen, einem auf Dimethyldioctadecylammoniumbromid (DDA) basierenden Hilfsmittel, AS-1, AS-2, QS21, einem auf Saponin basierenden Hilfsmittel, Syntex-Hilfsmittel in seiner mikrofluidisierten Form, MV, ddMV, einem auf Immunstimulationskomplex (Iscom) basierenden Hilfsmittel und einem inaktivierten Toxin besteht, ausgewählt wird.

**14.** Ein Impfstoff, der umfasst:

(a) eine Antigen-präsentierende Zelle, die das Polypeptid nach Anspruch 1 exprimiert; und
(b) einen nicht-spezifischen Immunantwortverstärker.

**15.** Ein Impfstoff, der umfasst:

(a) einen anti-idiotypischen Antikörper oder ein Antigen-bindendes Fragment davon, der/das spezifisch durch einen Antikörper, der spezifisch an das Polypeptid gemäß Anspruch 1 bindet, gebunden ist; und
(b) einen nicht-spezifischen Immunantwortverstärker.

**16.** Ein Impfstoff gemäß einem der Ansprüche 14 oder 15, wobei der Immunantwortverstärker ein Hilfsmittel ist.

**17.** Ein Impfstoff gemäß einem der Ansprüche 14 oder 15, wobei der Immunantwortverstärker bevorzugt eine T-Zellenantwort in einem Patienten verstärkt.

**18.** Die Verwendung der pharmazeutischen Zusammensetzung gemäß einem der Ansprüche 3-7 zur Herstellung eines Medikaments zur Behandlung einer bösartigen Erkrankung, die mit WT1 Exprimierung bei einem menschlichen Patienten verbunden ist.

**19.** Die Verwendung des Impfstoffs gemäß einem der Ansprüche 8-17 für die Herstellung eines Medikaments zur Behandlung einer bösartigen Erkrankungen, die mit einer WT1 Exprimierung bei einem menschlichen Patienten verbunden ist.

**20.** Die Verwendung der pharmazeutischen Zusammensetzung gemäß einem der Ansprüche 3-7 für die Herstellung eines Medikaments zur Inhibierung der Entwicklung einer bösartigen Erkrankung, die mit WT1 Exprimierung bei einem menschlichen Patienten verbunden ist.

**21.** Die Verwendung des Impfstoffs gemäß einem der Ansprüche 8-17 für die Herstellung eines Medikaments zur Inhibierung der Entwicklung einer bösartigen Erkrankung, die mit WT1 Exprimierung bei einem menschlichen Patienten verbunden ist.

**22.** Die Verwendung gemäß einem der Ansprüche 18 bis 21, wobei die bösartige Erkrankung Leukämie ist.

**23.** Die Verwendung gemäß Anspruch 22, wobei die Leukämie akute Knochenmarkleukämie, akute Lymphozytleukämie oder chronische Knochenmarkleukämie ist.

**24.** Die Verwendung gemäß einem der Ansprüche 18 bis 21, wobei die bösartige Erkrankung Krebs ist.

**25.** Die Verwendung gemäß Anspruch 24, wobei der Krebs Brust-, Lungen-, Thyroid- oder Magen-Darm-Krebs oder Melanom ist.

**26.** Ein in vitro Verfahren zur Entfernung von Zellen, die WT1 aus Knochenmark, peripherem Blut oder einer Fraktion von Knochenmark oder peripherem Blut exprimieren, das das In-Kontakt-Bringen von Knochenmark, peripherem Blut oder einer Fraktion von Knochenmark oder peripherem Blut mit T-Zellen, die spezifisch mit einem WT1 Polypeptid gemäß Anspruch 1 reagieren, umfasst, wobei der Schritt des In-Kontakt-Bringens unter Bedingungen und für eine ausreichende Zeit durchgeführt wird, um die Entfernung von WT1 positiven Zellen auf weniger als 10% der Anzahl von myeloiden oder lymphatischen Zellen in dem Knochenmark, peripheren Blut oder einer Fraktion von Knochenmark oder peripherem Blut zu ermöglichen.

**27.** Die Verwendung von Knochenmark, peripherem Blut oder einer Fraktion von Knochenmark oder peripherem Blut, das gemäß dem Verfahren nach Anspruch 26 hergestellt wurde, für die Herstellung eines Medikaments zur Inhibierung der Entwicklung einer bösartigen Erkrankung, die mit WT1 Exprimierung bei einem Patienten verbunden ist.

**28.** Die Verwendung gemäß Anspruch 27, wobei das Knochenmark, das periphere Blut oder die Fraktion autolog ist.

**29.** Die Verwendung gemäß Anspruch 27, wobei das Knochenmark, das periphere Blut oder die Fraktion syngenisch oder allogenisch ist.

**30.** Ein in vitro Verfahren zur Stimulierung und/oder Expandierung von T-Zellen, das das In-Kontakt-Bringen von T-Zellen mit einem WT1 Polypeptid gemäß Anspruch 1, einem Polynukleotid gemäß Anspruch 2 und/oder einer Antigenpräsentierenden Zelle, die ein WT1 Polypeptid gemäß Anspruch 1 exprimiert, unter Bedingungen und für eine ausreichende Zeit umfasst, die ausreicht, um die Stimulierung und/oder Expansion von T-Zellen zu ermöglichen.

**31.** Das Verfahren gemäß Anspruch 30, wobei die T-Zellen in Knochenmark, peripherem Blut oder einer Fraktion von Knochenmark oder peripherem Blut vorliegen.

**32.** Das Verfahren gemäß Anspruch 30, wobei das Knochenmark, das periphere Blut oder die Fraktion von einem Patienten erhalten wird, der von einer bösartigen Erkrankung, die mit WT1 Exprimierung verbunden ist, befallen ist.

**33.** Das Verfahren gemäß Anspruch 30, wobei das Knochenmark, das periphere Blut oder die Fraktion von einem Säugetier erhalten wird, das nicht von einer bösartigen Erkrankung, die mit WT1 Exprimierung verbunden ist, befallen ist.

**34.** Das Verfahren gemäß Anspruch 30, wobei die T-Zellen vor der Expansion geklont werden.

**35.** Die Verwendung von T-Zellen, die durch das Verfahren nach einem der Ansprüche 30 bis 34 erhältlich sind, für die Herstellung eines Medikaments zur Inhibierung der Entwicklung einer bösartigen Erkrankung, die mit WT1 Exprimierung bei einem Patienten verbunden ist.

**36.** Die Verwendung von einem oder mehreren der folgenden:

(a) ein Polypeptid gemäß Anspruch 1;
(b) ein Polynukleotid gemäß Anspruch 2;
(c) eine Antigen-präsentierende Zelle, die ein Polypeptid gemäß Anspruch 1 exprimiert;
(d) ein Antikörper oder Antigen-bindendes Fragment davon, der/das spezifisch an ein WT1 Polypeptid nach Anspruch 1 bindet; und
(e) eine T-Zelle, die spezifisch mit einem WT1 Polypeptid nach Anspruch 1 reagiert;

für die Herstellung eines Medikaments zur Beobachtung der Wirksamkeit einer Immunisierung oder Therapie für eine bösertige Erkrankungen, die mit WT1 Exprimierung bei einem Patienten verbunden ist.

**37.** Die Verwendung von einem oder mehreren der folgenden:

(a) ein Polypeptid gemäß Anspruch 1;
(b) ein Polynukleotid gemäß Anspruch 2;
(c) eine Antigen-präsentierends Zelle, die ein Polypeptid gemäß Anspruch 1 exprimiert;

(d) ein Antikörper oder Antigen-bindendes Fragment davon, der/das spezifisch an ein WT1 Polypeptid nach Anspruch 1 bindet; und
(e) eine T-Zelle, die spezifisch mit einem WT1 Polypeptid nach Anspruch 1 reagiert;

für die Herstellung eines Medikaments zur Bestimmung des Vorhandenseins oder des Fehlens einer bösartigen Erkrankungen, die mit WT1 Exprimierung bein einem Patienten verbunden ist.

**38.** Die Verwendung gemäß Anspruch 37, wobei die bösartige Erkrankung Krebs oder Leukämie ist.

**39.** Ein Polypeptid gemäß Anspruch 1 oder ein Polynukleotid gemäß Anspruch 2 für die Verwendung als eine aktive therapeutische Substanz.

**40.** Ein Polypeptid gemäß Anspruch 1 oder ein Polynukleotid gemäß Anspruch 2 für die Verwendung bei der Verstärkung oder Induzierung einer Immunantwort bei einem Patienten.

**41.** Ein Polypeptid gemäß Anspruch 1 oder ein Polynukleotid gemäß Anspruch 2 für die Verwendung bei der Inhibierung der Entwicklung einer bösartigen Erkrankung, die mit WT1 Exprimierung bei einem menschlichen Patienten verbunden ist.

## Revendications

**1.** Un polypeptide dans lequel le polypeptide est une portion immunogénique du polypeptide natif WT1 et se compose d'une séquence choisie à partir de la SEQ ID NO : 2 ou de la SEQ ID NO : 144.

**2.** Un polynucléotide encodant le polypeptide selon la revendication 1.

**3.** Une composition pharmaceutique comportant un transporteur ou un excipient acceptables sur le plan pharmaceutique en combinaison avec un polypeptide en accord avec la revendication 1.

**4.** Une composition pharmaceutique comportant un transporteur ou un excipient acceptables sur le plan pharmaceutique en combinaison avec un polynucléotide en accord avec la revendication 2.

**5.** Une composition pharmaceutique, comportant :

(a)un anticorps ou un fragment d'un antigène-liant de celui-ci qui se lie de façon spécifique au polypeptide selon la revendication 1 ; et
(b) un transporteur ou un excipient acceptable sur le plan pharmaceutique.

**6.** Une composition pharmaceutique, comportant :

(a)une cellule T réagissant de façon spécifique au polypeptide selon la revendication 1 ; et
(b)un transporteur ou un excipient acceptable sur le plan pharmaceutique.

**7.** Une composition pharmaceutique, comportant :

(a)une cellule présentatrice d'antigène qui exprime le polypeptide selon la revendication 1 ; et
(b) un transporteur ou un excipient acceptable sur le plan pharmaceutique.

**8.** Un vaccin comportant :

(a) le polypeptide selon la revendication 1 ; et
(b) un amplificateur non spécifique de la réponse immune qui amplifie de façon préférentielle la réponse des cellules T d'un patient.

**9.** Un vaccin comportant :

(a) le polynucléotide selon la revendication 2 ; et

(b) un amplificateur non spécifique de la réponse immune qui amplifie de façon préférentielle la réponse des cellules T d'un patient.

**10.** Un vaccin selon la revendication 8 ou 9, dans lequel l'amplificateur de la réponse immune est un adjuvant.

**11.** Un vaccin selon la revendication 8 ou 9, dans lequel l'amplificateur de la réponse immune comporte un adjuvant à base de Ribi Ajuvant Système.

**12.** Un vaccin en accord avec la revendication 8 ou 9, dans lequel l'amplificateur de la réponse immune comporte du GM-CSF.

**13.** Un vaccin selon la revendication 8 ou 9, dans lequel l'amplificateur de la réponse immune est choisi à partir du groupe constitué du Montanide ISA50, du Seppic Montanide ISA 720, du ligand Flt3, de microsphère, d'adjuvant à base de bromure de Diméthyle Dioctadécyle Ammonium (DDA), de l'AS-1, de l'AS-2, du QS21, d'adjuvant à base de saponine, de l'adjuvant Syntex dans sa forme micro fluidisée, du MV, du ddMV, d'un adjuvant à base de complexe immun stimulant (iscom) et de toxine inactivée.

**14.** Un vaccin, comportant :

(a) une cellule présentatrice d'antigène qui exprime le polypeptide selon la revendication 1 ; et
(b) un amplificateur non spécifique de la réponse immune.

**15.** Un vaccin comportant:

(a)un anticorps anti-idiotype ou un fragment antigène-liant de celui-ci qui est spécifiquement lié par un anticorps se liant spécifiquement a un polypeptide en accord avec la revendication 1 ; et
(b)un amplificateur non spécifique de la réponse immune.

**16.** Un vaccin selon l'une quelconque des revendications 14 ou 15, dans lequel l'amplificateur de la réponse immune est un adjuvant.

**17.** Un vaccin selon l'une quelconque des revendications 14 ou 15, dans lequel l'amplificateur de la réponse immune amplifie préférentiellement la réponse des cellules T chez un patient.

**18.** L'utilisation d'un composition pharmaceutique selon l'une quelconque des revendications 3-7 pour la fabrication d'un médicament pour le traitement d'une pathologie maligne associée à l'expression WT1 chez un patient humain.

**19.** L'utilisation d'un vaccin selon l'une quelconque des revendications 8-17 pour la fabrication d'un médicament pour le traitement d'une pathologie maligne associée à l'expression WT1 chez un patient humain.

**20.** L'utilisation d'une composition pharmaceutique selon l'une quelconque des revendications 3-7 pour la fabrication d'un médicament pour le traitement d'une pathologie maligne associée à une expression WT1 chez un patient humain.

**21.** L'utilisation d'un vaccin selon l'une quelconque des revendications 8-17 pour la fabrication d'un médicament capable d'inhiber le développement d'une pathologie maligne associée à l'expression WT1 chez un patient humain.

**22.** L'utilisation selon l'une quelconque des revendications de 18 à 21, dans laquelle la pathologie maligne est une leucémie.

**23.** L'utilisation selon la revendication 22, dans laquelle la leucémie est une leucémie aigue myéloïde, une leucémie aigue lymphoïde ou une leucémie myéloïde chronique.

**24.** L'utilisation selon l'une quelconque des revendications 18 à 21, dans laquelle la pathologie maligne est un cancer.

**25.** L'utilisation selon la revendication 24, dans laquelle le cancer est mammaire, pulmonaire, thyroïdien ou gastro-intestinal ou un mélanome.

**26.** Une méthode *in vitro* pour retirer les cellules exprimant le WT1 de la moelle osseuse, du sang périphérique, ou d'une partie de la moelle osseuse ou du sang périphérique, comportant la mise en contact de la moelle osseuse, du sang périphérique ou d'une fraction de moelle osseuse ou du sang périphérique avec des cellules T lesquelles réagissent de façon spécifique avec un polypeptide WT1 selon la revendication 1, dans laquelle l'étape de mise en contact est réalisée dans des conditions et selon un temps suffisant afin de permettre le retrait des cellules positives au WT1 jusqu'à moins de 10% du nombre de cellules myéloïdes ou lymphatiques dans la moelle osseuse, le sang périphérique ou une fraction de moelle osseuse ou du sang périphérique.

**27.** L'utilisation de moelle osseuse, de sang périphérique ou d'une fraction de moelle osseuse ou de sang périphérique préparé selon la méthode de la revendication 26 pour la fabrication d'un médicament inhibant le développement d'une pathologie maligne associée à l'expression du WT1 chez un patient.

**28.** L'utilisation selon la revendication 27, dans laquelle la moelle osseuse, le sang périphérique ou une fraction, est autologue.

**29.** L'utilisation selon la revendication 27, dans laquelle la moelle osseuse, le sang périphérique ou une fraction, est syngénique ou allogénique.

**30.** Une méthode *in vitro* pour la préparation et/ou la multiplication de cellules T, comportant la mise en contact entre des cellules et le polypeptide WT1 selon la revendication 1, un polynucléotide selon la revendication 2 et/ou une cellule présentatrice d'antigène exprimant le polypeptide WT1 selon la revendication 1, dans des conditions et selon un temps suffisant afin de permettre la stimulation et/ou la multiplication des cellules T.

**31.** La méthode selon la revendication 30, dans laquelle les cellules T sont présentes à l'intérieur de la moelle osseuse, dans le sang périphérique ou une fraction de moelle osseuse ou de sang périphérique.

**32.** La méthode selon la revendication 30, dans laquelle la moelle osseuse, le sang périphérique ou leur fraction est obtenue à partir de patient atteint d'une pathologie maligne associé à l'expression du WT1.

**33.** La méthode selon la revendication 30, dans laquelle la moelle osseuse, le sang périphérique ou la fraction est obtenue à partir de mammifère qui n'est pas atteint d'une pathologie maligne associé à l'expression du WT1.

**34.** La méthode selon la revendication 30, dans laquelle les cellules T sont clonées préalablement à leur multiplication.

**35.** L'utilisation de cellules T obtenues selon la méthode de l'une quelconque des revendications de 30 à 34 pour la fabrication d'un médicament afin d'inhiber le développement de pathologie maligne associé à l'expression de WT1 chez un patient.

**36.** L'utilisation de l'un quelconque ou plusieurs des points suivants :

(a)un polypeptide selon la revendication 1;
(b)un polynucléotide selon la revendication 2;
(c)une cellule présentatrice de l'antigène qui exprime un polypeptide selon la revendication 1 ;
(d)un anticorps ou un fragment antigène-liant de celui-ci qui lie spécifiquement le polypeptide WT1 selon la revendication 1 ; et
(e)une cellule T qui réagie spécifiquement avec le polypeptide WT1 selon la revendication 1 ; pour la fabrication d'un médicament pour la surveillance de l'efficacité d'une immunisation ou d'une thérapeutique d'une pathologie maligne associée avec l'expression du WT1 chez un patient.

**37.** L'utilisation de l'un quelconque ou de plusieurs des points suivants :

(a)un polypeptide selon la revendication 1 ;
(b)un polynucléotide selon la revendication 2 ;
(c)une cellule présentatrice de l'antigène qui exprime un polypeptide selon la revendication 1 ;
(d)un anticorps ou un fragment antigène-liant de celui-ci qui lie spécifiquement le polypeptide WT1 selon la revendication 1 ; et
(e)une cellule T qui réagie spécifiquement avec le polypeptide WT1 selon la revendication 1 ; pour la fabrication d'un médicament dont l'action est de déterminer la présence ou l'absence d'une pathologie maligne exprimant le WT1 chez un patient.

**38.** L'utilisation selon la revendication 37, dans laquelle la pathologie maligne est un cancer ou une leucémie.

**39.** Un polypeptide selon la revendication 1 ou un polynucléotide selon la revendication 2, pour l'utilisation comme une substance thérapeutique active.

**40.** Un polypeptide selon la revendication 1 ou un polynucléotide selon la revendication 2 pour l'utilisation dans l'amplification ou l'induction d'une réponse immune chez un patient.

**41.** Un polypeptide selon la revendication 1 ou un polynucléotide selon la revendication 2 pour l'utilisation dans l'inhibition du développement d'une pathologie maligne associé avec l'expression du WT1 chez un patient.

HU: MGSDVRDLNALLPAVPSLGGGGGCALPVSGAAQWAPVLDFAPPGASAYGSL
MO: MGSDVRDLNALLPAVSSLGGGGGCGLPVSGAAQWAPVLDFAPPGASAYGSL

HU: GGPAPPPAPPPPPPPPPHSFIKQEPSWGGAEPHEEQCLSAFTVHFSGQFTGTAG
MO: GGPAPPPAPPPPPPPPPHSFIKQEPSWGGAEPHEEQCLSAFTLHFSGQFTGTAG

HU: ACRYGPFGPPPPSQASSGQARMFPNAPYLPSCLESQPAIRNQGYSTVTFDGTPS
MO: ACRYGPFGPPPPSQASSGQARMFPNAPYLPSCLESQPTIRNQGYSTVTFDGAPS

HU: YGHTPSHHAAQFPNHSFKHEDPMGQQGSLGEQQYSVPPPVYGCHTPTDSCTG
MO: YGHTPSHHAAQFPNHSFKHEDPMGQQGSLGEQQYSVPPPVYGCHTPTDSCTG

HU: SQALLLRTPYSSDNLYQMTSQLECMTWNQMNLGATLKGVAAGSSSSVKWTE
MO: SQALLLRTPYSSDNLYQMTSQLECMTWNQMNLGATLKGMAAGSSSSVKWTE

HU: GQSNHSTGYESDNHTTPILCGAQYRIHTHGVFRGIQDVRRVPGVAPTLVRSAS
MO: GQSNHGIGYESDNHTAPILCGAQYRIHTHGVFRGIQDVRRVSGVAPTLVRSAS

HU: ETSEKRPFMCAYPGCNKRYFKLSHLQMHSRKHTGEKPYQCDFKDCERRFSR
MO: ETSEKRPFMCAYPGCNKRYFKLSHLQMHSRKHTGEKPYQCDFKDCERRFSR

HU: SDQLKRHQRRHTGVKPFQCKTCQRKFSRSDHLKTHTRTHTGKTSEKPFSCR
MO: SDQLKRHQRRHTGVKPFQCKTCQRKFSRSDHLKTHTRTHTGKTSEKPFSCR

HU: WPSCQKKFARSDELVRHHNMHQRNMTKLQLAL
MO: WHSCQKKFARSDELVRHHNMHQRNMTKLHVAL

*Fig. 1*

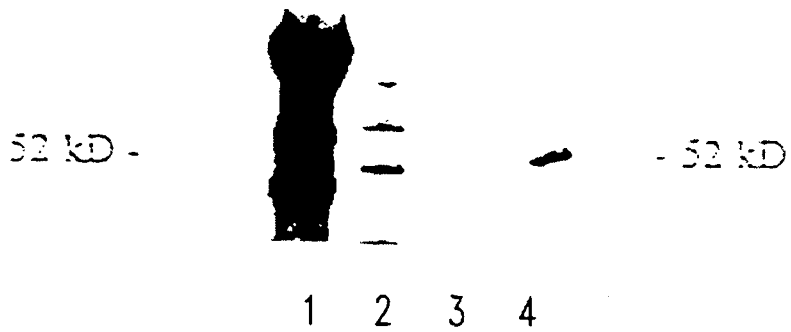


*Fig. 2*

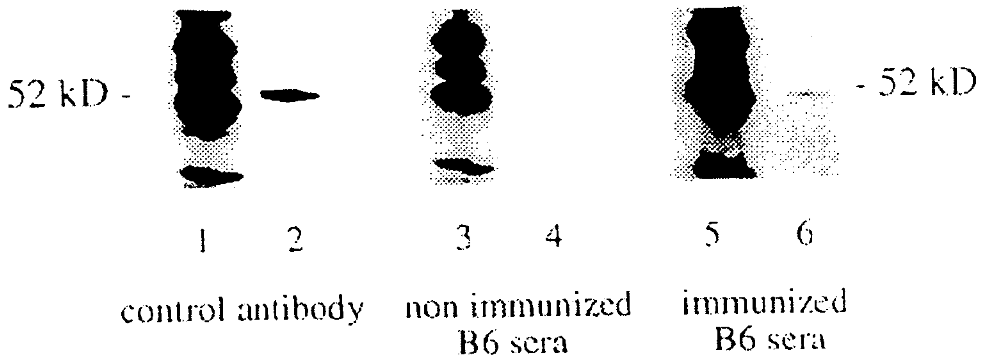
52 kD -
- 52 kD
1
2
3
4
5
6
control antibody
non immunized B6 sera
immunized B6 sera

*Fig. 3*

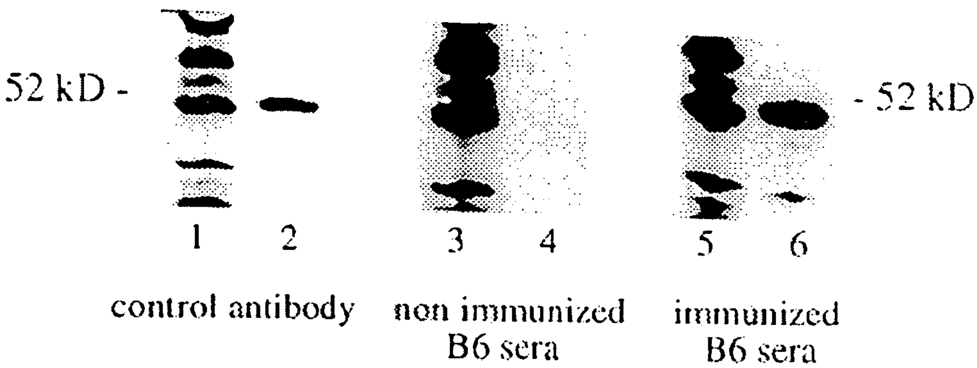
52 kD -
- 52 kD
1
2
3
4
5
6
control antibody
non immunized B6 sera
immunized B6 sera

*Fig. 4*

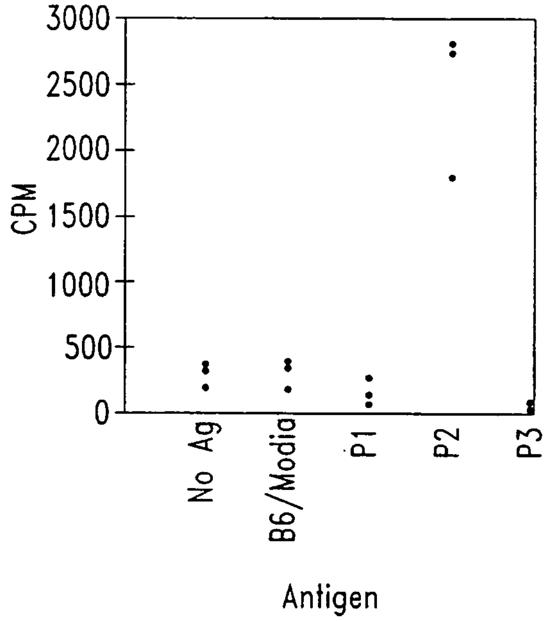


*Fig. 5A*

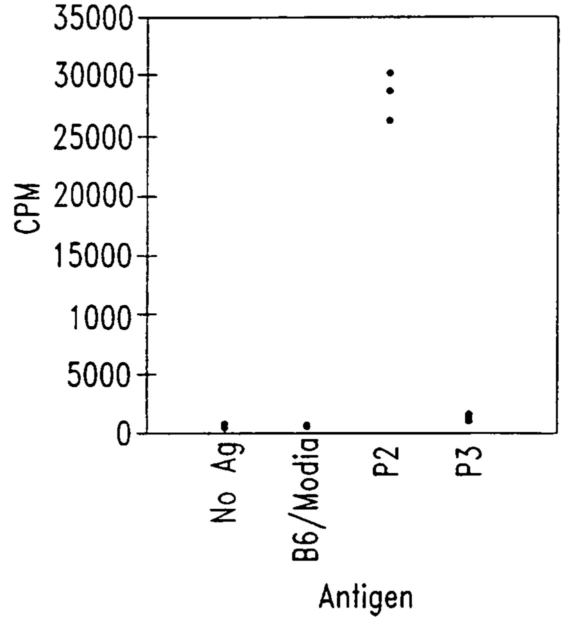


*Fig. 5B*

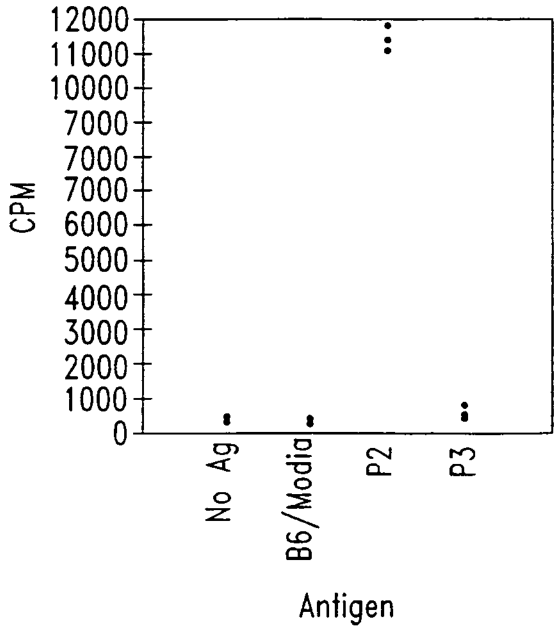


*Fig. 5C*

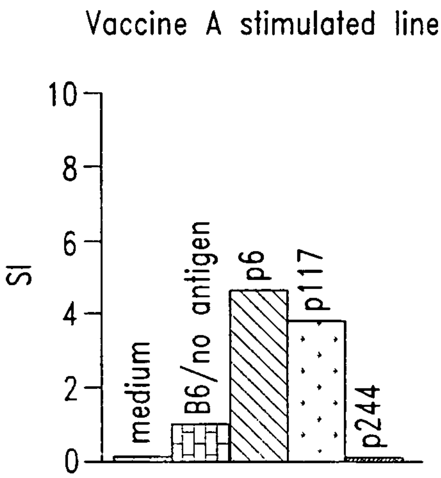
Vaccine A stimulated line
SI
10
8
6
4
2
0
medium
B6/no antigen
p6
p117
p244

*Fig. 6A*

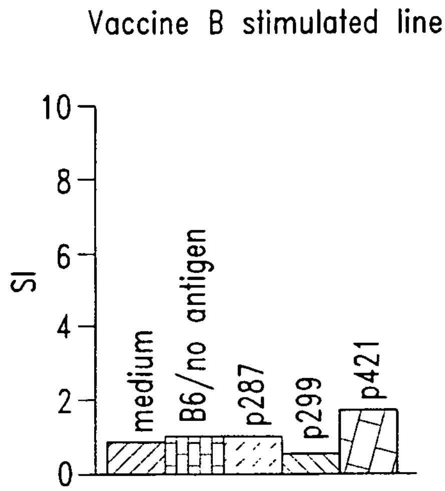
Vaccine B stimulated line
SI
10
8
6
4
2
0
medium
B6/no antigen
p287
p299
p421

*Fig. 6B*

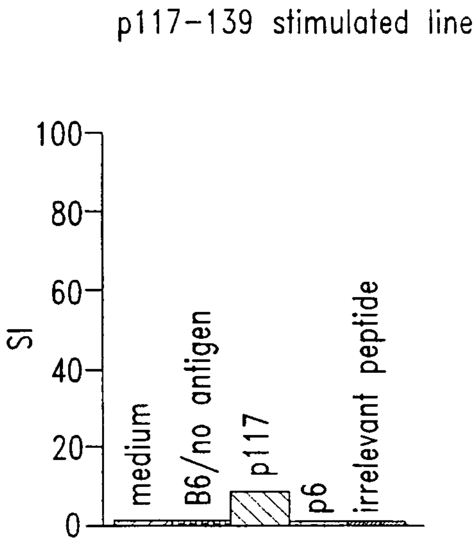


*Fig. 7A*

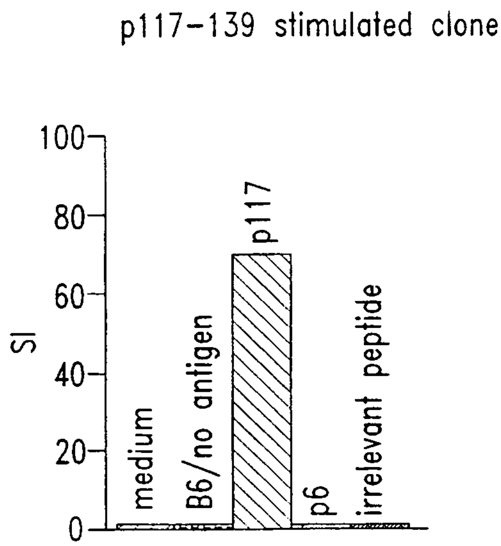


*Fig. 7B*

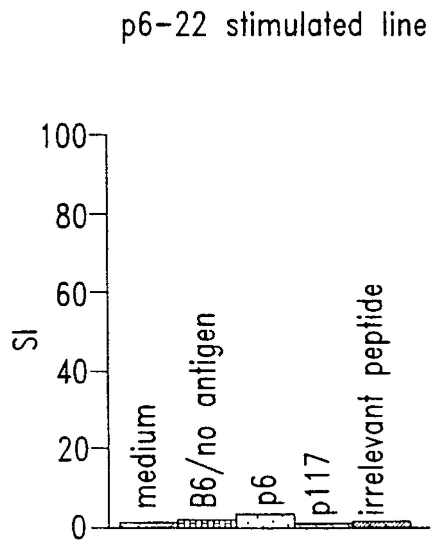


*Fig. 7C*

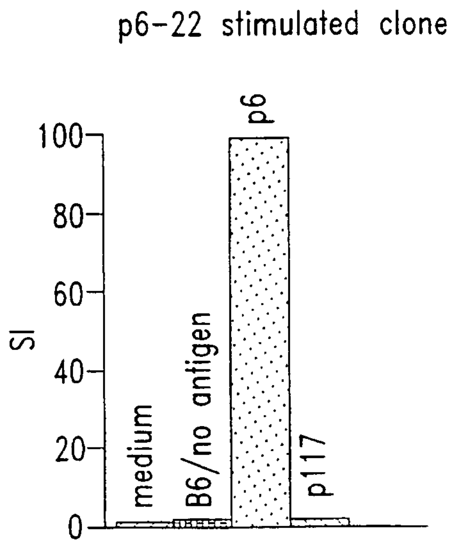


*Fig. 7D*

```
    5    10   15   20   25   30   35   40   45   50   55   60   65   70   75
MGSDVRDLNALLPAVPSLGGGGGCALPVSGAAQWAPVLDFAPPGASAYGSLGGPAPPPAPPPPPPPPPHSFIKQE
.....AAAAAAAAAAAAAAAAA........AAAAAA.........AAAAAAAAAAAA..................
..............................RRRR.........................................
...........................................................................
...........................................................................

   80   85   90   95  100  105  110  115  120  125  130  135  140  145  150
PSWGGAEPHEEQCLSAFTVHFSGQFTGTAGACRYGPFGPPPPSQASSGQARMFPNAPYLPSCLESQPAIRNQGYS
...............AAA......AAAA........................AAA......AAAAAA........
..........................RRRR........................RRRRR................
.............................................DDDDDDDD......................
...........................................................................

  155  160  165  170  175  180  185  190  195  200  205  210  215  220  225
TVTFDGTPSYGHTPSHHAAQFPNHSFKHEDPMGQQGSLGEQQYSVPPPVYGCHTPTDSCTGSQALLLRTPYSSDN
................AAAAA.............................AAAAAA.................AA
................RRRR.......................................................
.........................................................DDDDDDDDDDDDDD....
...........................................................................

  230  235  240  245  250  255  260  265  270  275  280  285  290  295  300
LYQMTSQLECMTWNQMNLGATLKGVAAGSSSSVKWTEGQSNHSTGYESDNHTTPILCGAQYRIHTHGVFRGIQDV
AAAAAAAA............AAA.AAA.....................................AAAAAAAAAAA
..................RRRRRRRRR....RRRR...............................RRRR.....
DDDDDD...............DDDDDDDDDD............................................
............................................................ddddd..........

  305  310  315  320  325  330  335  340  345  350  355  360  365  370  375
RRVPGVAPTLVRSASETSEKRPFMCAYPGCNKRYFKLSHLQMHSRKHTGEKPYQCDFKDCERRFSRSDQLKRHQR
AAAAA..AAAAAAAAAAA..........................................AAAA.AAAAAAAAA.
....RRRRR....................RRRR..........................................
.......DDDDDD..............................................................
...........................................................................

  380  385  390  395  400  405  410  415  420  425  430  435  440  445  450
RHTGVKPFQCKTCQRKFSRSDHLKTHTRTHTGKTSEKPFSCRWPSCQKKFARSDELVRHHNMHQRNMTKLQLAL
.............AAAA.AAAA..AA.....AAAA.........AAA....AAAAAAAA...AAA.........
...............................................RRRR..RRRR.................
..........................................................................
.................dddddddddddd.............................................
```

*Fig. 8A*

5 10 15 20 25 30 35 40 45 50 55 60 65 70 75
MGSDVRDLNALLPAVSSLGGGGGCGLPVSGAAQWAPVLDFAPPGASAYGSLGGPAPPPAPPPPPPPPPHSFIKQE
.....AAAAAAAAAAAAAAAAA.......AAAAAA.........AAAAAAAAAAAA...................
.............................RRRR..........................................
...........................................................................
...........................................................................

80 85 90 95 100 105 110 115 120 125 130 135 140 145 150
PSWGGAEPHEEQCLSAFTLHFSGQFTGTAGACRYGPFGPPPPSQASSGQARMFPNAPYLPSCLESQPTIRNQGYS
........................AAAA.....................AAA......AAAAAA...........
..........................RRRR....................RRRRR....................
.........................................DDDDDDDD..........................
...........................................................................

155 160 165 170 175 180 185 190 195 200 205 210 215 220 225
TVTFDGAPSYGHTPSHHAAQFPNHSFKHEDPMGQQGSLGEQQYSVPPPVYGCHTPTDSCTGSQALLLRTPYSSDN
................AAAAA..............................AAAAAA................AA
................RRRR.......................................................
..........................................................DDDDDDDDDDDDDD...
...........................................................................

230 235 240 245 250 255 260 265 270 275 280 285 290 295 300
LYQMTSQLECMTWNQMNLGATLKGMAAGSSSSVKWTEGQSNHGIGYESDNHTAPILCGAQYRIHTHGVFRGIQDV
AAAAAAAA............AAA.AAA.....................................AAAAAAAAAAA
..................RRRRRRRRRR.....RRRR.............................RRRR.....
DDDDDD...............DDDDDDDDDDD...........................................
.............................................................ddddd.........

305 310 315 320 325 330 335 340 345 350 355 360 365 370 375
RRVSGVAPTLVRSASETSEKRPFMCAYPGCNKRYFKLSHLQMHSRKHTGEKPYQCDFKDCERRFSRSDQLKRHQR
AAAAA..AAAAAAAAAAA..........................................AAAA.AAAAAAAAA.
....RRRRR.......................RRRR.......................................
..DDDDDDDDDDD..............................................................
...........................................................................

380 385 390 395 400 405 410 415 420 425 430 435 440 445 450
RHTGVKPFQCKTCQRKFSRSDHLKTHTRTHTGKTSEKPFSCRWHSCQKKFARSDELVRHHNMHQRNMTKLHVAL
.............AAAA.AAAA..AA.....AAAA...........AA....AAAAAAAA...AAAA.......
................................................RRRR..RRRR................
..........................................................................
..................dddddddddddd............................................

*Fig. 8B*

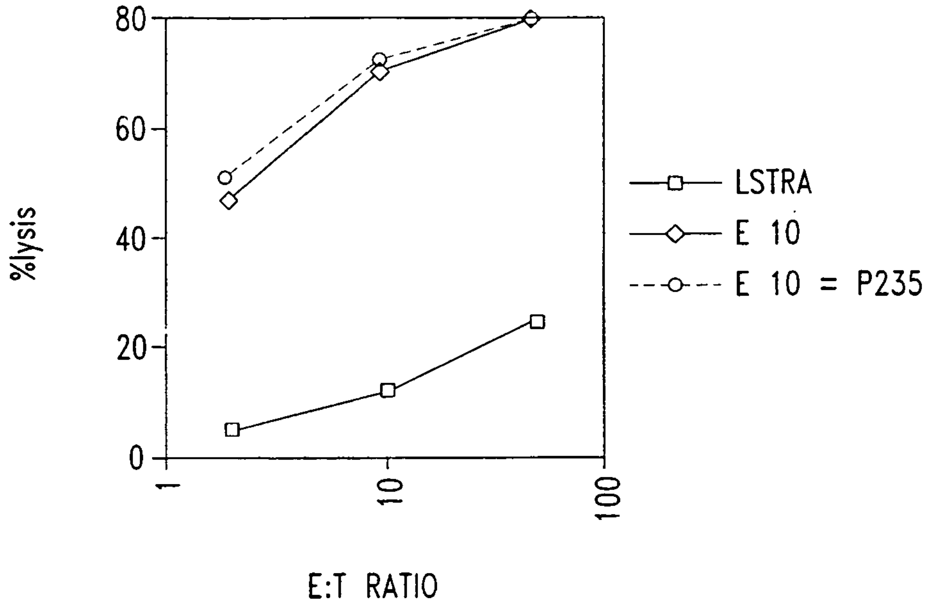
80
60
40
20
0
%lysis
1
10
100
E:T RATIO
LSTRA
E 10
E 10 = P235

*Fig. 9A*

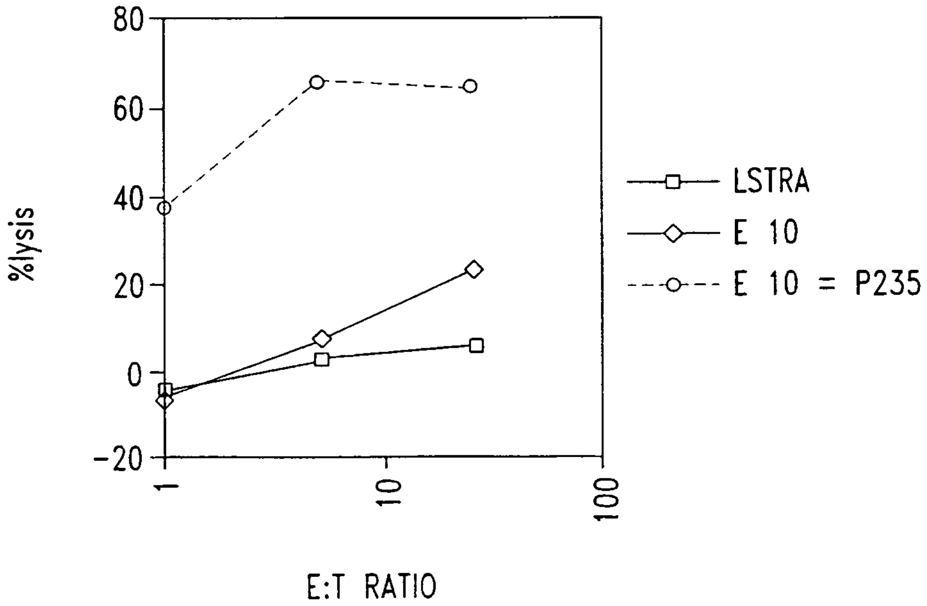
80
60
40
20
0
-20
%lysis
1
10
100
E:T RATIO
LSTRA
E 10
E 10 = P235

*Fig. 9B*

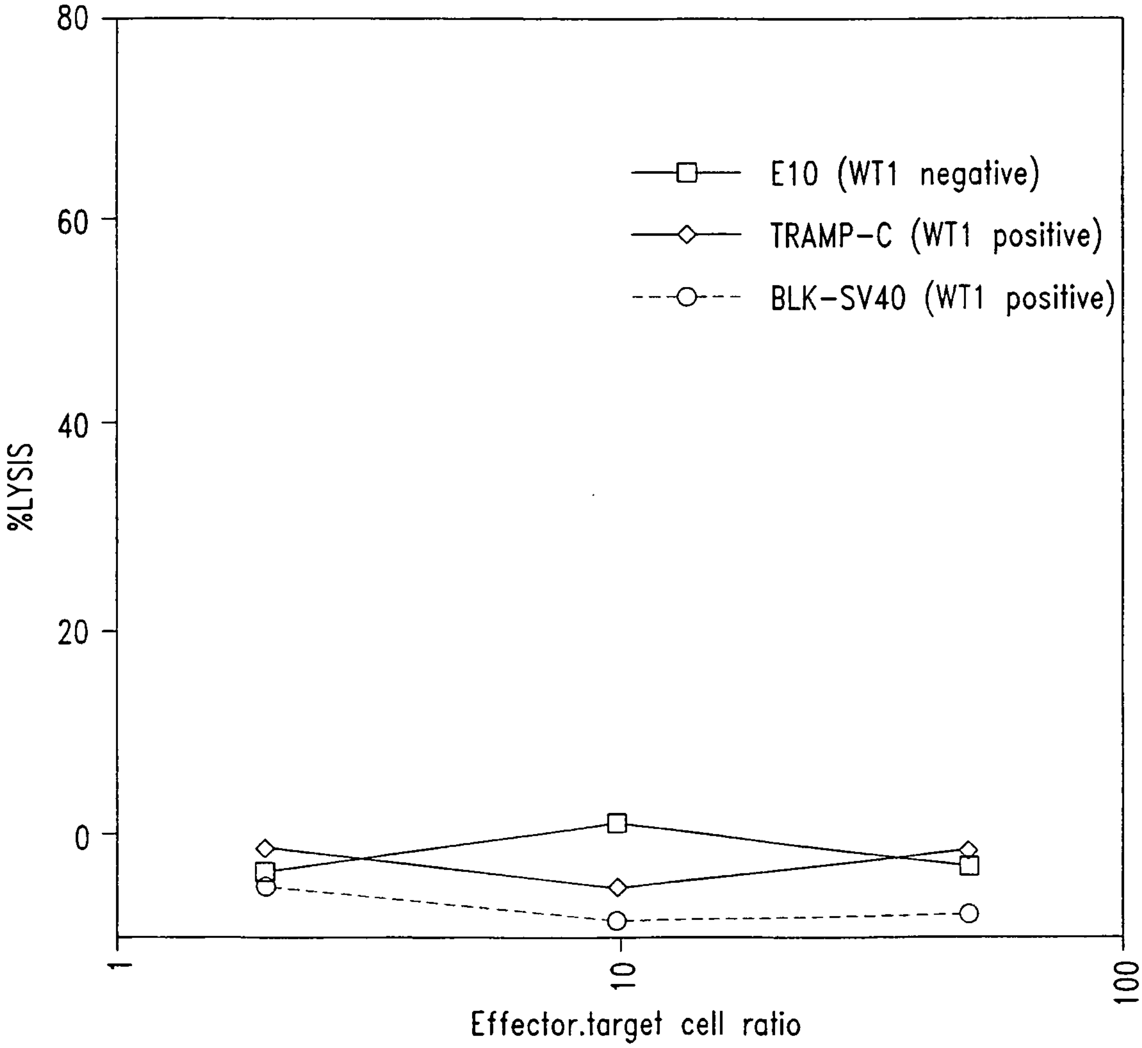
80
60
40
20
0
%LYSIS
E10 (WT1 negative)
TRAMP-C (WT1 positive)
BLK-SV40 (WT1 positive)
1
10
100
Effector.target cell ratio

*Fig. 10A*

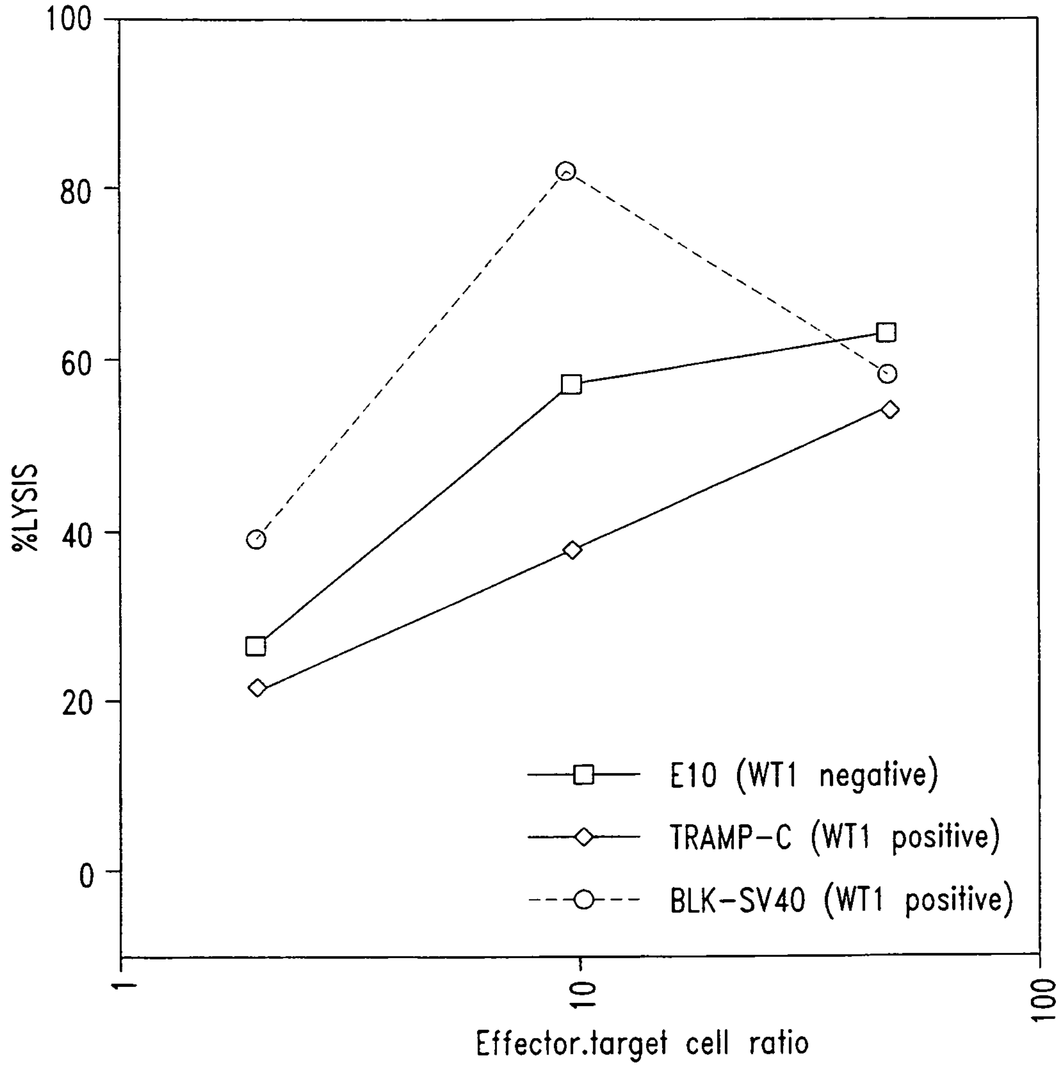
100
80
60
40
20
0
%LYSIS
E10 (WT1 negative)
TRAMP-C (WT1 positive)
BLK-SV40 (WT1 positive)
1
10
100
Effector.target cell ratio

*Fig. 10B*

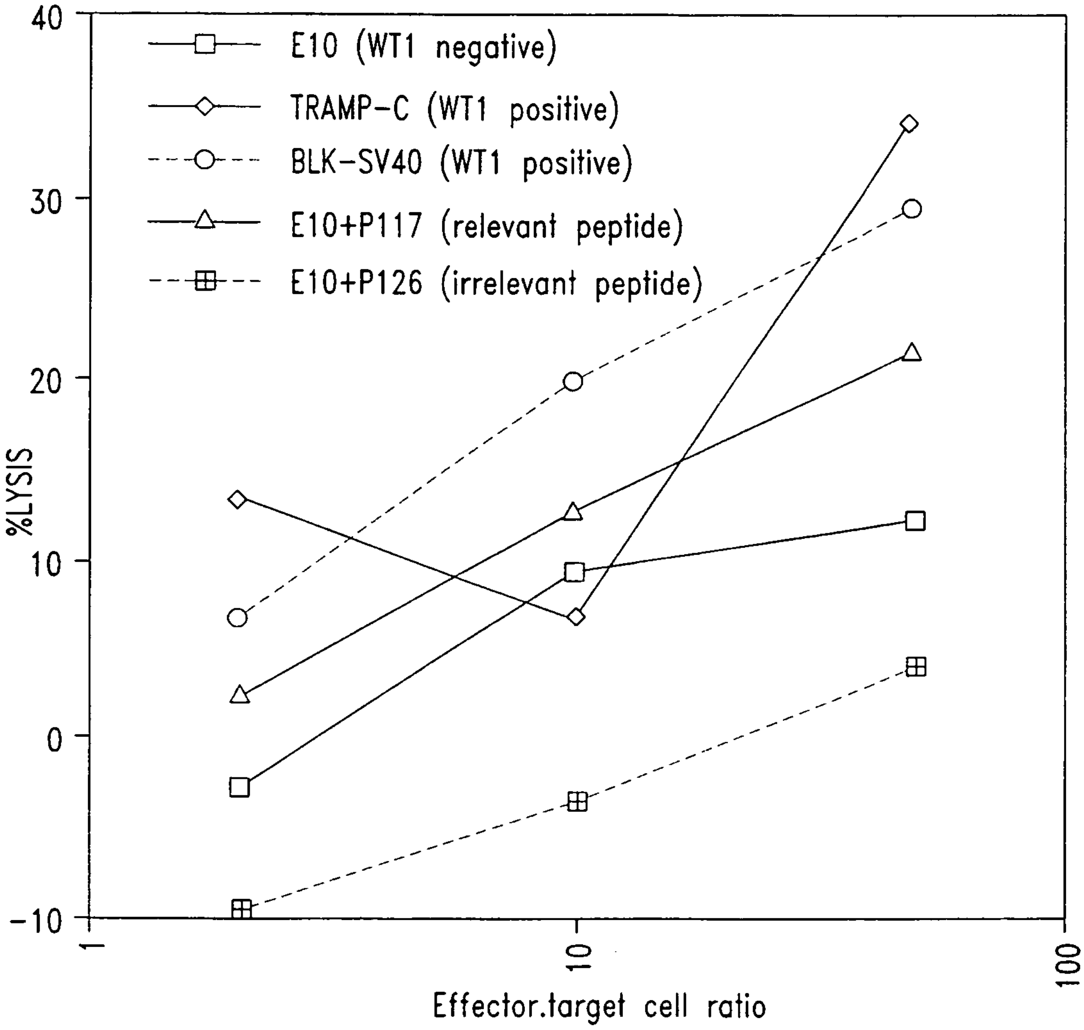
E10 (WT1 negative)
TRAMP-C (WT1 positive)
BLK-SV40 (WT1 positive)
E10+P117 (relevant peptide)
E10+P126 (irrelevant peptide)
40
30
20
10
0
-10
%LYSIS
1
10
100
Effector.target cell ratio

*Fig. 10C*

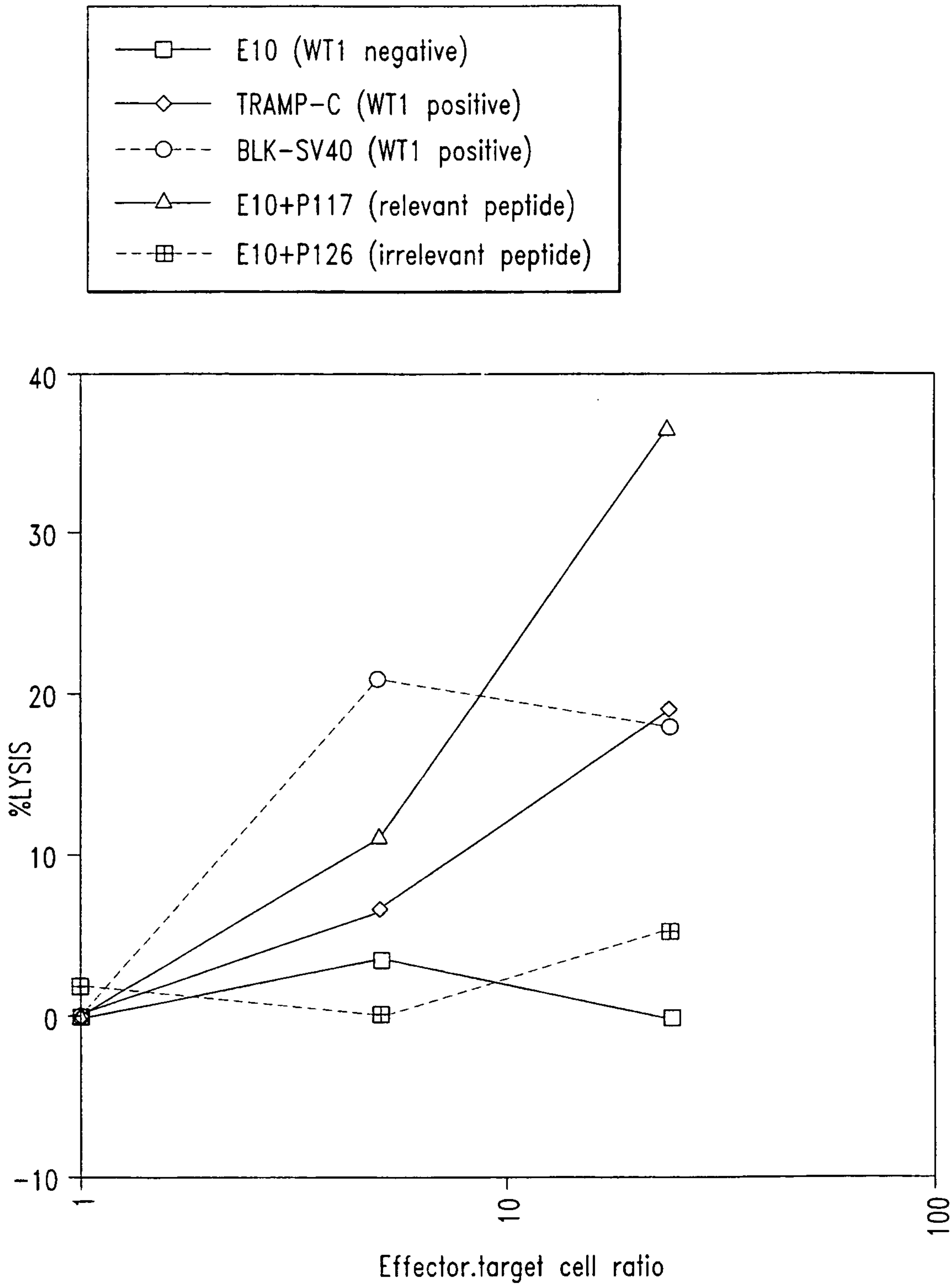
E10 (WT1 negative)
TRAMP-C (WT1 positive)
BLK-SV40 (WT1 positive)
E10+P117 (relevant peptide)
E10+P126 (irrelevant peptide)
40
30
20
10
0
-10
%LYSIS
1
10
100
Effector.target cell ratio

*Fig. 10D*

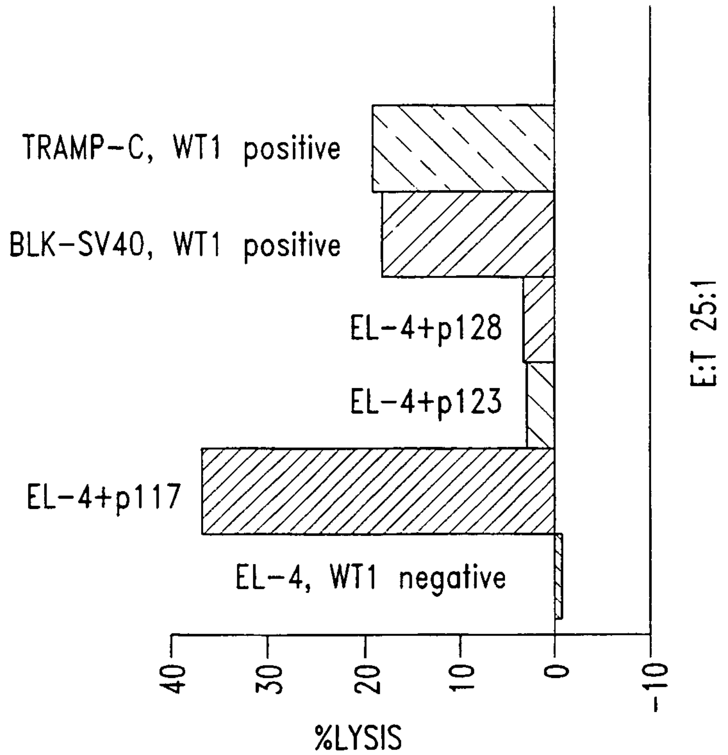


*Fig. 11B*

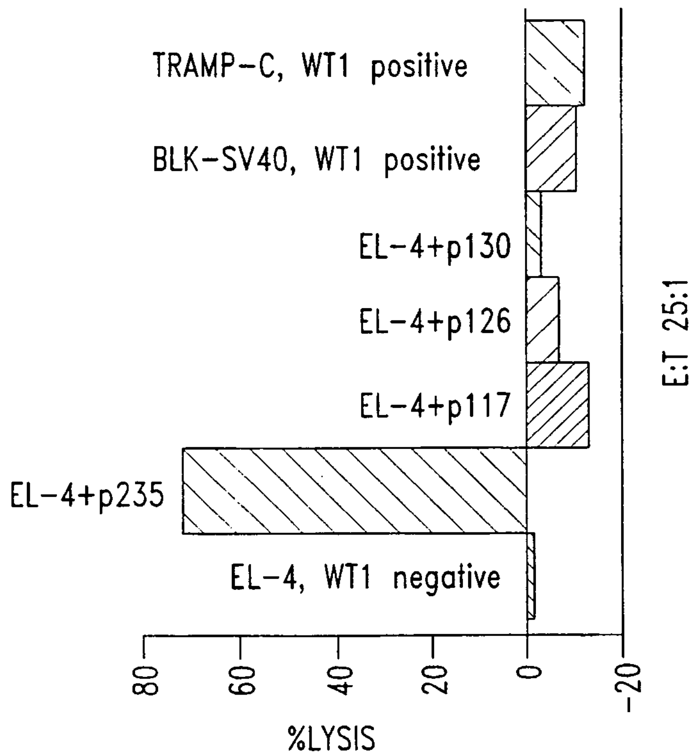


*Fig. 11A*

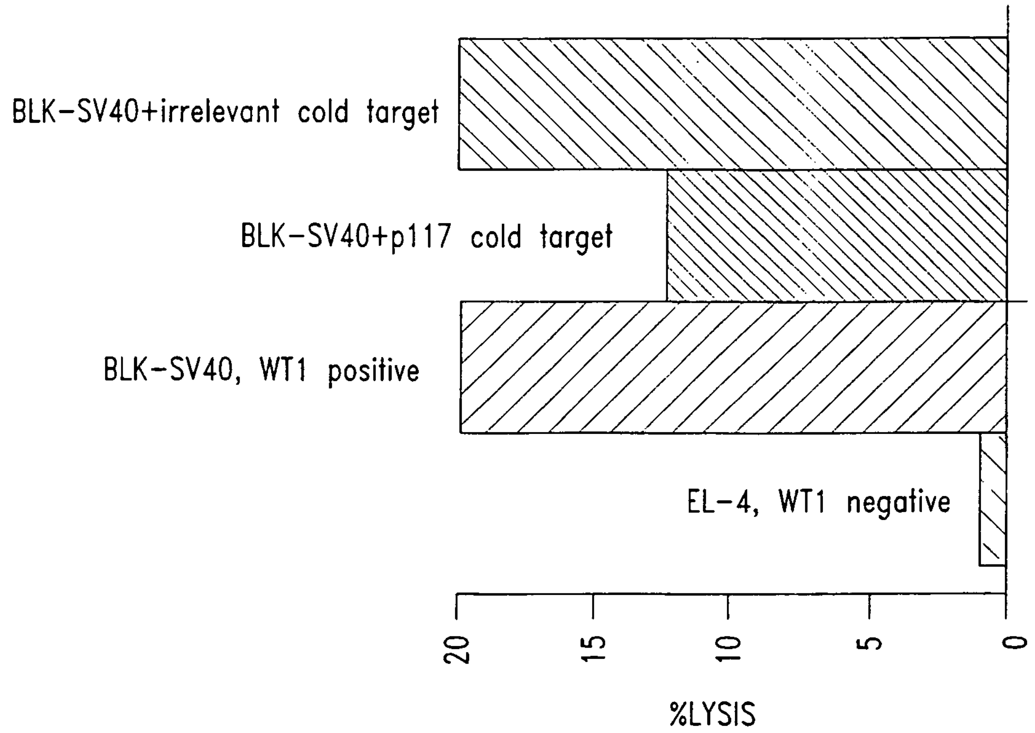


*Fig. 12B*

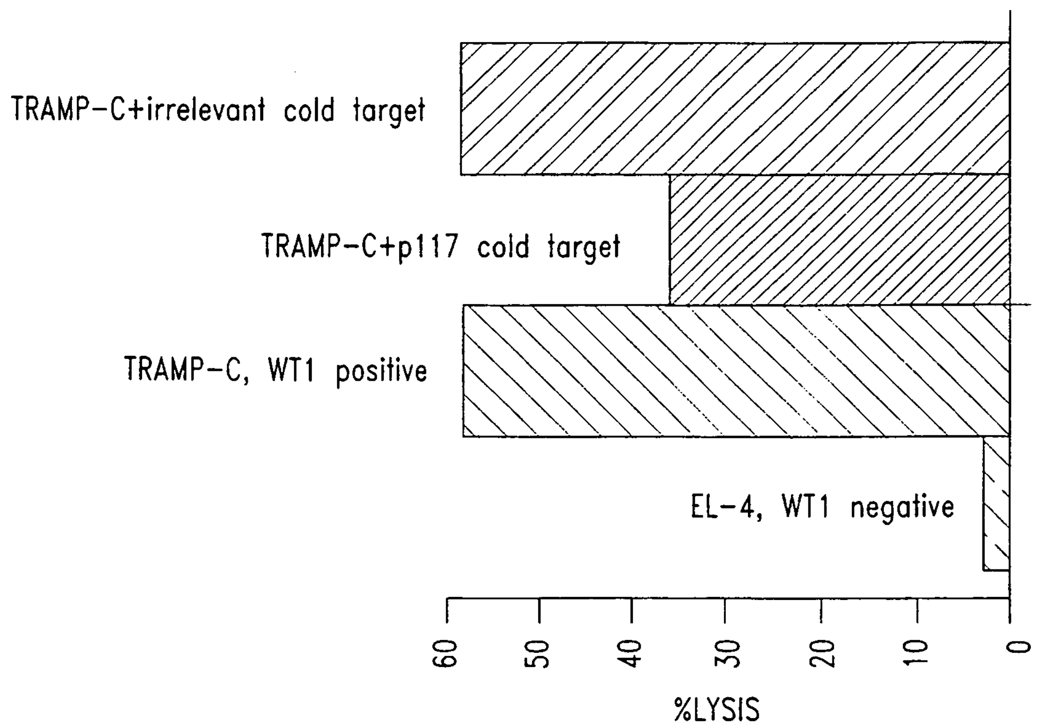


*Fig. 12A*

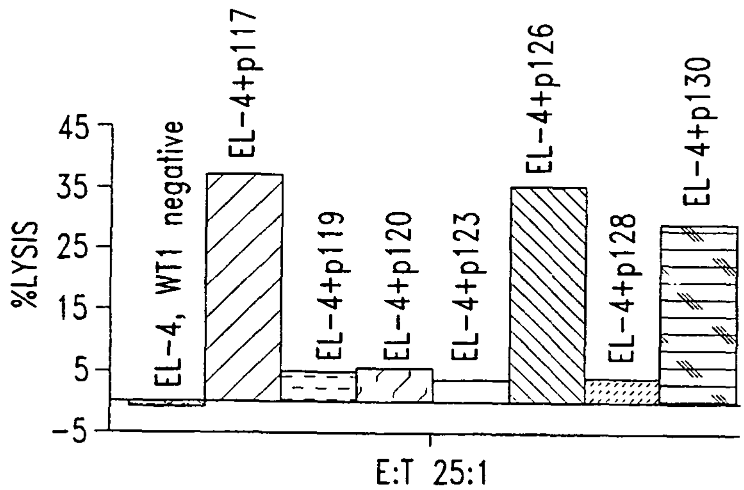


*Fig. 13A*

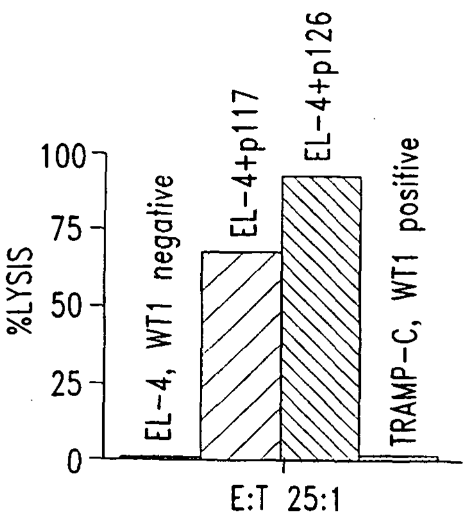


*Fig. 13B*

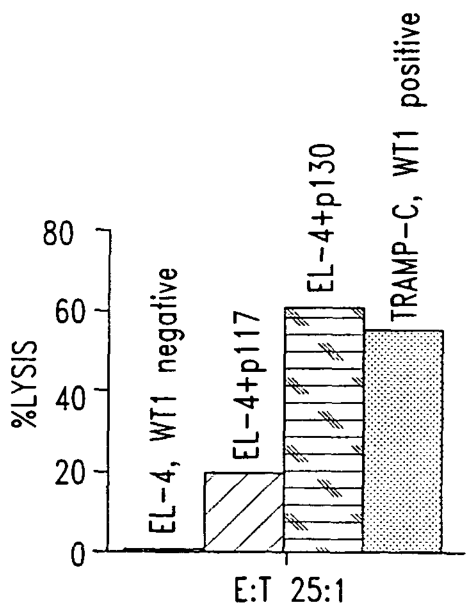


*Fig. 13C*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5350840 A, Call
- US 4671958 A, Rodwell
- US 4489710 A, Spitler
- US 4625014 A, Senter
- US 4638045 A, Kohn
- US 4569789 A, Blattler
- US 4507234 A, Kato
- US 4699784 A, Shih
- US 4429008 A
- US 4873088 A
- US 4735792 A
- US 4673562 A, Davison
- US 5240856 A
- US 5215926 A
- WO 8906280 A
- WO 9116116 A
- WO 9207243 A
- US 4897268 A
- US 5075109 A
- US 4376110 A, David
- US 4452901 A
- US 3817827 A
- US 3850752 A
- US 3901654 A
- US 3935074 A
- US 3984533 A
- US 3996345 A
- US 4034074 A
- US 4098876 A
- US 5359681 A
- WO 210121465 C1


### Non-patent literature cited in the description


- **PARKER et al.** *J. Immunol.,* 1994, vol. 152, 163
- **HARLOW ; LANE.** Antibodies: A Laboratory Manual. Cold Spring Harbor Laboratory, 1988
- **ROTHBARD ; TAYLOR.** *EMBO J.,* 1988, vol. 7, 93-100
- **DEAVIN et al.** *Mol. Immunol.,* 1996, vol. 33, 145-155
- **PARKER et al.** *J Immunol.,* 1994, vol. 152, 163
- **MERRIFIELD.** *J. Am. Chem. Soc.,* 1963, vol. 85, 2149-2146
- **ADELMAN et al.** *DNA,* 1983, vol. 2, 183
- **KOHLER ; MILSTEIN.** *Eur. J. Immunol.,* 1976, vol. 6, 511-519
- **CHEN et al.** *Cancer Res.,* 1994, vol. 54, 1065-1070
- **COLIGAN et al.** Current Protocols in Immunology. Wiley Interscience (Greene, 1998, vol. 1
- **ULMER et al.** *Science,* 1993, vol. 259, 1745-1749
- **COHEN.** *Science,* 1993, vol. 259, 1691-1692
- **REEVES et al.** *Cancer Res.,* 1996, vol. 56, 5672-5677
- **TUTING et al.** *J. Immunol.,* 1998, vol. 160, 1139-1147
- **NAIR et al.** *Nature Biotechnol.,* 1998, vol. 16, 364-369
- **WIDE et al.** Radioimmunoassay Methods. E. and S. Livingstone, 1970
- **BROWN et al.** *J. Biol. Chem.,* 1980, vol. 255, 4980-4983
- **RAINES ; ROSS.** *J. Biol. Chem.,* 1982, vol. 257, 5154-5160
- **BROOKS et al.** *Clin. Exp. Immunol.,* 1980, vol. 39, 477
- **BOWEN-POPE et al.** *Proc. Natl. Acad. Sci. USA,* 1984, vol. 81, 2396-2400
- **ROTHBARD ; TAYLOR.** *EMBO J,* 1988, vol. 7, 93-100
- **CHEN et al.** *Cancer Res.,* 1994, vol. 54, 1 065-1 070
- **LJUNGGREN et al.** *Nature,* 1990, vol. 346, 476-480
- **FRAIZER et al.** *Blood,* 1995, vol. 86, 4704-4706
- **KWOK ; HIGUCHI.** *Nature,* 1989, vol. 339, 237-238
- **INOUE et al.** *Blood,* 1996, vol. 88, 2267-2278
- **LOZZIO ; LOZZIO.** *Blood,* 1975, vol. 45, 321-334
- **FOSTER et al.** *Cancer Res.,* 1997, vol. 57, 3325-3330
- *Nature,* 1978, vol. 276, 510-511
- **GILLIS.** *Nature,* 1977, vol. 268, 154-156
- **GLYNN ; FEFER.** *Cancer Res.,* 1968, vol. 28, 434-439

- **AAROSTON ; TODARO.** *J. Cell. Physiol.,* 1968, vol. 72, 141-148
- **HORIBATA ; HARRIS.** *Exp. Cell. Res.,* 1970, vol. 60, 61
- **OLD et al.** *Ann. NY Acad Sci.,* 1962, vol. 101, 80-106
- *Proc. Natl. Acad. Sci. USA,* 1970, vol. 66, 344
- **MELLING et al.** *J. Immunol.,* 1976, vol. 117, 1267-1274
- **SLAVIN ; STROBER.** *Nature,* 1977, vol. 272, 624-626
- **GLYNN et al.** *Cancer Res.,* 1968, vol. 28, 434-439

SEQUENCE LISTING

<110> Gaiger, Alexander
Cheever, Martin A.

<120> COMPOSITIONS AND METHODS FOR WT1
SPECIFIC IMMUNOTHERAPY

<130> 210121.465C1

<140> US
<141> 1999-03-25

<160> 326

<170> FastSEQ for Windows Version 3.0

<210> 1
<211> 17
<212> PRT
<213> Homo sapien

<400> 1

```
Arg Asp Leu Asn Ala Leu Leu Pro Ala Val Pro Ser Leu Gly Gly Gly
 1               5                  10                  15
Gly
```

<210> 2
<211> 23
<212> PRT
<213> Homo sapien

<400> 2

```
Pro Ser Gln Ala Ser Ser Gly Gln Ala Arg Met Phe Pro Asn Ala Pro
 1               5                  10                  15
Tyr Leu Pro Ser Cys Leu Glu
            20
```

<210> 3
<211> 23
<212> PRT
<213> Mus musculus

<400> 3

```
Pro Ser Gln Ala Ser Ser Gly Gln Ala Arg Met Phe Pro Asn Ala Pro
 1              5                  10                  15
Tyr Leu Pro Ser Cys Leu Glu
            20
```

<210> 4
<211> 19
<212> PRT
<213> Homo sapien

<400> 4

```
Gly Ala Thr Leu Lys Gly Val Ala Ala Gly Ser Ser Ser Ser Val Lys
 1              5                  10                  15
Trp Thr Glu
```

<210> 5
<211> 22
<212> DNA
<213> Homo sapien

<400> 5
gagagtcaga cttgaaagca gt 22

<210> 6
<211> 20
<212> DNA
<213> Homo sapien

<400> 6
ctgagcctca gcaaatgggc 20

<210> 7
<211> 27
<212> DNA
<213> Homo sapien

<400> 7
gagcatgcat gggctccgac gtgcggg 27

<210> 8
<211> 25
<212> DNA
<213> Homo sapien

<400> 8
ggggtaccca ctgaacggtc cccga 25

<210> 9
<211> 18
<212> DNA
<213> Mus musculus

<400> 9
tccgagccgc acctcatg 18

<210> 10
<211> 18
<212> DNA
<213> Mus musculus

<400> 10
gcctgggatg ctggactg 18

<210> 11
<211> 27
<212> DNA
<213> Mus musculus

<400> 11
gagcatgcga tgggttccga cgtgcgg 27

<210> 12
<211> 29
<212> DNA
<213> Mus musculus

<400> 12
ggggtacctc aaagcgccac gtggagttt 29

<210> 13
<211> 17
<212> PRT
<213> Mus musculus

<400> 13

```
Arg Asp Leu Asn Ala Leu Leu Pro Ala Val Ser Ser Leu Gly Gly Gly
 1               5                  10                  15
Gly
```

<210> 14
<211> 19
<212> PRT
<213> Mus musculus

<400> 14

```
Gly Ala Thr Leu Lys Gly Met Ala Ala Gly Ser Ser Ser Ser Val Lys
 1               5                  10                  15
Trp Thr Glu
```

<210> 15
<211> 15
<212> PRT
<213> Homo sapien

<400> 15

```
Arg Ile His Thr His Gly Val Phe Arg Gly Ile Gln Asp Val Arg
 1               5                  10                  15
```

<210> 16
<211> 15
<212> PRT
<213> Mus musculus

<400> 16

```
Arg Ile His Thr His Gly Val Phe Arg Gly Ile Gln Asp Val Arg
1               5                   10                  15
```

<210> 17
<211> 14
<212> PRT
<213> Mus musculus

<400> 17

```
Val Arg Arg Val Ser Gly Val Ala Pro Thr Leu Val Arg Ser
1               5                   10
```

<210> 18
<211> 14
<212> PRT
<213> Homo sapien

<400> 18

```
Val Arg Arg Val Pro Gly Val Ala Pro Thr Leu Val Arg Ser
1               5                   10
```

<210> 19
<211> 15
<212> PRT
<213> Homo sapien

<400> 19

```
Cys Gln Lys Lys Phe Ala Arg Ser Asp Glu Leu Val Arg His His
1               5                   10                  15
```

<210> 20
<211> 15
<212> PRT
<213> Mus musculus

<400> 20

```
Cys Gln Lys Lys Phe Ala Arg Ser Asp Glu Leu Val Arg His His
1               5                   10                  15
```

<210> 21
<211> 21
<212> DNA
<213> Mus musculus

<400> 21
cccaggctgc aataagagat a 21

<210> 22
<211> 21
<212> DNA
<213> Mus musculus

<400> 22
atgttgtgat ggcggaccaa t 21

<210> 23
<211> 20
<212> DNA
<213> Homo sapien

<400> 23
gtggggcgcc ccaggcacca 20

<210> 24
<211> 24
<212> DNA
<213> Homo sapien

<400> 24
gtccttaatg ctacgcacga tttc 24

<210> 25
<211> 21
<212> DNA
<213> Homo sapien

<400> 25
ggcatctgag accagtgaga a 21

<210> 26
<211> 21
<212> DNA
<213> Homo sapien

<400> 26
gctgtcccac ttacagatgc a 21

<210> 27
<211> 21
<212> DNA
<213> Homo sapien

<400> 27
tcaaagcgcc agctggagtt t 21

<210> 28
<211> 9
<212> PRT
<213> Homo sapien

<400> 28

Ala Ala Gly Ser Ser Ser Ser Val Lys
1 5

<210> 29
<211> 9
<212> PRT
<213> Homo sapien

<400> 29

Ala Ala Gln Phe Pro Asn His Ser Phe
1 5

<210> 30
<211> 9
<212> PRT
<213> Homo sapien

<400> 30

Ala Glu Pro His Glu Glu Gln Cys Leu
1 5

<210> 31
<211> 9
<212> PRT
<213> Homo sapien

<400> 31

Ala Gly Ala Cys Arg Tyr Gly Pro Phe
1 5

<210> 32
<211> 9
<212> PRT
<213> Homo sapien

<400> 32

Ala Gly Ser Ser Ser Ser Val Lys Trp
1 5

<210> 33
<211> 9
<212> PRT
<213> Homo sapien

<400> 33

Ala Ile Arg Asn Gln Gly Tyr Ser Thr
1 5

<210> 34
<211> 9
<212> PRT
<213> Homo sapien

<400> 34

Ala Leu Leu Pro Ala Val Pro Ser Leu
1 5

<210> 35
<211> 9
<212> PRT
<213> Homo sapien

<400> 35

Ala Leu Leu Pro Ala Val Ser Ser Leu
1 5

<210> 36
<211> 9
<212> PRT
<213> Homo sapien

<400> 36

Ala Gln Phe Pro Asn His Ser Phe Lys
1 5

<210> 37
<211> 9
<212> PRT
<213> Homo sapien

<400> 37

Ala Gln Trp Ala Pro Val Leu Asp Phe
1 5

<210> 38
<211> 9
<212> PRT
<213> Homo sapien

<400> 38

Ala Arg Met Phe Pro Asn Ala Pro Tyr
1 5

<210> 39
<211> 9
<212> PRT
<213> Homo sapien

<400> 39

Ala Arg Ser Asp Glu Leu Val Arg His
1 5

<210> 40
<211> 9
<212> PRT
<213> Homo sapien

<400> 40

Ala Ser Ser Gly Gln Ala Arg Met Phe
1 5

<210> 41
<211> 9
<212> PRT
<213> Homo sapien

<400> 41

Ala Tyr Gly Ser Leu Gly Gly Pro Ala
1 5

<210> 42
<211> 9
<212> PRT
<213> Homo sapien

<400> 42

Ala Tyr Pro Gly Cys Asn Lys Arg Tyr
1 5

<210> 43
<211> 9
<212> PRT
<213> Homo sapien

<400> 43

Cys Ala Leu Pro Val Ser Gly Ala Ala
1 5

<210> 44
<211> 9
<212> PRT
<213> Homo sapien

<400> 44

Cys Ala Tyr Pro Gly Cys Asn Lys Arg
1 5

<210> 45
<211> 9
<212> PRT
<213> Homo sapien

<400> 45

Cys His Thr Pro Thr Asp Ser Cys Thr
1 5

<210> 46
<211> 9
<212> PRT
<213> Homo sapien

<400> 46

Cys Lys Thr Cys Gln Arg Lys Phe Ser
1 5

<210> 47
<211> 9
<212> PRT
<213> Homo sapien

<400> 47

Cys Leu Glu Ser Gln Pro Ala Ile Arg

1 5

<210> 48
<211> 9
<212> PRT
<213> Homo sapien

<400> 48

Cys Leu Ser Ala Phe Thr Val His Phe
1 5

<210> 49
<211> 9
<212> PRT
<213> Homo sapien

<400> 49

Cys Met Thr Trp Asn Gln Met Asn Leu
1 5

<210> 50
<211> 9
<212> PRT
<213> Homo sapien

<400> 50

Cys Arg Trp Pro Ser Cys Gln Lys Lys
1 5

<210> 51
<211> 9
<212> PRT
<213> Homo sapien

<400> 51

Cys Arg Tyr Gly Pro Phe Gly Pro Pro
1 5

<210> 52
<211> 9
<212> PRT
<213> Homo sapien

<400> 52

Cys Thr Gly Ser Gln Ala Leu Leu Leu
1 5

<210> 53
<211> 9
<212> PRT
<213> Homo sapien

<400> 53

Asp Glu Leu Val Arg His His Asn Met
1 5

<210> 54
<211> 9
<212> PRT

<213> Homo sapien

<400> 54

Asp Phe Ala Pro Pro Gly Ala Ser Ala
1 5

<210> 55
<211> 9
<212> PRT
<213> Homo sapien

<400> 55

Asp Phe Lys Asp Cys Glu Arg Arg Phe
1 5

<210> 56
<211> 9
<212> PRT
<213> Homo sapien

<400> 56

Asp Gly Thr Pro Ser Tyr Gly His Thr
1 5

<210> 57
<211> 9
<212> PRT
<213> Homo sapien

<400> 57

Asp His Leu Lys Thr His Thr Arg Thr
1 5

<210> 58
<211> 9
<212> PRT
<213> Homo sapien

<400> 58

Asp Leu Asn Ala Leu Leu Pro Ala Val
1 5

<210> 59
<211> 9
<212> PRT
<213> Homo sapien

<400> 59

Asp Pro Met Gly Gln Gln Gly Ser Leu
1 5

<210> 60
<211> 9
<212> PRT
<213> Homo sapien

<400> 60

Asp Gln Leu Lys Arg His Gln Arg Arg
1 5

<210> 61
<211> 9
<212> PRT
<213> Homo sapien

<400> 61

Asp Ser Cys Thr Gly Ser Gln Ala Leu
1 5

<210> 62
<211> 9
<212> PRT
<213> Homo sapien

<400> 62

Asp Val Arg Asp Leu Asn Ala Leu Leu
1 5

<210> 63
<211> 9
<212> PRT
<213> Homo sapien

<400> 63

Asp Val Arg Arg Val Pro Gly Val Ala
1 5

<210> 64
<211> 9
<212> PRT
<213> Homo sapien

<400> 64

Glu Asp Pro Met Gly Gln Gln Gly Ser
1 5

<210> 65
<211> 9
<212> PRT
<213> Homo sapien

<400> 65

Glu Glu Gln Cys Leu Ser Ala Phe Thr
1 5

<210> 66
<211> 9
<212> PRT
<213> Homo sapien

<400> 66

Glu Lys Pro Tyr Gln Cys Asp Phe Lys
1 5

<210> 67
<211> 9
<212> PRT
<213> Homo sapien

<400> 67

Glu Lys Arg Pro Phe Met Cys Ala Tyr
1 5

<210> 68
<211> 9
<212> PRT
<213> Homo sapien

<400> 68

Glu Pro His Glu Glu Gln Cys Leu Ser
1 5

<210> 69
<211> 9
<212> PRT
<213> Homo sapien

<400> 69

Glu Gln Cys Leu Ser Ala Phe Thr Val
1 5

<210> 70
<211> 9
<212> PRT
<213> Homo sapien

<400> 70

Glu Ser Asp Asn His Thr Ala Pro Ile
1 5

<210> 71
<211> 9
<212> PRT
<213> Homo sapien

<400> 71

Glu Ser Asp Asn His Thr Thr Pro Ile
1 5

<210> 72
<211> 9
<212> PRT
<213> Homo sapien

<400> 72

Glu Ser Gln Pro Ala Ile Arg Asn Gln
1 5

<210> 73
<211> 9
<212> PRT
<213> Homo sapien

<400> 73

Glu Thr Ser Glu Lys Arg Pro Phe Met
1 5

<210> 74
<211> 9
<212> PRT
<213> Homo sapien

<400> 74

Phe Ala Pro Pro Gly Ala Ser Ala Tyr
1 5

<210> 75
<211> 9
<212> PRT

<213> Homo sapien

<400> 75

Phe Ala Arg Ser Asp Glu Leu Val Arg
1 5

<210> 76
<211> 9
<212> PRT
<213> Homo sapien

<400> 76

Phe Gly Pro Pro Pro Pro Ser Gln Ala
1 5

<210> 77
<211> 9
<212> PRT
<213> Homo sapien

<400> 77

Phe Lys Asp Cys Glu Arg Arg Phe Ser
1 5

<210> 78
<211> 9
<212> PRT
<213> Homo sapien

<400> 78

Phe Lys Leu Ser His Leu Gln Met His
1 5

<210> 79
<211> 9
<212> PRT
<213> Homo sapien

<400> 79

Phe Pro Asn Ala Pro Tyr Leu Pro Ser
1 5

<210> 80
<211> 9
<212> PRT
<213> Homo sapien

<400> 80

Phe Gln Cys Lys Thr Cys Gln Arg Lys
1 5

<210> 81
<211> 9
<212> PRT
<213> Homo sapien

<400> 81

Phe Arg Gly Ile Gln Asp Val Arg Arg
1 5

<210> 82
<211> 9
<212> PRT
<213> Homo sapien

<400> 82

Phe Ser Gly Gln Phe Thr Gly Thr Ala
1 5

<210> 83
<211> 9
<212> PRT
<213> Homo sapien

<400> 83

Phe Ser Arg Ser Asp Gln Leu Lys Arg
1 5

<210> 84
<211> 9
<212> PRT
<213> Homo sapien

<400> 84

Phe Thr Gly Thr Ala Gly Ala Cys Arg
1 5

<210> 85
<211> 9
<212> PRT
<213> Homo sapien

<400> 85

Phe Thr Val His Phe Ser Gly Gln Phe
1 5

<210> 86
<211> 9
<212> PRT
<213> Homo sapien

<400> 86

Gly Ala Ala Gln Trp Ala Pro Val Leu
1 5

<210> 87
<211> 9
<212> PRT
<213> Homo sapien

<400> 87

Gly Ala Glu Pro His Glu Glu Gln Cys
1 5

<210> 88
<211> 9
<212> PRT
<213> Homo sapien

<400> 88

Gly Ala Thr Leu Lys Gly Val Ala Ala
1 5

<210> 89
<211> 9
<212> PRT
<213> Homo sapien

<400> 89

Gly Cys Ala Leu Pro Val Ser Gly Ala
1 5

<210> 90
<211> 9
<212> PRT
<213> Homo sapien

<400> 90

Gly Cys Asn Lys Arg Tyr Phe Lys Leu
1 5

<210> 91
<211> 9
<212> PRT

<213> Homo sapien

<400> 91

Gly Glu Lys Pro Tyr Gln Cys Asp Phe
1 5

<210> 92
<211> 9
<212> PRT
<213> Homo sapien

<400> 92

Gly Gly Gly Gly Cys Ala Leu Pro Val
1 5

<210> 93
<211> 9
<212> PRT
<213> Homo sapien

<400> 93

Gly Gly Pro Ala Pro Pro Pro Ala Pro
1 5

<210> 94
<211> 9
<212> PRT
<213> Homo sapien

<400> 94

Gly His Thr Pro Ser His His Ala Ala
1 5

<210> 95
<211> 9
<212> PRT
<213> Homo sapien

<400> 95

Gly Lys Thr Ser Glu Lys Pro Phe Ser
1 5

<210> 96
<211> 9
<212> PRT
<213> Homo sapien

<400> 96

Gly Pro Phe Gly Pro Pro Pro Pro Ser
1 5

<210> 97
<211> 9
<212> PRT
<213> Homo sapien

<400> 97

Gly Pro Pro Pro Pro Ser Gln Ala Ser
1 5

<210> 98
<211> 9
<212> PRT
<213> Homo sapien

<400> 98

Gly Gln Ala Arg Met Phe Pro Asn Ala
1 5

<210> 99
<211> 9
<212> PRT
<213> Homo sapien

<400> 99

Gly Gln Phe Thr Gly Thr Ala Gly Ala
1 5

<210> 100
<211> 9
<212> PRT
<213> Homo sapien

<400> 100

Gly Gln Ser Asn His Ser Thr Gly Tyr
1 5

<210> 101
<211> 9
<212> PRT
<213> Homo sapien

<400> 101

Gly Ser Asp Val Arg Asp Leu Asn Ala
1 5

<210> 102
<211> 9
<212> PRT
<213> Homo sapien

<400> 102

Gly Ser Gln Ala Leu Leu Leu Arg Thr
1 5

<210> 103
<211> 9
<212> PRT
<213> Homo sapien

<400> 103

Gly Val Phe Arg Gly Ile Gln Asp Val
1 5

<210> 104
<211> 9
<212> PRT
<213> Homo sapien

<400> 104

Gly Val Lys Pro Phe Gln Cys Lys Thr
1 5

<210> 105
<211> 9
<212> PRT
<213> Homo sapien

<400> 105

Gly Tyr Glu Ser Asp Asn His Thr Ala
1 5

<210> 106
<211> 9
<212> PRT
<213> Homo sapien

<400> 106

Gly Tyr Glu Ser Asp Asn His Thr Thr
1 5

<210> 107
<211> 9
<212> PRT
<213> Homo sapien

<400> 107

His Glu Glu Gln Cys Leu Ser Ala Phe
1 5

<210> 108
<211> 9
<212> PRT
<213> Homo sapien

<400> 108

His His Asn Met His Gln Arg Asn Met
1 5

<210> 109
<211> 9
<212> PRT
<213> Homo sapien

<400> 109

His Gln Arg Arg His Thr Gly Val Lys

1 5

<210> 110
<211> 9
<212> PRT
<213> Homo sapien

<400> 110

His Ser Phe Lys His Glu Asp Pro Met
1 5

<210> 111
<211> 9
<212> PRT
<213> Homo sapien

<400> 111

His Ser Arg Lys His Thr Gly Glu Lys
1 5

<210> 112
<211> 9
<212> PRT
<213> Homo sapien

<400> 112

His Thr Gly Glu Lys Pro Tyr Gln Cys
1 5

<210> 113
<211> 9
<212> PRT
<213> Homo sapien

<400> 113

His Thr His Gly Val Phe Arg Gly Ile
1 5

<210> 114
<211> 9
<212> PRT
<213> Homo sapien

<400> 114

His Thr Arg Thr His Thr Gly Lys Thr
1 5

<210> 115
<211> 9
<212> PRT
<213> Homo sapien

<400> 115

His Thr Thr Pro Ile Leu Cys Gly Ala
1 5

<210> 116
<211> 9
<212> PRT
<213> Homo sapien

<400> 116

Ile Leu Cys Gly Ala Gln Tyr Arg Ile
1 5

<210> 117
<211> 9
<212> PRT

<213> Homo sapien

<400> 117

Ile Arg Asn Gln Gly Tyr Ser Thr Val
1 5

<210> 118
<211> 9
<212> PRT
<213> Homo sapien

<400> 118

Lys Asp Cys Glu Arg Arg Phe Ser Arg
1 5

<210> 119
<211> 9
<212> PRT
<213> Homo sapien

<400> 119

Lys Phe Ala Arg Ser Asp Glu Leu Val
1 5

<210> 120
<211> 9
<212> PRT
<213> Homo sapien

<400> 120

Lys Phe Ser Arg Ser Asp His Leu Lys
1 5

<210> 121
<211> 9
<212> PRT
<213> Homo sapien

<400> 121

Lys His Glu Asp Pro Met Gly Gln Gln
1 5

<210> 122
<211> 9
<212> PRT
<213> Homo sapien

<400> 122

Lys Lys Phe Ala Arg Ser Asp Glu Leu
1 5

<210> 123
<211> 9
<212> PRT
<213> Homo sapien

<400> 123

Lys Pro Phe Ser Cys Arg Trp Pro Ser
1 5

<210> 124
<211> 9
<212> PRT
<213> Homo sapien

<400> 124

Lys Pro Tyr Gln Cys Asp Phe Lys Asp
1 5

<210> 125
<211> 9
<212> PRT
<213> Homo sapien

<400> 125

Lys Gln Glu Pro Ser Trp Gly Gly Ala
1 5

<210> 126
<211> 9
<212> PRT
<213> Homo sapien

<400> 126

Lys Arg His Gln Arg Arg His Thr Gly
1 5

<210> 127
<211> 9
<212> PRT
<213> Homo sapien

<400> 127

Lys Arg Tyr Phe Lys Leu Ser His Leu
1 5

<210> 128
<211> 9
<212> PRT
<213> Homo sapien

<400> 128

Lys Thr Cys Gln Arg Lys Phe Ser Arg
1 5

<210> 129
<211> 9
<212> PRT
<213> Homo sapien

<400> 129

Lys Thr Ser Glu Lys Pro Phe Ser Cys
1 5

<210> 130
<211> 9
<212> PRT
<213> Homo sapien

<400> 130

Leu Asp Phe Ala Pro Pro Gly Ala Ser
1 5

<210> 131
<211> 9
<212> PRT
<213> Homo sapien

<400> 131

Leu Glu Cys Met Thr Trp Asn Gln Met
1 5

<210> 132
<211> 9
<212> PRT
<213> Homo sapien

<400> 132

Leu Glu Ser Gln Pro Ala Ile Arg Asn
1 5

<210> 133
<211> 9

<212> PRT
<213> Homo sapien

<400> 133

Leu Gly Ala Thr Leu Lys Gly Val Ala
1 5

<210> 134
<211> 9
<212> PRT
<213> Homo sapien

<400> 134

Leu Gly Gly Gly Gly Gly Cys Ala Leu
1 5

<210> 135
<211> 9
<212> PRT
<213> Homo sapien

<400> 135

Leu Lys Gly Val Ala Ala Gly Ser Ser
1 5

<210> 136
<211> 9
<212> PRT
<213> Homo sapien

<400> 136

Leu Lys Arg His Gln Arg Arg His Thr
1 5

<210> 137
<211> 9
<212> PRT
<213> Homo sapien

<400> 137

Leu Lys Thr His Thr Arg Thr His Thr
1 5

<210> 138
<211> 9
<212> PRT
<213> Homo sapien

<400> 138

Leu Pro Val Ser Gly Ala Ala Gln Trp
1 5

<210> 139
<211> 9
<212> PRT
<213> Homo sapien

<400> 139

Leu Gln Met His Ser Arg Lys His Thr
1 5

<210> 140
<211> 9
<212> PRT
<213> Homo sapien

<400> 140

Leu Arg Thr Pro Tyr Ser Ser Asp Asn
1 5

<210> 141
<211> 9
<212> PRT
<213> Homo sapien

<400> 141

Leu Ser His Leu Gln Met His Ser Arg
1 5

<210> 142
<211> 9
<212> PRT
<213> Homo sapien

<400> 142

Met Cys Ala Tyr Pro Gly Cys Asn Lys
1 5

<210> 143
<211> 9
<212> PRT
<213> Homo sapien

<400> 143

Met His Gln Arg Asn Met Thr Lys Leu
1 5

<210> 144
<211> 9
<212> PRT
<213> Homo sapien

<400> 144

Asn Ala Pro Tyr Leu Pro Ser Cys Leu
1 5

<210> 145
<211> 9
<212> PRT
<213> Homo sapien

<400> 145

Asn Lys Arg Tyr Phe Lys Leu Ser His
1 5

<210> 146
<211> 9
<212> PRT
<213> Homo sapien

<400> 146

Asn Leu Gly Ala Thr Leu Lys Gly Val
1 5

<210> 147
<211> 9
<212> PRT
<213> Homo sapien

<400> 147

Asn Leu Tyr Gln Met Thr Ser Gln Leu
1 5

<210> 148
<211> 9
<212> PRT
<213> Homo sapien

<400> 148

Asn Met His Gln Arg Asn Met Thr Lys
1 5

<210> 149
<211> 9
<212> PRT
<213> Homo sapien

<400> 149

Asn Met Thr Lys Leu Gln Leu Ala Leu
1 5

<210> 150
<211> 9
<212> PRT
<213> Homo sapien

<400> 150

Asn Gln Gly Tyr Ser Thr Val Thr Phe
1 5

<210> 151
<211> 9
<212> PRT
<213> Homo sapien

<400> 151

Asn Gln Met Asn Leu Gly Ala Thr Leu
1 5

<210> 152
<211> 9
<212> PRT
<213> Homo sapien

<400> 152

Pro Ala Ile Arg Asn Gln Gly Tyr Ser
1 5

<210> 153
<211> 9
<212> PRT
<213> Homo sapien

<400> 153

Pro Gly Ala Ser Ala Tyr Gly Ser Leu
1 5

<210> 154
<211> 9
<212> PRT

<213> Homo sapien

<400> 154

Pro His Glu Glu Gln Cys Leu Ser Ala
1 5

<210> 155
<211> 9
<212> PRT
<213> Homo sapien

<400> 155

Pro Ile Leu Cys Gly Ala Gln Tyr Arg
1 5

<210> 156
<211> 9
<212> PRT
<213> Homo sapien

<400> 156

Pro Pro Pro Pro His Ser Phe Ile Lys
1 5

<210> 157
<211> 9
<212> PRT
<213> Homo sapien

<400> 157

Pro Pro Pro Pro Pro His Ser Phe Ile
1 5

<210> 158
<211> 9
<212> PRT
<213> Homo sapien

<400> 158

Pro Pro Pro Pro Pro Pro His Ser Phe
1 5

<210> 159
<211> 9
<212> PRT
<213> Homo sapien

<400> 159

Pro Ser Cys Gln Lys Lys Phe Ala Arg
1 5

<210> 160
<211> 9
<212> PRT
<213> Homo sapien

<400> 160

Gln Ala Leu Leu Leu Arg Thr Pro Tyr
1 5

<210> 161
<211> 9
<212> PRT
<213> Homo sapien

<400> 161

Gln Ala Ser Ser Gly Gln Ala Arg Met
1 5

<210> 162
<211> 9
<212> PRT
<213> Homo sapien

<400> 162

Gln Cys Asp Phe Lys Asp Cys Glu Arg
1 5

<210> 163
<211> 9
<212> PRT
<213> Homo sapien

<400> 163

Gln Cys Lys Thr Cys Gln Arg Lys Phe
1 5

<210> 164
<211> 9
<212> PRT
<213> Homo sapien

<400> 164

Gln Asp Val Arg Arg Val Pro Gly Val
1 5

<210> 165
<211> 9
<212> PRT
<213> Homo sapien

<400> 165

Gln Phe Thr Gly Thr Ala Gly Ala Cys
1 5

<210> 166
<211> 9
<212> PRT
<213> Homo sapien

<400> 166

Gln Gly Ser Leu Gly Glu Gln Gln Tyr
1 5

<210> 167
<211> 9
<212> PRT
<213> Homo sapien

<400> 167

Gln Leu Glu Cys Met Thr Trp Asn Gln
1 5

<210> 168
<211> 9
<212> PRT
<213> Homo sapien

<400> 168

Gln Met Asn Leu Gly Ala Thr Leu Lys
1 5

<210> 169
<211> 9
<212> PRT
<213> Homo sapien

<400> 169

Gln Met Thr Ser Gln Leu Glu Cys Met
1 5

<210> 170
<211> 9
<212> PRT
<213> Homo sapien

<400> 170

Gln Pro Ala Ile Arg Asn Gln Gly Tyr
1 5

<210> 171
<211> 9
<212> PRT
<213> Homo sapien

<400> 171

Gln Gln Tyr Ser Val Pro Pro Pro Val

1 5

<210> 172
<211> 9
<212> PRT
<213> Homo sapien

<400> 172

Gln Arg Lys Phe Ser Arg Ser Asp His
1 5

<210> 173
<211> 9
<212> PRT
<213> Homo sapien

<400> 173

Gln Arg Asn Met Thr Lys Leu Gln Leu
1 5

<210> 174
<211> 9
<212> PRT
<213> Homo sapien

<400> 174

Gln Trp Ala Pro Val Leu Asp Phe Ala
1 5

<210> 175
<211> 9
<212> PRT
<213> Homo sapien

<400> 175

Gln Tyr Arg Ile His Thr His Gly Val
1 5

<210> 176
<211> 9
<212> PRT
<213> Homo sapien

<400> 176

Gln Tyr Ser Val Pro Pro Pro Val Tyr
1 5

<210> 177
<211> 9
<212> PRT
<213> Homo sapien

<400> 177

Arg Asp Leu Asn Ala Leu Leu Pro Ala
1 5

<210> 178
<211> 9
<212> PRT
<213> Homo sapien

<400> 178

Arg Phe Ser Arg Ser Asp Gln Leu Lys
1 5

<210> 179
<211> 9
<212> PRT
<213> Homo sapien

<400> 179

Arg Gly Ile Gln Asp Val Arg Arg Val
1 5

<210> 180
<211> 9
<212> PRT
<213> Homo sapien

<400> 180

Arg His His Asn Met His Gln Arg Asn
1 5

<210> 181
<211> 9
<212> PRT
<213> Homo sapien

<400> 181

Arg His Gln Arg Arg His Thr Gly Val
1 5

<210> 182
<211> 9
<212> PRT
<213> Homo sapien

<400> 182

Arg Ile His Thr His Gly Val Phe Arg
1 5

<210> 183
<211> 9
<212> PRT
<213> Homo sapien

<400> 183

Arg Lys Phe Ser Arg Ser Asp His Leu
1 5

<210> 184
<211> 9
<212> PRT
<213> Homo sapien

<400> 184

Arg Lys His Thr Gly Glu Lys Pro Tyr
1 5

<210> 185
<211> 9
<212> PRT
<213> Homo sapien

<400> 185

Arg Met Phe Pro Asn Ala Pro Tyr Leu
1 5

<210> 186
<211> 9
<212> PRT
<213> Homo sapien

<400> 186

Arg Asn Met Thr Lys Leu Gln Leu Ala
1 5

<210> 187
<211> 9
<212> PRT
<213> Homo sapien

<400> 187

Arg Arg Phe Ser Arg Ser Asp Gln Leu
1 5

<210> 188
<211> 9
<212> PRT
<213> Homo sapien

<400> 188

Arg Arg His Thr Gly Val Lys Pro Phe
1 5

<210> 189
<211> 9
<212> PRT
<213> Homo sapien

<400> 189

Arg Arg Val Pro Gly Val Ala Pro Thr
1 5

<210> 190
<211> 9
<212> PRT

<213> Homo sapien

<400> 190

Arg Ser Ala Ser Glu Thr Ser Glu Lys
1 5

<210> 191
<211> 9
<212> PRT
<213> Homo sapien

<400> 191

Arg Ser Asp Glu Leu Val Arg His His
1 5

<210> 192
<211> 9
<212> PRT
<213> Homo sapien

<400> 192

Arg Ser Asp His Leu Lys Thr His Thr
1 5

<210> 193
<211> 9
<212> PRT
<213> Homo sapien

<400> 193

Arg Ser Asp Gln Leu Lys Arg His Gln
1 5

<210> 194
<211> 9
<212> PRT
<213> Homo sapien

<400> 194

Arg Thr Pro Tyr Ser Ser Asp Asn Leu
1 5

<210> 195
<211> 9
<212> PRT
<213> Homo sapien

<400> 195

Arg Val Pro Gly Val Ala Pro Thr Leu
1 5

<210> 196
<211> 9
<212> PRT
<213> Homo sapien

<400> 196

Arg Trp Pro Ser Cys Gln Lys Lys Phe
1 5

<210> 197
<211> 9
<212> PRT
<213> Homo sapien

<400> 197

Ser Ala Ser Glu Thr Ser Glu Lys Arg
1 5

<210> 198
<211> 9
<212> PRT
<213> Homo sapien

<400> 198

Ser Cys Leu Glu Ser Gln Pro Ala Ile
1 5

<210> 199
<211> 9
<212> PRT
<213> Homo sapien

<400> 199

Ser Cys Leu Glu Ser Gln Pro Thr Ile
1 5

<210> 200
<211> 9
<212> PRT
<213> Homo sapien

<400> 200

Ser Cys Gln Lys Lys Phe Ala Arg Ser
1 5

<210> 201
<211> 9
<212> PRT
<213> Homo sapien

<400> 201

Ser Cys Arg Trp Pro Ser Cys Gln Lys
1 5

<210> 202
<211> 9
<212> PRT
<213> Homo sapien

<400> 202

Ser Cys Thr Gly Ser Gln Ala Leu Leu
1 5

<210> 203
<211> 9
<212> PRT
<213> Homo sapien

<400> 203

Ser Asp Glu Leu Val Arg His His Asn
1 5

<210> 204
<211> 9
<212> PRT
<213> Homo sapien

<400> 204

Ser Asp Asn His Thr Thr Pro Ile Leu
1 5

<210> 205
<211> 9
<212> PRT
<213> Homo sapien

<400> 205

Ser Asp Asn Leu Tyr Gln Met Thr Ser
1 5

<210> 206
<211> 9
<212> PRT

<213> Homo sapien

<400> 206

Ser Asp Val Arg Asp Leu Asn Ala Leu
1 5

<210> 207
<211> 9
<212> PRT
<213> Homo sapien

<400> 207

Ser Glu Lys Pro Phe Ser Cys Arg Trp
1 5

<210> 208
<211> 9
<212> PRT
<213> Homo sapien

<400> 208

Ser Glu Lys Arg Pro Phe Met Cys Ala
1 5

<210> 209
<211> 9
<212> PRT
<213> Homo sapien

<400> 209

Ser Glu Thr Ser Glu Lys Arg Pro Phe
1 5

<210> 210
<211> 9
<212> PRT
<213> Homo sapien

<400> 210

Ser Phe Ile Lys Gln Glu Pro Ser Trp
1 5

<210> 211
<211> 9
<212> PRT
<213> Homo sapien

<400> 211

Ser Gly Ala Ala Gln Trp Ala Pro Val
1 5

<210> 212
<211> 9
<212> PRT
<213> Homo sapien

<400> 212

Ser Gly Gln Ala Arg Met Phe Pro Asn
1 5

<210> 213
<211> 9
<212> PRT
<213> Homo sapien

<400> 213

Ser His His Ala Ala Gln Phe Pro Asn
1 5

<210> 214
<211> 9
<212> PRT
<213> Homo sapien

<400> 214

Ser Leu Gly Glu Gln Gln Tyr Ser Val
1 5

<210> 215
<211> 9
<212> PRT
<213> Homo sapien

<400> 215

Ser Leu Gly Gly Gly Gly Gly Cys Ala
1 5

<210> 216
<211> 9
<212> PRT
<213> Homo sapien

<400> 216

Ser Gln Ala Ser Ser Gly Gln Ala Arg
1 5

<210> 217
<211> 9
<212> PRT
<213> Homo sapien

<400> 217

Ser Ser Asp Asn Leu Tyr Gln Met Thr
1 5

<210> 218
<211> 9
<212> PRT
<213> Homo sapien

<400> 218

Ser Val Pro Pro Pro Val Tyr Gly Cys
1 5

<210> 219
<211> 9
<212> PRT
<213> Homo sapien

<400> 219

Thr Cys Gln Arg Lys Phe Ser Arg Ser
1 5

<210> 220
<211> 9
<212> PRT
<213> Homo sapien

<400> 220

Thr Asp Ser Cys Thr Gly Ser Gln Ala
1 5

<210> 221
<211> 9
<212> PRT
<213> Homo sapien

<400> 221

Thr Glu Gly Gln Ser Asn His Ser Thr
1 5

<210> 222
<211> 9
<212> PRT

<213> Homo sapien

<400> 222

Thr Gly Lys Thr Ser Glu Lys Pro Phe
1 5

<210> 223
<211> 9
<212> PRT
<213> Homo sapien

<400> 223

Thr Gly Ser Gln Ala Leu Leu Leu Arg
1 5

<210> 224
<211> 9
<212> PRT
<213> Homo sapien

<400> 224

Thr Gly Thr Ala Gly Ala Cys Arg Tyr
1 5

<210> 225
<211> 9
<212> PRT
<213> Homo sapien

<400> 225

Thr Gly Tyr Glu Ser Asp Asn His Thr
1 5

<210> 226
<211> 9
<212> PRT
<213> Homo sapien

<400> 226

Thr Leu Val Arg Ser Ala Ser Glu Thr
1 5

<210> 227
<211> 9
<212> PRT
<213> Homo sapien

<400> 227

Thr Pro Ile Leu Cys Gly Ala Gln Tyr
1 5

<210> 228
<211> 9
<212> PRT
<213> Homo sapien

<400> 228

Thr Pro Ser His His Ala Ala Gln Phe
1 5

<210> 229
<211> 9
<212> PRT
<213> Homo sapien

<400> 229

Thr Pro Ser Tyr Gly His Thr Pro Ser
1 5

<210> 230
<211> 9
<212> PRT
<213> Homo sapien

<400> 230

Thr Pro Thr Asp Ser Cys Thr Gly Ser
1 5

<210> 231
<211> 9
<212> PRT
<213> Homo sapien

<400> 231

Thr Pro Tyr Ser Ser Asp Asn Leu Tyr
1 5

<210> 232
<211> 9
<212> PRT
<213> Homo sapien

<400> 232

Thr Ser Glu Lys Pro Phe Ser Cys Arg
1 5

<210> 233
<211> 9
<212> PRT
<213> Homo sapien

<400> 233

Thr Ser Glu Lys Arg Pro Phe Met Cys

1 5

<210> 234
<211> 9
<212> PRT
<213> Homo sapien

<400> 234

Thr Ser Gln Leu Glu Cys Met Thr Trp
1 5

<210> 235
<211> 9
<212> PRT
<213> Homo sapien

<400> 235

Thr Val His Phe Ser Gly Gln Phe Thr
1 5

<210> 236
<211> 9
<212> PRT
<213> Homo sapien

<400> 236

Val Ala Ala Gly Ser Ser Ser Ser Val
1 5

<210> 237
<211> 9
<212> PRT
<213> Homo sapien

<400> 237

Val Ala Pro Thr Leu Val Arg Ser Ala
1 5

<210> 238
<211> 9
<212> PRT
<213> Homo sapien

<400> 238

Val Phe Arg Gly Ile Gln Asp Val Arg
1 5

<210> 239
<211> 9
<212> PRT
<213> Homo sapien

<400> 239

Val Lys Pro Phe Gln Cys Lys Thr Cys
1 5

<210> 240
<211> 9
<212> PRT
<213> Homo sapien

<400> 240

Val Lys Trp Thr Glu Gly Gln Ser Asn
1 5

<210> 241
<211> 9
<212> PRT
<213> Homo sapien

<400> 241

Val Leu Asp Phe Ala Pro Pro Gly Ala
1 5

<210> 242
<211> 9
<212> PRT
<213> Homo sapien

<400> 242

Val Pro Gly Val Ala Pro Thr Leu Val
1 5

<210> 243
<211> 9
<212> PRT
<213> Homo sapien

<400> 243

Val Arg His His Asn Met His Gln Arg
1 5

<210> 244
<211> 9
<212> PRT
<213> Homo sapien

<400> 244

Val Thr Phe Asp Gly Thr Pro Ser Tyr
1 5

<210> 245
<211> 9
<212> PRT
<213> Homo sapien

<400> 245

Trp Asn Gln Met Asn Leu Gly Ala Thr
1 5

<210> 246
<211> 9
<212> PRT
<213> Homo sapien

<400> 246

Trp Pro Ser Cys Gln Lys Lys Phe Ala
1 5

<210> 247
<211> 9
<212> PRT
<213> Homo sapien

<400> 247

Trp Thr Glu Gly Gln Ser Asn His Ser
1 5

<210> 248
<211> 9
<212> PRT
<213> Homo sapien

<400> 248

Tyr Phe Lys Leu Ser His Leu Gln Met
1 5

<210> 249
<211> 9
<212> PRT
<213> Homo sapien

<400> 249

Tyr Gly His Thr Pro Ser His His Ala
1 5

<210> 250
<211> 9
<212> PRT
<213> Homo sapien

<900> 250

Tyr Pro Gly Cys Asn Lys Arg Tyr Phe
1 5

<210> 251
<211> 9
<212> PRT
<213> Homo sapien

<400> 251

Tyr Gln Met Thr Ser Gln Leu Glu Cys
1 5

<210> 252
<211> 9
<212> PRT
<213> Homo sapien

<400> 252

Tyr Arg Ile His Thr His Gly Val Phe
1 5

<210> 253
<211> 9
<212> PRT

<213> Homo sapien

<400> 253

Tyr Ser Ser Asp Asn Leu Tyr Gln Met
1 5

<210> 254
<211> 9
<212> PRT
<213> Mus musculus

<400> 254

Ala Glu Pro His Glu Glu Gln Cys Leu
1 5

<210> 255
<211> 9
<212> PRT
<213> Mus musculus

<400> 255

Ala Leu Leu Pro Ala Val Ser Ser Leu
1 5

<210> 256
<211> 9
<212> PRT
<213> Mus musculus

<400> 256

Ala Tyr Gly Ser Leu Gly Gly Pro Ala
1 5

<210> 257
<211> 9
<212> PRT
<213> Mus musculus

<400> 257

Ala Tyr Pro Gly Cys Asn Lys Arg Tyr
1 5

<210> 258
<211> 9
<212> PRT
<213> Mus musculus

<400> 258

Cys Met Thr Trp Asn Gln Met Asn Leu
1 5

<210> 259
<211> 9
<212> PRT
<213> Mus musculus

<400> 259

Cys Thr Gly Ser Gln Ala Leu Leu Leu
1 5

<210> 260
<211> 9
<212> PRT
<213> Mus musculus

<400> 260

Asp Gly Ala Pro Ser Tyr Gly His Thr
1 5

<210> 261
<211> 9
<212> PRT
<213> Mus musculus

<400> 261

Asp Leu Asn Ala Leu Leu Pro Ala Val
1 5

<210> 262
<211> 9
<212> PRT
<213> Mus musculus

<400> 262

Asp Pro Met Gly Gln Gln Gly Ser Leu
1 5

<210> 263
<211> 9
<212> PRT
<213> Mus musculus

<400> 263

Asp Ser Cys Thr Gly Ser Gln Ala Leu
1 5

<210> 264
<211> 9
<212> PRT
<213> Mus musculus

<400> 264

Asp Val Arg Asp Leu Asn Ala Leu Leu
1 5

<210> 265
<211> 9
<212> PRT
<213> Mus musculus

<400> 265

Glu Gln Cys Leu Ser Ala Phe Thr Leu
1 5

<210> 266
<211> 9
<212> PRT
<213> Mus musculus

<400> 266

Glu Ser Asp Asn His Thr Ala Pro Ile
1 5

<210> 267
<211> 9
<212> PRT
<213> Mus musculus

<400> 267

Phe Pro Asn Ala Pro Tyr Leu Pro Ser
1 5

<210> 268
<211> 9
<212> PRT
<213> Mus musculus

<400> 268

Gly Cys Asn Lys Arg Tyr Phe Lys Leu
1 5

<210> 269
<211> 9
<212> PRT

<213> Mus musculus

<400> 269

Gly Gln Ala Arg Met Phe Pro Asn Ala
1 5

<210> 270
<211> 9
<212> PRT
<213> Mus musculus

<400> 270

Gly Val Phe Arg Gly Ile Gln Asp Val
1 5

<210> 271
<211> 9
<212> PRT
<213> Mus musculus

<400> 271

Gly Tyr Glu Ser Asp Asn His Thr Ala
1 5

<210> 272
<211> 9
<212> PRT
<213> Mus musculus

<400> 272

His Ser Phe Lys His Glu Asp Pro Met
1 5

<210> 273
<211> 9
<212> PRT
<213> Mus musculus

<400> 273

His Thr His Gly Val Phe Arg Gly Ile
1 5

<210> 274
<211> 9
<212> PRT
<213> Mus musculus

<400> 274

Ile Leu Cys Gly Ala Gln Tyr Arg Ile
1 5

<210> 275
<211> 9
<212> PRT
<213> Mus musculus

<400> 275

Lys Phe Ala Arg Ser Asp Glu Leu Val
1 5

<210> 276
<211> 9
<212> PRT
<213> Mus musculus

<400> 276

Lys Arg Tyr Phe Lys Leu Ser His Leu
1 5

<210> 277
<211> 9
<212> PRT
<213> Mus musculus

<400> 277

Lys Thr Ser Glu Lys Pro Phe Ser Cys
1 5

<210> 278
<211> 9
<212> PRT
<213> Mus musculus

<400> 278

Leu Glu Cys Met Thr Trp Asn Gln Met
1 5

<210> 279
<211> 9
<212> PRT
<213> Mus musculus

<400> 279

Leu Gly Gly Gly Gly Gly Cys Gly Leu
1 5

<210> 280
<211> 9
<212> PRT
<213> Mus musculus

<400> 280

Leu Gln Met His Ser Arg Lys His Thr
1 5

<210> 281
<211> 9
<212> PRT
<213> Mus musculus

<400> 281

Met His Gln Arg Asn Met Thr Lys Leu
1 5

<210> 282
<211> 9
<212> PRT
<213> Mus musculus

<400> 282

Asn Ala Pro Tyr Leu Pro Ser Cys Leu
1 5

<210> 283
<211> 9
<212> PRT
<213> Mus musculus

<400> 283

Asn Leu Gly Ala Thr Leu Lys Gly Met
1 5

<210> 284
<211> 9
<212> PRT
<213> Mus musculus

<400> 284

Asn Leu Tyr Gln Met Thr Ser Gln Leu
1 5

<210> 285
<211> 9
<212> PRT

<213> Mus musculus

<400> 285

Asn Met Thr Lys Leu His Val Ala Leu
1 5

<210> 286
<211> 9
<212> PRT
<213> Mus musculus

<400> 286

Asn Gln Met Asn Leu Gly Ala Thr Leu
1 5

<210> 287
<211> 9
<212> PRT
<213> Mus musculus

<400> 287

Pro Gly Ala Ser Ala Tyr Gly Ser Leu
1 5

<210> 288
<211> 9
<212> PRT
<213> Mus musculus

<400> 288

Gln Ala Ser Ser Gly Gln Ala Arg Met
1 5

<210> 289
<211> 9
<212> PRT
<213> Mus musculus

<400> 289

Gln Met Thr Ser Gln Leu Glu Cys Met
1 5

<210> 290
<211> 9
<212> PRT
<213> Mus musculus

<400> 290

Gln Gln Tyr Ser Val Pro Pro Pro Val
1 5

<210> 291
<211> 9
<212> PRT
<213> Mus musculus

<400> 291

Gln Tyr Arg Ile His Thr His Gly Val
1 5

<210> 292
<211> 9
<212> PRT
<213> Mus musculus

<400> 292

Gln Tyr Ser Val Pro Pro Pro Val Tyr
1 5

<210> 293
<211> 9
<212> PRT
<213> Mus musculus

<400> 293

Arg Met Phe Pro Asn Ala Pro Tyr Leu
1 5

<210> 294
<211> 9
<212> PRT
<213> Mus musculus

<400> 294

Arg Thr Pro Tyr Ser Ser Asp Asn Leu
1 5

<210> 295
<211> 9
<212> PRT
<213> Mus musculus

<400> 295

Arg Val Ser Gly Val Ala Pro Thr Leu
1 5

<210> 296
<211> 9
<212> PRT
<213> Mus musculus

<400> 296

Ser Cys Leu Glu Ser Gln Pro Thr Ile
1 5

<210> 297
<211> 9
<212> PRT
<213> Mus musculus

<400> 297

Ser Cys Gln Lys Lys Phe Ala Arg Ser
1 5

<210> 298
<211> 9
<212> PRT
<213> Mus musculus

<400> 298

Ser Asp Val Arg Asp Leu Asn Ala Leu
1 5

<210> 299
<211> 9
<212> PRT
<213> Mus musculus

<400> 299

Ser Leu Gly Glu Gln Gln Tyr Ser Val
1 5

<210> 300
<211> 9
<212> PRT
<213> Mus musculus

<400> 300

Thr Cys Gln Arg Lys Phe Ser Arg Ser
1 5

<210> 301
<211> 9
<212> PRT
<213> Mus musculus

<400> 301

Thr Glu Gly Gln Ser Asn His Gly Ile
1 5

<210> 302
<211> 9
<212> PRT
<213> Mus musculus

<400> 302

Thr Leu His Phe Ser Gly Gln Phe Thr
1 5

<210> 303
<211> 9
<212> PRT
<213> Mus musculus

<400> 303

Thr Leu Val Arg Ser Ala Ser Glu Thr
1 5

<210> 304
<211> 9
<212> PRT
<213> Mus musculus

<400> 304

Val Leu Asp Phe Ala Pro Pro Gly Ala
1 5

<210> 305
<211> 9
<212> PRT
<213> Mus musculus

<400> 305

Trp Asn Gln Met Asn Leu Gly Ala Thr
1 5

<210> 306
<211> 9
<212> PRT
<213> Mus musculus

<400> 306

Tyr Phe Lys Leu Ser His Leu Gln Met
1 5

<210> 307
<211> 9
<212> PRT
<213> Mus musculus

<400> 307

Tyr Gln Met Thr Ser Gln Leu Glu Cys
1 5

<210> 308
<211> 9
<212> PRT
<213> Mus musculus

<400> 308

Tyr Ser Ser Asp Asn Leu Tyr Gln Met
1 5

<210> 309
<211> 6
<212> PRT
<213> Homo sapien

<400> 309

Gly Ala Ala Gln Trp Ala
1 5

<210> 310
<211> 12
<212> PRT
<213> Homo sapien

<400> 310

Ala Ser Ala Tyr Gly Ser Leu Gly Gly Pro Ala Pro
1 5 10

<210> 311
<211> 15
<212> PRT
<213> Homo sapien

<400> 311

```
Ala Phe Thr Val His Phe Ser Gly Gln Phe Thr Gly Thr Ala Gly
 1               5                  10                  15
```

<210> 312
<211> 5
<212> PRT
<213> Homo sapien

<400> 312

```
His Ala Ala Gln Phe
 1               5
```

<210> 313
<211> 32
<212> PRT
<213> Homo sapien

<400> 313

```
Cys His Thr Pro Thr Asp Ser Cys Thr Gly Ser Gln Ala Leu Leu Leu
 1               5                  10                  15
Arg Thr Pro Tyr Ser Ser Asp Asn Leu Tyr Gln Met Thr Ser Gln Leu
            20                  25                  30
```

<210> 314
<211> 32
<212> PRT
<213> Homo sapien

<400> 314

```
Arg Ile His Thr His Gly Val Phe Arg Gly Ile Gln Asp Val Arg Arg
 1               5                  10                  15
Val Pro Gly Val Ala Pro Thr Leu Val Arg Ser Ala Ser Glu Thr Ser
            20                  25                  30
```

<210> 315
<211> 4
<212> PRT
<213> Homo sapien

<400> 315

```
Arg Tyr Phe Lys
 1
```

<210> 316
<211> 14
<212> PRT
<213> Homo sapien

<400> 316

```
Glu Arg Arg Phe Ser Arg Ser Asp Gln Leu Lys Arg His Gln
 1               5                   10
```

<210> 317
<211> 22
<212> PRT
<213> Homo sapien

<400> 317

```
Gln Arg Lys Phe Ser Arg Ser Asp His Leu Lys Thr His Thr Arg Thr
 1               5                   10                  15
His Thr Gly Lys Thr Ser
            20
```

<210> 318
<211> 21
<212> PRT
<213> Homo sapien

<400> 318

```
Cys Gln Lys Lys Phe Ala Arg Ser Asp Glu Leu Val Arg His His Asn
 1               5                   10                  15
Met His Gln Arg Asn
            20
```

<210> 319
<211> 449
<212> PRT
<213> Homo sapien

<400> 319

```
Met Gly Ser Asp Val Arg Asp Leu Asn Ala Leu Leu Pro Ala Val Pro
 1               5                  10                  15
Ser Leu Gly Gly Gly Gly Gly Cys Ala Leu Pro Val Ser Gly Ala Ala
            20                  25                  30
Gln Trp Ala Pro Val Leu Asp Phe Ala Pro Pro Gly Ala Ser Ala Tyr
        35                  40                  45
Gly Ser Leu Gly Gly Pro Ala Pro Pro Pro Ala Pro Pro Pro Pro Pro
    50                  55                  60
Pro Pro Pro Pro His Ser Phe Ile Lys Gln Glu Pro Ser Trp Gly Gly
65                  70                  75                  80
Ala Glu Pro His Glu Glu Gln Cys Leu Ser Ala Phe Thr Val His Phe
                85                  90                  95
Ser Gly Gln Phe Thr Gly Thr Ala Gly Ala Cys Arg Tyr Gly Pro Phe
            100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Ala Ile
    130                 135                 140
Arg Asn Gln Gly Tyr Ser Thr Val Thr Phe Asp Gly Thr Pro Ser Tyr
145                 150                 155                 160
Gly His Thr Pro Ser His His Ala Ala Gln Phe Pro Asn His Ser Phe
                165                 170                 175
Lys His Glu Asp Pro Met Gly Gln Gln Gly Ser Leu Gly Glu Gln Gln
            180                 185                 190
Tyr Ser Val Pro Pro Pro Val Tyr Gly Cys His Thr Pro Thr Asp Ser
        195                 200                 205
Cys Thr Gly Ser Gln Ala Leu Leu Leu Arg Thr Pro Tyr Ser Ser Asp
    210                 215                 220
Asn Leu Tyr Gln Met Thr Ser Gln Leu Glu Cys Met Thr Trp Asn Gln
225                 230                 235                 240
Met Asn Leu Gly Ala Thr Leu Lys Gly Val Ala Ala Gly Ser Ser Ser
                245                 250                 255
Ser Val Lys Trp Thr Glu Gly Gln Ser Asn His Ser Thr Gly Tyr Glu
            260                 265                 270
Ser Asp Asn His Thr Thr Pro Ile Leu Cys Gly Ala Gln Tyr Arg Ile
        275                 280                 285
His Thr His Gly Val Phe Arg Gly Ile Gln Asp Val Arg Arg Val Pro
    290                 295                 300
Gly Val Ala Pro Thr Leu Val Arg Ser Ala Ser Glu Thr Ser Glu Lys
305                 310                 315                 320
Arg Pro Phe Met Cys Ala Tyr Pro Gly Cys Asn Lys Arg Tyr Phe Lys
                325                 330                 335
Leu Ser His Leu Gln Met His Ser Arg Lys His Thr Gly Glu Lys Pro
            340                 345                 350
Tyr Gln Cys Asp Phe Lys Asp Cys Glu Arg Arg Phe Ser Arg Ser Asp
        355                 360                 365
Gln Leu Lys Arg His Gln Arg Arg His Thr Gly Val Lys Pro Phe Gln
    370                 375                 380
Cys Lys Thr Cys Gln Arg Lys Phe Ser Arg Ser Asp His Leu Lys Thr
385                 390                 395                 400
His Thr Arg Thr His Thr Gly Lys Thr Ser Glu Lys Pro Phe Ser Cys
                405                 410                 415
Arg Trp Pro Ser Cys Gln Lys Lys Phe Ala Arg Ser Asp Glu Leu Val
            420                 425                 430
Arg His His Asn Met His Gln Arg Asn Met Thr Lys Leu Gln Leu Ala
        435                 440                 445
Leu
```

<210> 320

<211> 449
<212> PRT
<213> Mus musculus

<400> 320

```
Met Gly Ser Asp Val Arg Asp Leu Asn Ala Leu Leu Pro Ala Val Ser
 1               5                  10                  15
Ser Leu Gly Gly Gly Gly Gly Cys Gly Leu Pro Val Ser Gly Ala Ala
            20                  25                  30
Gln Trp Ala Pro Val Leu Asp Phe Ala Pro Pro Gly Ala Ser Ala Tyr
        35                  40                  45
Gly Ser Leu Gly Gly Pro Ala Pro Pro Pro Ala Pro Pro Pro Pro Pro
    50                  55                  60
Pro Pro Pro Pro His Ser Phe Ile Lys Gln Glu Pro Ser Trp Gly Gly
65                  70                  75                  80
Ala Glu Pro His Glu Glu Gln Cys Leu Ser Ala Phe Thr Leu His Phe
                85                  90                  95
Ser Gly Gln Phe Thr Gly Thr Ala Gly Ala Cys Arg Tyr Gly Pro Phe
            100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Thr Ile
    130                 135                 140
Arg Asn Gln Gly Tyr Ser Thr Val Thr Phe Asp Gly Ala Pro Ser Tyr
145                 150                 155                 160
Gly His Thr Pro Ser His His Ala Ala Gln Phe Pro Asn His Ser Phe
                165                 170                 175
```

```
Lys His Glu Asp Pro Met Gly Gln Gln Gly Ser Leu Gly Glu Gln Gln
            180                 185                 190
Tyr Ser Val Pro Pro Pro Val Tyr Gly Cys His Thr Pro Thr Asp Ser
        195                 200                 205
Cys Thr Gly Ser Gln Ala Leu Leu Leu Arg Thr Pro Tyr Ser Ser Asp
    210                 215                 220
Asn Leu Tyr Gln Met Thr Ser Gln Leu Glu Cys Met Thr Trp Asn Gln
225                 230                 235                 240
Met Asn Leu Gly Ala Thr Leu Lys Gly Met Ala Ala Gly Ser Ser Ser
                245                 250                 255
Ser Val Lys Trp Thr Glu Gly Gln Ser Asn His Gly Ile Gly Tyr Glu
            260                 265                 270
Ser Asp Asn His Thr Ala Pro Ile Leu Cys Gly Ala Gln Tyr Arg Ile
        275                 280                 285
His Thr His Gly Val Phe Arg Gly Ile Gln Asp Val Arg Arg Val Ser
    290                 295                 300
Gly Val Ala Pro Thr Leu Val Arg Ser Ala Ser Glu Thr Ser Glu Lys
305                 310                 315                 320
Arg Pro Phe Met Cys Ala Tyr Pro Gly Cys Asn Lys Arg Tyr Phe Lys
                325                 330                 335
Leu Ser His Leu Gln Met His Ser Arg Lys His Thr Gly Glu Lys Pro
            340                 345                 350
Tyr Gln Cys Asp Phe Lys Asp Cys Glu Arg Arg Phe Ser Arg Ser Asp
        355                 360                 365
Gln Leu Lys Arg His Gln Arg Arg His Thr Gly Val Lys Pro Phe Gln
    370                 375                 380
Cys Lys Thr Cys Gln Arg Lys Phe Ser Arg Ser Asp His Leu Lys Thr
385                 390                 395                 400
His Thr Arg Thr His Thr Gly Lys Thr Ser Glu Lys Pro Phe Ser Cys
                405                 410                 415
Arg Trp His Ser Cys Gln Lys Lys Phe Ala Arg Ser Asp Glu Leu Val
            420                 425                 430
Arg His His Asn Met His Gln Arg Asn Met Thr Lys Leu His Val Ala
        435                 440                 445
Leu
```

<210> 321
<211> 9
<212> PRT
<213> Homo sapien and Mus musculus

<400> 321

```
Pro Ser Gln Ala Ser Ser Gly Gln Ala
 1               5
```

<210> 322
<211> 9
<212> PRT
<213> Homo sapien and Mus musculus

<400> 322

```
Ser Ser Gly Gln Ala Arg Met Phe Pro
 1               5
```

<210> 323

<211> 9
<212> PRT
<213> Homo sapien and Mus musculus

<400> 323

Gln Ala Arg Met Phe Pro Asn Ala Pro
1 5

<210> 324
<211> 9
<212> PRT
<213> Homo sapien and Mus musculus

<400> 324

Met Phe Pro Asn Ala Pro Tyr Leu Pro
1 5

<210> 325
<211> 9
<212> PRT
<213> Homo sapien and Mus musculus

<400> 325

Pro Asn Ala Pro Tyr Leu Pro Ser Cys
1 5

<210> 326
<211> 9
<212> PRT
<213> Homo sapien and Mus musculus

<400> 326

Ala Pro Tyr Leu Pro Ser Cys Leu Glu
1 5